# EUROPEAN PATENT APPLICATION

(11) **EP 4 692 252 A1**
(43) Date of publication of application: **11.02.2026**
(21) Application number: 24780613.6
(22) Date of filing: 28.03.2024
(51) Int. Cl.: C09D 11/16, A01N 25/02, A01N 47/12, A01P 1/00, A01P 3/00, A61K 8/11, A61K 8/81, A61Q 1/02, A61Q 5/06, C08F 20/10

(54) **FUNCTIONAL PARTICLE DISPERSION**

(30) Priority: 31.03.2023 JP 2023057637; 01.11.2023 JP 2023187917
(71) Applicant: MITSUBISHI PENCIL COMPANY, LIMITED, Tokyo 140-8537 (JP)
(72) Inventor: HAGA, Hisato, Tokyo 140-8537 (JP)
(74) Representative: Vossius & Partner Patentanwälte Rechtsanwälte mbB
(86) International application number: PCT/JP2024/012597
(87) International publication number: WO 2024/204512

(57) **Abstract**

A functional particle dispersion, and an aqueous ink composition for writing instruments containing the same are provided. The functional particle dispersion has, in a compatible manner, antimicrobial effects, reduction performance, and fragrance performance at high level and long-lasting effects of these (slow releasing properties) without adversely affecting other blended components and the like, exhibits excellent dispersion stability even in a compounding system that is easily affected by ions and the like, has a high degree of freedom in design of the functional particles, and can be used in various applications. The functional particle dispersion of the present disclosure at least contains functional particles dispersed in water and has a measured zeta potential value of the surface of the functional particle being a range of -15 to +15 mV, the functional particles including a (meth)acrylate monomer represented by the following General Formula (I), at least one type of specific antimicrobial component, a reducing component, and at least one type of aroma component as a fragrance. In Formula (I) provided above, A is H or the like, and R is H, an alkyl group having 1 to 22 carbon atoms, or the like.

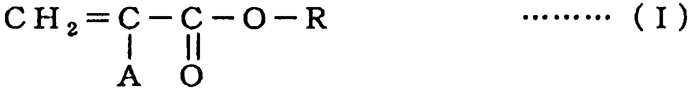

## Description

### Technical Field

The present description relates to a functional particle dispersion having excellent antimicrobial effects, fragrance performances, reduction performances against oxygen (oxygen absorbing ability), or the like, as well as excellent dispersion stability.

### Background Art

Various types of functional particles having various properties are known, including antimicrobial particles having antimicrobial effects, and reducing particles that can provide, in a compatible manner, the strength and long-lasting effects (slow releasing properties) of reduction performances against oxygen (oxygen absorbing ability).

The present applicant proposed a reducing particle dispersion in which reducing particles containing at least a (meth)acrylate monomer represented by a specific formula and a specific reducing component are dispersed in water (e.g., see Patent Document 1), and an antimicrobial particle dispersion in which antimicrobial particles at least containing a (meth)acrylate monomer represented by a specific formula and a specific antimicrobial component are dispersed in water (e.g., see Patent Document 2).

The reducing particle dispersion and antimicrobial particle dispersion disclosed in the aforementioned Patent Documents 1 and 2 are functional particle dispersions having novel excellent effects; however, when these dispersions are added to a compounding system of a fine liquid dispersion or a compounding system of an aqueous ink, the stability becomes slightly insufficient in some cases.

When further studies were conducted to investigate the reasons for this, each of the dispersions described in the aforementioned Patent Documents 1 and 2, in which antimicrobial particles and the like are structured by electric charge repulsion by using an anionic surfactant as a polymerizable surfactant to achieve a high dispersion force in the reducing particle dispersion or antimicrobial particle dispersion described above, had issues in some cases such as lacking capability of maintaining an initial state in which dispersion of the compounding system described above is stable, lacking capability of retaining a stable surface tension of an ink compared to its initial state, or the like due to electric charges of various particles contained in the ink and the like.

### Citation List

### Patent Document

Patent Document 1: JP 2022-138102 A (Claims, Examples, etc.)
Patent Document 2: JP 2023-001833 A (Claims, Examples, etc.)

### Summary of Invention

### Technical Problem

In light of the aforementioned issues in known technologies, the present disclosure is to solve those issues. An object of the disclosure is to provide a functional particle dispersion, which provides antimicrobial effects, fragrance performances, or the strength and long-lasting effects (slow releasing) of the reduction performance against oxygen (oxygen absorbing ability) in a compatible manner, has excellent dispersion stability and stability of surface tension even in a compounding system that is easily affected by ions, is low-irritative, has a high degree of freedom in design of a compounding system that is easily affected by ions even in a case where the functional particles are contained, and can be used in various applications.

### Solution to Problem

The present discloser conducted intensive studies in view of the aforementioned known issues and found that the functional particle dispersion for the aforementioned object and the like can be obtained by dispersing, in water, functional particles for specific physical properties containing at least a (meth)acrylate monomer represented by a specific formula and a specific functional component, and thus completed the present disclosure.

That is, a functional particle dispersion of the first aspect is characterized that the functional particles being formed from at least a (meth)acrylate monomer represented by General Formula (I) set forth below and at least one antimicrobial component selected from the following Group X, are dispersed in water and have a measured zeta potential value of the functional particles in a range of -15 to +15 mV.

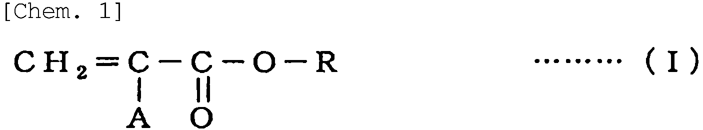

In Formula (I) provided above, A represents a hydrogen atom (H) or a methyl group (CH₃), and R represents a hydrogen atom (H), an alkyl group having 1 to 22 carbon atoms, or a substituent having a polyalkylene glycol chain containing an alkylene chain of 2 to 18 carbon atoms, where the alkyl group or the substituent having a polyalkylene glycol chain may have, as a substituent, a phenyl group, a benzyl group, an epoxy group, a hydroxyl group, a dialkylamino group, an alkoxy group having 1 to 18 carbon atoms, a perfluoroalkyl group having 1 to 18 carbon atoms, or a trialkoxysilyl group.

Group X: an iodopropargyl compound, thiabendazole, sodium pentachlorophenol, 1,2-benzisothiazolin-3-one, 2,3,5,6-tetrachloro-4(methylsulfonyl)pyridine, phenol, sodium benzoate, sodium dehydroacetate, potassium sorbate, morpholine, cresol, methylisothiazolinone, chloromethylisothiazolinone, octylisothiazolinone, dichlorooctylisothiazolinone, hexahydro-1,3,5-tris(2-hydroxyethyl)-1,3,5-triazine, 2-bromo-2-nitropropane-1,3-diol, sodium 2-pyridinethiol-1-oxide, sodium pyrithione, 2-(4-thiozolyl)benzimidazole, 4-terpineol, 1,8-cineol, thymol, diisothiocyanate, eucalyptus oil, longifolene, isopropylmethylphenol, 2-methyl-4-isothiazolin-3-one, citral, eugenol, allyl isothiocyanate, d-limonene, tannic acid, ethylparaben, benzalkonium chloride, glyceryl caprylate, a glycerin fatty acid ester, chlorphenesin, salicylic acid, ethyl parahydroxybenzoate, butyl parahydroxybenzoate, propyl parahydroxybenzoate, methyl parahydroxybenzoate, bisabolol, hinokitiol, phenylethyl alcohol, phenethyl alcohol, phenoxyethanol, butylparaben, propylparaben, , and methylparaben.

Furthermore, a functional particle dispersion of the second aspect is characterized by that the functional particles being formed from a(meth)acrylate monomer represented by General Formula (I) provided above, at least one antimicrobial component selected from Group X described above, and at least one type of fragrance component are dispersed in water and have a measured zeta potential value of the functional particles in a range of -15 to +15 mV.

Furthermore, a functional particle dispersion of the third aspect is characterized by that the functional particles being formed from a(meth)acrylate monomer represented by General Formula (I) provided above and at least one reducing component selected from Group Z set forth below are dispersed in water and have a measured zeta potential value of the functional particles in a range of -15 to +15 mV.

Group Z: polyphenols, copper chlorophyll, flavonoids, anthocyanidins, dibutylhydroxytoluene, and butylhydroxyanisole. An aqueous ink composition for writing instruments of the present disclosure is characterized by containing the functional particle dispersion according to any one of the first aspect to the third aspect described above.

A cosmetic of the present disclosure is characterized by comprising the functional particle dispersion according to any one of the first aspect to the third aspect described above. Advantageous Effects of Invention

According to the present disclosure, a functional particle dispersion that can provide antimicrobial effects, fragrance performance, or strength and long-lasting effects (slow releasing properties) of the reduction performance against oxygen (oxygen absorbing ability) in a compatible manner, that has excellent dispersion stability and stability of surface tension even in a compounding system that is easily affected by ions or the like, that is low-irritative, that has a high degree of freedom in design of a compounding system that is easily affected by ions or the like even in a case where the functional particles are contained, and that can be used in various applications, and an aqueous ink composition for writing instruments containing the functional particle dispersion and a cosmetic containing the functional particle dispersion are provided.

The object and effects of the present disclosure can be recognized and obtained especially using the components and combinations indicated in the claims. Both the general explanation described above and the detailed explanation described below are exemplary and explanatory and do not limit the present disclosure described in claims.

### Brief Description of Drawings

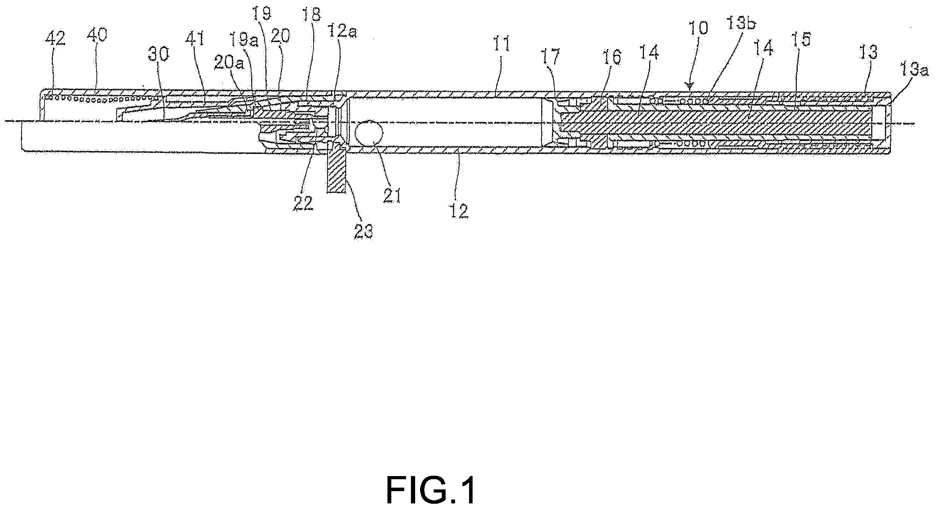

FIG. 1 is a partial cross-sectional view illustrating an example of a cosmetic applicator that stores a cosmetic of the present disclosure.

### Description of Embodiments

Embodiments of the present disclosure will be described below in detail. However, it should be noted that the technical scope of the present disclosure is not limited to the embodiments detailed below and includes the invention described in claims and equivalents thereof. Hereinafter, "the present disclosure" refers to functional particle dispersions of the first embodiments to the third embodiments described below. Furthermore, "antimicrobial effects" includes an antifungal effect and a preservative effect. In addition, "antimicrobial (performance)" includes an antifungal (performance) and a preservative (performance).

### First Embodiment of Functional Particle Dispersion

The first embodiment of a functional particle dispersion is characterized by that the functional particles comprising a (meth)acrylate monomer represented by General Formula (I) below and at least one antimicrobial component selected from Group X set forth below are dispersed in water and have a measured zeta potential value of the functional particles in a range of -15 to +15 mV.

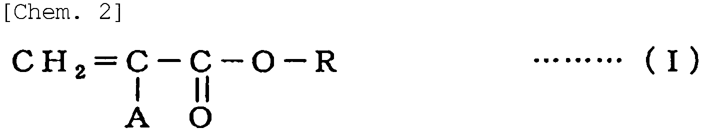

In Formula (I) provided above, A represents a hydrogen atom (H) or a methyl group (CH₃), and R represents a hydrogen atom (H), an alkyl group having 1 to 22 carbon atoms, or a substituent having a polyalkylene glycol chain containing an alkylene chain of 2 to 18 carbon atoms, where the alkyl group or the substituent having a polyalkylene glycol chain may have, as a substituent, a phenyl group, a benzyl group, an epoxy group, a hydroxyl group, a dialkylamino group, an alkoxy group having 1 to 18 carbon atoms, a perfluoroalkyl group having 1 to 18 carbon atoms, or a trialkoxysilyl group.

Group X: an iodopropargyl compound, thiabendazole, sodium pentachlorophenol, 1,2-benzisothiazolin-3-one, 2,3,5,6-tetrachloro-4(methylsulfonyl)pyridine, p-hydroxybenzoate, phenol, sodium benzoate, sodium dehydroacetate, potassium sorbate, morpholine, cresol, methylisothiazolinone, chloromethylisothiazolinone, octylisothiazolinone, dichlorooctylisothiazolinone, hexahydro-1,3,5-tris(2-hydroxyethyl)-1,3,5-triazine, 2-bromo-2-nitropropane-1,3-diol, sodium 2-pyridinethiol-1-oxide, sodium pyrithione, 2-(4-thiozolyl)benzimidazole, 4-terpineol, 1,8-cineol, thymol, diisothiocyanate, eucalyptus oil, longifolene, isopropylmethylphenol, 2-methyl-4-isothiazolin-3-one, citral, eugenol, allyl isothiocyanate, d-limonene, tannic acid, sodium benzoate, ethylparaben, benzalkonium chloride, glyceryl caprylate, a glycerin fatty acid ester, chlorphenesin, salicylic acid, ethyl parahydroxybenzoate, butyl parahydroxybenzoate, propyl parahydroxybenzoate, methyl parahydroxybenzoate, bisabolol, hinokitiol, phenylethyl alcohol, phenethyl alcohol, phenoxyethanol, butylparaben, propylparaben, , and methylparaben.

The (meth)acrylate monomer represented by the aforementioned General Formula (I) used in the present disclosure is used from the viewpoints of the strength of a functional component that can be encapsulated, the ability to produce stable particles, the absence of adverse effects on other blended components, the strong long-lasting effects, and the like.

R in the aforementioned General Formula (I) represents a hydrogen atom (H), an alkyl group having 1 to 22 carbon atoms, or a substituent having a polyalkylene glycol chain containing an alkylene chain of 2 to 18 carbon atoms, and the alkyl group or the substituent having a polyalkylene glycol chain may have, as a substituent, a phenyl group, a benzyl group, an epoxy group, a hydroxyl group, a dialkylamino group, an alkoxy group having 1 to 18 carbon atoms, a perfluoroalkyl group having 1 to 18 carbon atoms, or a trialkoxysilyl group, and examples thereof include a linear or branched alkyl group having 1 to 20 carbon atoms, a cycloalkyl group having 3 to 10 carbon atoms, and an alkyl group having 1 to 18 carbon atoms which may have, as a substituent, an epoxy group, a hydroxyl group, a dialkylamino group, or an alkoxy group having 1 to 4 carbon atoms, and above all, an alkyl group having 1 to 6 carbon atoms which may have, as a substituent, an epoxy group, a hydroxyl group, or an alkoxy group having 1 to 2 carbon atoms, and an alkyl group having 1 to 6 carbon atoms which may have, as a substituent, an epoxy group are exemplified. Preferably, R in the aforementioned General Formula (I) is a linear or branched alkyl group having 1 to 20 carbon atoms, a cycloalkyl group having 3 to 10 carbon atoms, a hydroxyl group, a trifluoroethyl group, a dimethylaminoethyl group, a methoxyethyl group, a hydroxyethyl group, a hydroxypropyl group, an allyl group, a tetrahydrofurfuryl group, a phenyl group, a benzyl group, a butoxydiethylene glycol group, a methoxypolyethylene glycol group, a dimethylaminoethyl group, a diethylaminoethyl group, a dimethylaminoethyl group, a glycidyl group, ethyl phosphate, 1,4-butanediol, 1,6-hexanediol, or 1,9-nonanediol.
Note that, in the present description, the expression "(meth)acrylic acid" represents "acrylic acid and/or methacrylic acid".

Specific examples of the (meth)acrylate represented by the aforementioned General Formula (I) include at least one (each alone, or two or more; the same applies hereinafter) selected from: methyl (meth)acrylate, ethyl (meth)acrylate, propyl (meth)acrylate, isopropyl (meth)acrylate, n-butyl (meth)acrylate, isobutyl (meth)acrylate, t-butyl (meth)acrylate, hexyl (meth)acrylate, octyl (meth)acrylate, 2-ethylhexyl (meth)acrylate, lauryl (meth)acrylate, palmityl (meth)acrylate, stearyl (meth)acrylate, behenyl (meth)acrylate, cyclohexyl (meth)acrylate, phenyl (meth)acrylate, benzyl (meth)acrylate, isobornyl (meth)acrylate, glycidyl (meth)acrylate, tetrahydrofurfuryl (meth)acrylate, allyl (meth)acrylate, 2-hydroxyethyl (meth)acrylate, hydroxypropyl (meth)acrylate, 2-methoxyethyl (meth)acrylate, 2-ethoxyethyl (meth)acrylate, dimethylaminoethyl (meth)acrylate, dimethylaminoethyl (meth)acrylate methyl chloride salt, diethylaminoethyl (meth)acrylate, ethylene glycol di(meth)acrylate, triethylene glycol di(meth)acrylate, 1,3-butylene glycol di(meth)acrylate, 1,6-hexanediol di(meth)acrylate, trimethylolpropane tri(meth)acrylate, 2-(meth)acryloyloxyethyl phthalate, 2-(meth)acryloyloxyethyl hexahydrophthalate, trifluoroethyl (meth)acrylate, butoxyethyl (meth)acrylate, methoxytetraethylene glycol (meth)acrylate, 2-hydroxypropyl (meth)acrylate, 3-chloro-2-hydroxypropyl (meth)acrylate, 2-hydroxy-3-phenoxypropyl (meth)acrylate, diethylene glycol (meth)acrylate, 2-(dimethylamino)ethyl (meth)acrylate, 2-(dimethylamino)propyl, 2-(dimethylamino)butyl (meth)acrylate, 2-isocyanoethyl (meth)acrylate, 2-(acetoacetoxy)ethyl (meth)acrylate, perfluoroethyl methacrylate having perfluoroalkyl with 1 to 18 carbon atoms, 2-(methacryloyloxy)ethyl phosphate, trialkoxysilylpropyl (meth)acrylate, and dialkoxymethylsilylpropyl (meth)acrylate.

Among these, from the viewpoints of industrial availability, easy handling and safety at the time of production, and further improvement of the effects of the present disclosure, preferably, methyl (meth)acrylate, ethyl (meth)acrylate, propyl (meth)acrylate, isopropyl (meth)acrylate, n-butyl (meth)acrylate, isobutyl (meth)acrylate, t-butyl (meth)acrylate, hexyl (meth)acrylate, octyl (meth)acrylate, 2-ethylhexyl (meth)acrylate, and cyclohexyl (meth)acrylate are desired.

In the first embodiment, in addition to the (meth)acrylate monomer described above, from the viewpoint of obtaining a durable functional effects and the like, preferably, a hydrophobic vinyl monomer besides the (meth)acrylate monomer described above, and an aqueous monomer can be further used.

As the hydrophobic vinyl monomer, for example, at least one of monomers such as styrenes such as styrene and methylstyrene besides the (meth)acrylate monomer described above can be used. Examples of the hydrophobic vinyl monomer that can be used include at least one of styrene, methylstyrene, chloromethyl styrene, alkyl styrene having an alkyl group having 1 to 12 carbon atoms, methoxy styrene, chlorostyrene, bromostyrene, divinylbenzene, phenyl styrene, vinyl naphthalene, vinyl acetate, vinyl chloride, acrylonitrile, or methacrylonitrile.

Examples of the aqueous monomer that can be used include at least one of glycerin monomethacrylate, sodium 2-sulfoethyl methacrylate, polyethylene glycol monomethacrylate, polypropylene glycol monomethacrylate, polyethylene glycol-polypropylene glycol monomethacrylate, polyethylene glycol-tetramethylene glycol-monomethacrylate, propylene glycol-polybutylene glycol-monomethacrylate, or vinyl pyrrolidone.

The antimicrobial component used in the first embodiment includes at least one (each alone, or a mixture of two or more; the same applies hereinafter) selected from the aforementioned Group X consisting of: an iodopropargyl compound, thiabendazole, sodium pentachlorophenol, 1,2-benzisothiazolin-3-one, 2,3,5,6-tetrachloro-4(methylsulfonyl)pyridine, para-hydroxybenzoate (ethyl, methyl, propyl, isopropyl, butyl, isobutyl, or the like), phenol, sodium benzoate, sodium dehydroacetate, potassium sorbate, morpholine, cresol, methylisothiazolinone, chloromethylisothiazolinone, octylisothiazolinone, dichlorooctylisothiazolinone, hexahydro-1,3,5-tris(2-hydroxyethyl)-1,3,5-triazine, 2-bromo-2-nitropropane-1,3-diol, sodium 2-pyridinethiol-1-oxide, sodium pyrithione, 2-(4-thiozolyl)benzimidazole, 4-terpineol, 1,8-cineol, thymol, diisothiocyanate, eucalyptus oil, longifolene, isopropylmethylphenol, 2-methyl-4-isothiazolin-3-one, citral, eugenol, allyl isothiocyanate, d-limonene, tannic acid, sodium benzoate, ethylparaben, benzalkonium chloride, a glyceryl caprylate, glycerin fatty acid ester, chlorphenesin, salicylic acid, ethyl parahydroxybenzoate, butyl parahydroxybenzoate, propyl parahydroxybenzoate, methyl parahydroxybenzoate, bisabolol, hinokitiol, phenylethyl alcohol, phenethyl alcohol, phenoxyethanol, butylparaben, propylparaben, , and methylparaben.

Examples of the iodopropargyl compound that can be used include at least one selected from 3-iodo-2-propynylpropylcarbamate, 3-iodo-2-propynylbutylcarbamate (IPBC), 3-iodo-2-propynyl-m-chlorophenylcarbamate, 3-iodo-2-propynylphenylcarbamate, 3-iodo-2-propynyl 2,4,5-trichlorophenylether, 3-iodo-2-propynyl 4-chlorophenylformal (IPCF), di-(3-iodo-2-propynyl) hexyldicarbamate, 3-iodo-2-propynyloxyethanolethylcarbamate, 3-iodo-2-propynyloxyethanolphenylcarbamate, 3-iodo-2-propynylthioxothioethylcarbamate, 3-iodo-2-propynylcarbamic acid ester (IPC), N-iodopropargyloxycarbonylalanine, N-iodopropargyloxycarbonylalanine ethyl ester, 3-(3-iodopropargyl)benzoxazol-2-one, 3-(3-iodopropargyl)-6-chlorobenzoxazol-2-one, 3-iodo-2-propynylalcohol, 4-chlorophenyl 3-iodopropargylformal, 3-bromo-2,3-diiodo-2-propenylethylcarbamate, 3-iodo-2-propynyl-n-hexylcarbamate, or 3-iodo-2-propynylcyclohexylcarbamate.

The antimicrobial components of the aforementioned Group X including these iodopropargyl compounds are compounds which are known in the related art and have high safety and antimicrobial properties, a production method of each compound is also known, and the compounds can be prepared by various production methods. In addition, commercially available products of the compounds included in the aforementioned Group X, if present, can be used. In the present disclosure, from the viewpoints of further safety and stability, preferably, among the aforementioned iodopropargyl compounds, at least 3-iodo-2-propynyl butyl carbamate (hereinafter, may be simply referred to as "IPBC") is preferably contained (IPBC alone or a mixture containing IPBC), and as a component other than the iodopropargyl compounds, at least one of thiabendazole, 2-bromo-2-nitropropane-1,3-diol, phenoxyethanol, 4-terpineol, 1,8-cineol, tannic acid, benzalkonium chloride, glycerin fatty acid ester, potassium sorbate, parabens (butylparaben, propylparaben, ethylparaben, methylparaben), chloromethylisothiazolinone, methylisothiazoline, or benzisothiazolinone is desirably used.

A functional particle dispersion of the first embodiment at least contains the (meth)acrylate monomer represented by General Formula (I) provided above and at least one antimicrobial component selected from Group X described above and has a measured zeta potential value of the functional particles being in a range of - 15 to +15 mV, or preferably in a range of -10 to +10 mV.

In the present disclosure (including Examples and the like described below), "measurement of zeta potential of functional particles" refers to a value obtained by the following measurement method. A zeta potential V is a value obtained by performing measurements at a measurement temperature of 25°C using a zeta potential analyzer DT-300 (available from Dispersion Technology, Inc.) and determining an arithmetical mean of three measurements.

By setting the measured zeta potential value of the functional particles described above to a range of -15 to +15 mV, even in a compounding system that is easily affected by ions or the like, an initial state in which dispersion of the functional particles is stable can be maintained. The obtained functional particle dispersion has excellent stability of dispersion and surface tension, is low-irritative, has a high degree of freedom in design of compounding, and can be used in various applications.

By setting the measured zeta potential value of the functional particles to a range of -15 to +15 mV, even in a compounding system that is easily affected by ions, for example, in an ordinary alkali swelling emulsion or polysaccharide fibers, such as cellulose nanofibers (e.g., gel having a hydrogen bond network of CNF), which are sensitive to electric charges as a thickener, an initial state in which dispersion of the functional particles is stable can be maintained. The obtained functional particle dispersion has excellent stability of dispersion and surface tension, is low-irritative, has a high degree of freedom in design of compounding, and can be used in various applications.

When the measured zeta potential value described above is outside the range of -15 to +15 mV, the effects of the present disclosure cannot be exhibited. When the measured zeta potential value is less than - 15 mV, change of the compounding system becomes larger over time, and the dispersion tends to fail. On the other hand, a measured zeta potential value of greater than +15 mV is not preferred because, similarly, change of the compounding system becomes larger over time, and dispersion tends to fail.

To adjust the measured zeta potential value described above to a range of -15 to + 15 mV, for example, although the functional particle dispersion described above is obtained by emulsion polymerization or the like, a functional particle dispersion having a predetermined zeta potential can be obtained by using a nonionic polymerizable surfactant (e.g., type and amount) as a polymerizable surfactant used during the emulsion polymerization. For the production of the functional particle dispersion of the first embodiment, for example, a dispersion of functional particles having the target measured zeta potential value in a range of -15 to +15 mV described above can be obtained by dissolving at least one of the antimicrobial components selected from Group X described above in the (meth)acrylate monomer described above (each alone or two or more; the same applies hereinafter) or a monomer mixture containing the (meth)acrylate monomer described above and a hydrophobic vinyl monomer besides that, using ammonium persulfate, potassium persulfate, hydrogen peroxide, or the like as a polymerization initiator or further using a reducing agent in combination with the aforementioned polymerization initiator as a polymerization initiator, and using a nonionic polymerizable surfactant (type, suitable amount) as a polymerizable surfactant.

Examples of the nonionic polymerizable surfactant that can be used include AQUARON AN-10, AN-20, AN-30, AN-5065 (these are polyoxyethylene styrenated propenyl phenyl ether: number of moles of EO addition 10, 20, 30, 40, 50), AQUARON RN-10, RN-20, RN-30, RN-50 (these are polyoxyethylene propenyl phenyl ether (nonylphenyl-based): number of moles of EO addition 10, 20, 30, 50) available from DKS Co., Ltd., LATEMUL PD-420, PD-430, PD-450 (these are polyoxyalkylene alkenyl ether) available from Kao Corporation, Blemmer PE-90, PE-200, PE-350 (these are PEG monomethacrylate: number of moles of EO addition 2, 4.5, 8), Blemmer AE-200, AE-400 (these are PEG monoacrylate: number of moles of EO addition 4.5, 10), Blemmer PP-1000, PP-500, PP-800 (these are PPG monomethacrylate: number of moles of PO addition 4 to 6, 9, 13), Blemmer AP-200, AP-400, AP-550, AP-800 (these are PPG monoacrylate: number of moles of PO addition: 3.5, 6, 9, 13) available from NOF Corporation, UNIOX PKA-5001, PKA-5002, PKA-5003, PKA-5004 (these are polyethylene glycol-allyl ether), UNISAFE PKA-5014F (this is polyoxypropylene allyl ether) available from NOF Corporation, ADEKA REASOAP ER-10, ER-20, ER-30, ER-40 (these are alkyl-type: number of moles of EO addition: 10, 20, 30, 40), and ADEKA REASOAP NE-10, NE-20, NE-30 (these are alkylphenol-type: (nonylphenyl-based) number of moles of EO addition: 10, 20, 30) available from ADEKA Corporation.

The used amount of these nonionic polymerizable surfactants is required to be an amount that makes the measured zeta potential value described above in a range of -15 to +15 mV. Although the used amount varies depending on at least one of the antimicrobial components selected from Group X described above to be used and the like, the used amount of these nonionic polymerizable surfactants is adjusted in a range of 0.1 to 50 mass% relative to the total amount of the monomers described above.

In the present disclosure (first embodiment to third embodiment), the functional particle dispersion having the measured zeta potential value of the particles in the predetermined range described above is formed by using a nonionic polymerizable surfactant (e.g., type and amount) as a polymerizable surfactant used during emulsion polymerization. With the reducing particle dispersion and antimicrobial particle dispersion obtained by using an anionic polymerizable surfactant described in Production Examples of Patent Documents 1 and 2 described above, the predetermined zeta potential described above cannot be achieved, and problems, such that a stable initial state cannot be maintained or a stable surface tension cannot be maintained compared to the initial state, may occur and thus the effects of the present disclosure cannot be exhibited.

Furthermore, in the present disclosure including the first embodiment, in a range that does not impair the effects of the present disclosure, the production may be performed by emulsion polymerization and the like by using a crosslinking agent, such as triallyl isocyanurate, triallyl isocyanurate, polyethylene glycol dimethacrylate, polypropylene glycol dimethacrylate, pentaerythritol acrylate, ditrimethylolpropane acrylate, dipentaerythritol acrylate, tripentaerythritol acrylate, methoxylated bisphenol A methacrylate, ethoxylated bisphenol A dimethacrylate, pentaerythritol methacrylate, ditrimethylolpropane methacrylate, dipentaerythritol methacrylate, tripentaerythritol methacrylate, ethoxylated polyglycerol methacrylate, allyl methacrylate, or 1,4-butanediol diacrylate, and as necessary, a polymerizable surfactant (emulsifier), such as ammonium polyoxyethylene-1-(allyloxymethyl)-alkyl ether sulfate, ether sulfate, ammonium polyoxyethylene nonyl propenyl phenyl ether sulfate, polyoxyethylene nonyl propenyl phenyl ether, ammonium polyacrylate, styrene-maleic acid copolymer ammonium, polyoxyethylene alkyl ether, polyoxyethylene styrenated phenyl ether, polyoxyethylene polyoxypropylene glycol, polyoxyalkylene decyl ether, polyoxyethylene tridecyl ether, an alkyl benzene sulfonate salt, a dioctyl sulfosuccinate salt, sodium lauryl sulfate, polyoxyethylene alkyl ether phosphate ester, polyoxyethylene styrenated phenyl ether phosphate ester, polyoxyethylene styrenated phenyl ether sulfate salt, polyoxyethylene alkyl ether sulfate salt, polyoxyethylene sorbitan monolaurate (polysorbate 20), polyoxyethylene sorbitan palmitate (polysorbate 40), polyoxyethylene sorbitan monostearate (polysorbate 60), or polyoxyethylene sorbitan oleate (polysorbate 80), and after being produced as a liquid dispersion of the functional particle dispersion, the functional particle dispersion can be formed by drying or the like.

The content of the aforementioned crosslinking agent, such as triallyl isocyanurate, is 0 to 50 mass%, and preferably 0.1 to 25 mass%, relative to the total amount of the monomers described above.

Use of the aforementioned crosslinking agent, such as triallyl isocyanurate, is preferred because heat resistance, mechanical properties, hydrolysis resistance, and weather resistance of the functional particle dispersion can be improved.

In the emulsion polymerization described above in the present disclosure including the first embodiment, a dicyclopenta(te)nyl (meth)acrylate monomer or the like may be further mixed with the aforementioned (meth)acrylate monomer and the like in an appropriate amount to carry out the emulsion polymerization. With the polymer obtained by further mixing the dicyclopenta(te)nyl (meth)acrylate monomer followed by the emulsion polymerization, even when water in the liquid dispersion is volatilized, the stability is less likely to be impaired, and a functional particle dispersion having superior stability can be obtained.

Examples of the dicyclopenta(te)nyl (meth)acrylate monomer that can be used include a dicyclopentanyl acrylate monomer, dicyclopentenyl acrylate, a dicyclopentanyl methacrylate monomer, and dicyclopentenyl methacrylate.

Furthermore, in the emulsion polymerization described above in the present disclosure including the first embodiment, in addition to the (meth)acrylate monomer described above, the hydrophobic vinyl monomers described above besides this, and the dicyclopenta(te)nyl (meth)acrylate monomer described above, monomers having a reactive crosslinking group such as an epoxy group, a hydroxymethylamide group, and an isocyanate group, or a polyfunctional monomer having two or more vinyl groups may be blended in an appropriate amount for crosslinking.

In the present disclosure including the first embodiment, in the polymer components constituting the functional particle dispersion, the content of the (meth)acrylate monomer described above needs to be 30 mass% or greater, and is preferably 30 to 95 mass%, and more preferably 30 to 70 mass%, relative to the total of the polymer components constituting the functional particle dispersion.

Note that, in the present disclosure including the first embodiment, the term "total of the polymer components" refers to polymerizable components constituting the functional particle dispersion, and specifically, it refers to the total amount of the (meth)acrylate monomer used, the other monomer components used, and the crosslinking agent described below.

When the content of the (meth)acrylate monomer described above is 30 mass% or greater relative to the total of the polymer components, the effects of the present disclosure can be exhibited. On the other hand, when this content is less than 30 mass%, stability over time is poor and desired slow releasing properties cannot be achieved, which are not preferred.

In the polymer components constituting the functional particle dispersion, the content of the other monomer components other than the aforementioned (meth)acrylate monomer is the remainder of the total amount of the (meth)acrylate monomer used and the crosslinking agent described below.

Preferably, the content of the other monomer components is desired to be 0.5 to 70 mass% relative to the total of the polymer components from the viewpoints of further exhibiting the effects of the present disclosure and of dispersibility and reactivity.

In the first embodiment, from the viewpoints of achieving sufficient antimicrobial performance, durable antimicrobial effects, stability, and the like, the content (solids content) of the antimicrobial component selected from Group X described above is desirably 1 mass% or greater, preferably 5 mass% or greater, more preferably 10 to 50 mass%, and particularly preferably 15 to 40 mass%, relative to the total of the polymer components.

When the content of the antimicrobial component is 1 mass% or greater, sufficient antimicrobial performance and durable antimicrobial effects can be exhibited. On the other hand, when the content of the antimicrobial component is less than 1 mass%, the antimicrobial performance is unsatisfactory, and as a result, the effects of the present disclosure cannot be exhibited.

In the first embodiment, the functional particle dispersion (liquid dispersion) in which functional particles having antimicrobial properties and having the predetermined zeta potential described above (the measured zeta potential value is in a range of -15 to +15 mV) are dispersed in water can be obtained by the preferred aspect described above. The preferred aspect described above specifically includes at least dissolving the antimicrobial component selected from Group X described above in the (meth)acrylate monomer described above and performing emulsion polymerization using the nonionic polymerizable surfactant (e.g., type and amount) or at least dissolving the antimicrobial component described above after polymerization of a monomer mixture containing the (meth)acrylate monomer described above and another monomer component and performing emulsion polymerization using the nonionic polymerizable surfactant (e.g., type and amount). The production amount of the functional particles in the functional particle dispersion obtained under these production conditions varies depending on blending amounts of the aforementioned (meth)acrylate monomer, the antimicrobial component, the nonionic polymerizable surfactant, and the like to be used, the polymerization conditions, and the like. From the viewpoints of productivity, workability, efficiency, and the like, the functional particles are preferably produced to have a solids content of 1 to 50 mass%. More preferably, the functional particles are produced to have a solids content of 10 to 40 mass%.

This functional particle dispersion (liquid dispersion) having the antimicrobial properties becomes a functional particle dispersion which has strength and long-lasting effects of antimicrobial performance, without adversely affecting other blended components and the like, and is excellent in stability by being made into the functional particle dispersion of the present disclosure, compared with a case where the antimicrobial component described above is used alone. In particular, a functional particle dispersion, which can maintain or improve the antimicrobial effects of the antimicrobial component even after a long period of time, can be obtained.

This functional particle dispersion (liquid dispersion) can maintain the initial state, in which dispersion is stable, even in a compounding system that is easily affected by ions or a compound system such as an aqueous ink, is low-irritative, has a high degree of freedom in design of a compounding system that is easily affected by ions or the like even in a case where the functional particles are contained, and can be suitably used in various applications.

In addition, in the first embodiment, an oil-soluble (lipophilic) preservative component or the like is preferably used as the antimicrobial component. Thus, in particular, for the problem in that the preservative component or the like easily adheres to a plastic container, thereby impairing the antimicrobial effects including an antiseptic power over time, the antimicrobial component is encapsulated in the particles made of the (meth)acrylic acid component and the like of the first embodiment to suppress adhesion to the container wall surface, thereby achieving a specific effect that the antimicrobial effects can be maintained even after a long period of time.

In the present disclosure including the first embodiment, an average particle size of the functional particles in the resulting functional particle dispersion varies depending on the (meth)acrylate monomer, the type of the other monomers to be used, the contents, the polymerization conditions in the polymerization, and the like. The average particle size of the functional particles is preferably 10 to 800 nm, more preferably 20 to 300 nm, and even more preferably 30 to 200 nm.

When the average particle size is within the preferred range described above, excellent storage stability and the like is achieved, and the functional particles having an average particle suitable for various applications described below can be used. In a case where the functional particle dispersion is used for an aqueous ink for writing instruments, pen cores for writing instruments such as felt-tip pens, marking pens, or ballpoint pens are not clogged, and further, excellent storage stability is exhibited.

Note that the "average particle size" specified in the present disclosure including the first embodiment is a histogram average particle size based on scattered light intensity distribution and, in the present disclosure (including Examples described below), is a value of D50 measured by using a particle size distribution measuring equipment [FPAR1000 (available from Otsuka Electronics Co., Ltd.)].

In the functional particle dispersion of the present disclosure including the first embodiment, the content of the functional particles contained in the dispersion is preferably 0.1 to 50 mass%, more preferably 1 to 30 mass%, in terms of solids content, depending on applications and the like described below.

When the content of the functional particles is less than 0.1 mass% in terms of solids content, the effects of the present disclosure cannot be exhibited. On the other hand, when the content of the functional particles exceeds 50 mass%, long-term storage stability tends to decrease.

The functional particle dispersion having the antimicrobial properties of the first embodiment configured as described above has high stability and safety, can maintain the initial state in which dispersion is stable, is low-irritative, has a high degree of freedom in design of a compounding system that is easily affected by ions or the like even in a case where the functional particles are contained, and can be used for various purposes, such as to impart antimicrobial properties to various products such as medical apparatus, baby products, nursing products, bath products, kitchen utensils, tableware, drinking water piping parts, daily hygiene products, household electrical appliances, clothing, building materials, agricultural materials, automobile interior parts, stationery, and ink compositions for writing instruments and inkjet printers.

The functional particle dispersion having the antimicrobial properties of the first embodiment configured as described above can be specifically used to impart antimicrobial properties to various products such as medical apparatus, baby products, nursing products, bath products, kitchen utensils, tableware, drinking water piping parts, daily hygiene products, household electrical appliances, clothing, building materials, agricultural materials, automobile interior parts, stationery, and ink compositions for writing instruments and inkjet printers.

In addition to the aforementioned applications, as specific applications, the functional particle dispersion can be suitably used for detergents such as a laundry detergent, a softener, a household detergent, a dish detergent, and a detergent for a hard surface; personal care applications such as a shampoo, a conditioner, a skin lotion, a milky lotion, a cream, a sunscreen, a foundation, an eye makeup product, a hair coloring product, a nail care product, an antiperspirant, and a toothpaste; a paint, an adhesive agent, a building material, a resin emulsion, a wood preservative, a cement admixture, a boiler, cooling equipment, waste water treatment equipment, industrial water treatment applications such as for industrial water (papermaking process water in a papermaking process, cooling water and washing water for various industries); a medical instrument, a food additive, and electronic devices such as a solar cell module, an organic device, and a heat-ray shielding film; and a water tank and a chemical bath for suppressing water mildew on aquatic organisms (such as fish).

The functional particle dispersion of the present disclosure has high stability and safety, is excellent in the antimicrobial effects of the antimicrobial component after a long period of time, and does not affect other blended components, and in addition, can maintain the initial state, in which dispersion is stable, even in a compounding system of a liquid dispersion that is sensitive to electric charges or a compound system such as an aqueous ink. Thus, as described above, the functional particle dispersion can be used to impart antimicrobial properties to various products, and in particular, can be suitably used in detergent applications, personal care applications, industrial water treatment applications, food additives, electronic device applications, and water tank and chemical bath applications as water mold suppression for aquatic organisms (such as fish). For example, hereinafter, a case where the functional particle dispersion is used for an aqueous ink composition for writing instruments is described.

The aqueous ink composition for writing instruments according to the first embodiment at least contains the functional particle dispersion having antimicrobial properties and having the aforementioned configuration, and can contain a colorant and a water-soluble organic solvent in addition to this functional particle dispersion.

The content of the functional particles in the ink composition is preferably 0.1 to 30.0 mass%, and more preferably 1.0 to 15.0 mass%, in terms of solids content relative to the total amount of the ink composition from the viewpoint of exhibiting the effects of the present disclosure without impairing writing performance and from the viewpoint of storage stability.

As the colorant that can be used, for example, a water-soluble dye, a pigment such as an inorganic pigment, an organic pigment, a plastic pigment, hollow resin particles having voids within the particles that are used as a white pigment, resin particles (pseudo-pigment) dyed with a dye having excellent color development and dispersibility, a thermochromic pigment, a photochromic pigment, or the like can be used.

For the water-soluble dye, a direct dye, an acid dye, an edible dye, or a basic dye can be used in an appropriate amount within a range in which the effects of the present disclosure are not impaired.

The content of the colorant varies depending on the type of the writing instrument and the like, and is 1 to 30 mass% relative to the total amount of the ink composition.

The water-soluble organic solvent that can be used includes, for example, at least one of alkylene glycols such as ethylene glycol, triethylene glycol, tetraethylene glycol, propylene glycol, dipropylene glycol, 1,2-propanediol, 1,3-propanediol, 1,2-butanediol, 2,3-butanediol, 1,3-butanediol, 1,4-butanediol, 1,2-pentanediol, 1,5-pentanediol, 2,5-hexanediol, 3-methyl-1,3-butanediol, 2-methylpentane-2,4-diol, 3-methylpentane-1,3,5-triol, and 1,2,3-hexanetriol; polyalkylene glycols such as polyethylene glycol and polypropylene glycol; glycerols such as glycerol, diglycerol, and triglycerol; lower alkyl ethers of glycols such as ethylene glycol monomethyl ether, ethylene glycol monoethyl ether, diethylene glycol monomethyl ether, diethylene glycol monoethyl ether, and diethylene glycol mono-n-butyl ether; N-methyl-2-pyrrolidone, or 1,3-dimethyl-2-imidalizinone.

Additionally, a water-soluble solvent, for example, alcohols such as methyl alcohol, ethyl alcohol, isopropyl alcohol, n-butyl alcohol, tert-butyl alcohol, isobutyl alcohol, hexyl alcohol, octyl alcohol, nonyl alcohol, decyl alcohol, or benzyl alcohol, amides such as dimethylformamide or diethylacetamide, or ketones such as acetone, can also be mixed.

The content of these water-soluble organic solvents varies depending on the type of writing instruments such as felt-tip pens, marking pens, and ballpoint pens, and is 1 to 40 mass% relative to the total amount of the ink composition. The ink composition in which the content of the solvent is 10 mass% or less is particularly effective from the viewpoint of further improving drying properties of the drawn lines, and the content is more preferably 3 to 8 mass%.

Besides the functional particle dispersion described above, the colorant, and the water-soluble solvent, the aqueous ink composition for writing instruments of the first embodiment can appropriately contain, as the balance, water as a solvent (e.g., tap water, purified water, distilled water, ion-exchanged water, or pure water) as well as a dispersant, a lubricant, a pH adjuster, a corrosion inhibitor, a thickener, an evaporation inhibitor, a surfactant, or the like, within a range in which the effects of the present disclosure are not impaired.

Examples of the dispersant that can be used include nonionic and anionic surfactants, and water-soluble resins. Preferably, water-soluble polymers are used.

Examples of the lubricant include non-ionic types such as fatty acid esters of polyhydric alcohols, higher fatty acid esters of sugars, polyoxyalkylene higher fatty acid esters, and alkyl phosphate esters; anionic types such as alkyl sulfonates of higher fatty acid amides and alkyl allyl sulfonates; derivatives of polyalkylene glycols, fluorochemical surfactants, and polyether modified silicones, which are also used as surface treating agents for pigments.

Examples of the pH adjuster include ammonia, urea, monoethanolamine, diethanolamine, triethanolamine, alkali metal salts of carbonic acid and phosphoric acid such as sodium tripolyphosphate and sodium carbonate, and hydrates of alkali metals such as sodium hydroxide. Examples of the corrosion inhibitor include benzotriazole, tolyltriazole, dicyclohexylammonium nitrite, and saponins.

Examples of the thickener include polysaccharide fibers such as carboxymethyl cellulose (CMC) or salts thereof, fermented cellulose, crystalline cellulose, cellulose nanofibers (CNF), chitin nanofibers, and chitosan nanofibers, and alkali thickeners such as alkali swelling emulsion.

The cellulose nanofibers (CNF) serve as a component that can form stable hand-drawn lines because scratchiness and ink pooling are less likely to occur even in circumstances such as fast writing and writing by a left hand. Also in a compounding system containing the CNF, the initial state in which dispersion of the functional particles is stable can be maintained, and excellent functions, such as dispersion stability of the functional particles, can be exhibited in addition to the ink performance without impairing satisfactory drawn line density because irritation is less even in an unstable system (ionic system) of an aqueous ink for writing instruments.

Cellulose nanofibers (CNF) are materials obtained by making a pulp or the like that is a cellulose raw material finer to nanometer level and refer to fine fibers having a fiber size of approximately 2 to 500 nm. Cellulose nanofibers can be obtained by making a pulp finer by applying a mechanical force to the pulp or can be obtained by defibration of modified cellulose fibers, such as carboxylated cellulose fibers (also referred to as oxidized cellulose fibers), carboxymethylated cellulose fibers, phosphoric acid-esterified cellulose fibers, or phosphorous acid-esterified cellulose fibers.

As the cellulose nanofibers (CNF) as a thickener that can be used, oxidized cellulose fibers or the modified cellulose fibers described above obtained by the aforementioned production can be used, or commercially available products, if present, can be used. Examples thereof include at least one of RHEOCRYSTA series (e.g., RHEOCRYSTA I-2SX, C-2SP, I-2AX, I-2SXS) available from DKS Co., Ltd., BiNFi-s series (e.g., FMa-10002) available from Sugino Machine Limited, ELLEX-S available from Daio Paper Corporation, or nanoforest-S available from Chuetsu Pulp & Paper Co., Ltd. Although the amount of these thickeners varies depending on the type of writing instrument (e.g., ballpoint pen, marking pen, or felt-tip pen), the amount of the thickener, in terms of solids content, is preferably 0.1 to 5.0 mass%, and more preferably 0.1 to 3.0 mass%, relative to the total amount of the ink composition.

Examples of the polysaccharide fibers that can be used include xanthan gum, guar gum, hydroxypropylated guar gum, casein, gum arabic, gelatin, amylose, agarose, agaropectin, arabinan, curdlan, callose, carboxymethyl starch, chitin, chitosan, quince seed, glucomannan, gellan gum, tamarind seed gum, dextran, nigeran, hyaluronic acid, pustulan, funoran, HM pectin, porphyran, laminaran, lichenan, carrageenan, alginic acid, tragacanth gum, alkasy gum, succinoglycan, locust bean gum, and tara gum. Furthermore, examples of the alkali thickener that can be used include an alkali swelling emulsion such as a polyacrylic acid-based thickener, and a commercially available product can be used. Examples of the commercially available product include "PRIMAL ASE60", "PRIMAL TT615", "PRIMAL RM5" (these are trade names) available from Rohm and Haas, and "RHEOTECH 3800" and "RHEOTECH 4800" available from Arkema.

One of these may be used alone, or two or more may be used in combination. Commercially available products of these, if present, can be used.

Examples of the vaporization inhibitor include pentaerythritol, p-xylene glycol, trimethylolpropane, triethylolpropane, and dextrin. Examples of the surfactant include fluorine-based, silicone-based, and acetylene glycol-based surfactants.

Examples of the fixing agent include at least one selected from water-soluble resins having a hydrophobic portion in the molecule, such as polyacrylic acid, water-soluble styrene-acrylic resins, water-soluble styrene-maleic acid resins, polyvinyl alcohol, polyvinylpyrrolidone, water-soluble maleic acid resins, water-soluble styrene resins, , water-soluble ester-acrylic resins, ethylene-maleic acid copolymers, polyethylene oxide, and water-soluble urethane resins, and resin emulsions such as polyolefin-based emulsions, acrylic emulsions, vinyl acetate-based emulsions, urethane-based emulsions, styrene-butadiene emulsions, and styrene-acrylonitrile emulsions, and it is desirable to use each of them alone or more, or a total of two or more of them.

The aqueous ink composition for writing instruments of the first embodiment can be prepared by appropriately combining the functional particle dispersion described above, the water-soluble solvent, and other components, depending on the application of the ink for writing instruments (e.g., for a ballpoint pen, a marking pen), and then mixing those by stirring using a mixer such as a homomixer, a homogenizer, or a disperser, and, as necessary, further filtering or centrifuging the mixture to remove coarse particles in the ink composition.

In addition, the pH (25°C) of the aqueous ink composition for writing instruments of the first embodiment is desired to be adjusted to preferably 5 to 10, and more preferably 6 to 9.5, using a pH adjuster from the viewpoints of usability, safety, stability of the ink itself, and matching properties with the ink container.

The aqueous ink composition for writing instruments of the present disclosure including the first embodiment is loaded in a ballpoint pen, a marking pen, or the like provided with a pen tip such as a ballpoint pen tip, a fiber tip, a felt tip, or a plastic tip.

The ballpoint pen includes an instrument where the aqueous ink composition for writing instruments having the aforementioned composition is accommodated in an ink container (refill) for a ballpoint pen having a ball with a diameter of 0.18 to 2.0 mm, and where a material which is not compatible with the aqueous ink composition accommodated in the ink container and which has a small specific gravity with respect to the aqueous ink composition, for example, polybutene, silicone oil, and mineral oil is accommodated as an ink follower.

Note that the structures of the ballpoint pen and the marking pen are not particularly limited, and the ballpoint pen and the marking pen may be, for example, a direct liquid type pen provided with a collector structure (ink holding mechanism) using a shaft cylinder itself as an ink container in which the shaft cylinder is filled with the aqueous ink composition for writing instruments having the configuration described above.

In the aqueous ink composition for writing instruments of the first embodiment configured as described above, the functional particle dispersion having the antimicrobial properties described above to be used is blended in the aqueous ink composition for writing instruments. Thus, the functional particle dispersion has the strength and long-lasting effects of the antimicrobial performance in the ink composition without adversely affecting other blended components or the like, and the persistent effect is maintained for a long period of time. In addition, these particles do not impair the storage stability and the writing performance, which can further enhance a degree of freedom in ink design. Accordingly, it is possible to obtain an aqueous ink composition for writing instruments suitable for writing instruments such as a ballpoint pen and a marking pen.

Furthermore, because the measured zeta potential value of the functional particles having the antimicrobial properties described above in the dispersion used in the aqueous ink composition for writing instruments of the first embodiment is in the predetermined range, the initial state in which dispersion of the functional particles is stable can be maintained even in a compounding system of an aqueous ink that is sensitive to electric charges and that uses polysaccharide fibers, such as cellulose nanofibers (e.g., gel having a hydrogen bond network of CNF), as a thickener of the ink for the writing instrument. In addition, irritation is less even in a compounding system of an unstable aqueous ink, such as an ionic compounding system, an aqueous ink composition for writing instruments having excellent dispersion stability can be obtained without impairing satisfactory drawn line density.

The case where the functional particle dispersion of the first embodiment is used for the aqueous ink composition for writing instruments has been described above. The functional particle dispersion of the first embodiment is excellent in antimicrobial effects of the antimicrobial component even after a long period of time, has high safety, has a wide antibacterial spectrum by selecting the antimicrobial component to be used without adversely affecting other blended components, blend physical properties, and the like, has excellent stability, and can be used at a blending proportion suitable for the detergent application, personal care application, industrial water treatment application, food additive application, electronic device application, and water tank and chemical bath application as water mold suppression for aquatic organisms (such as fish) described above.

Second Embodiment of Functional Particle Dispersion A functional particle dispersion of a second embodiment is characterized by that functional particles are dispersed in water and have a measured zeta potential value of the functional particles in a range of -15 to +15 mV, and the functional particles comprise a (meth)acrylate monomer represented by General Formula (I) provided above, at least one antimicrobial component selected from Group X described above, and at least one type of fragrance component.

The functional particle dispersion of the second embodiment is different from the functional particle dispersion of the first embodiment described above in that the functional particles comprise further at least one type of fragrance component together with using the (meth)acrylate monomer represented by General Formula (I) provided above and at least one of the antimicrobial components selected from Group X described above. The type and the content of the (meth)acrylate monomer represented by General Formula (I) provided above as well as the type and the content of at least one of the antimicrobial components selected from Group X described above and the like are the same as those described in detail for the functional particle dispersion of the first embodiment described above, and thus the explanations thereof are omitted.

For the fragrance component used in the second embodiment, examples thereof include at least one fragrance component selected from a terpene-based fragrance compound, an alcohol-based fragrance compound, a hydrocarbon-based fragrance compound, a phenol-based fragrance compound, an ester-based fragrance compound, a carbonate-based fragrance compound, an aldehyde-based fragrance compound, a ketone-based fragrance compound, an acetal-based fragrance compound, an ether-based fragrance compound, a carboxylic acid-based fragrance compound, a lactone-based fragrance compound, a nitrile-based fragrance compound, a Schiff base-based fragrance compound, a natural essential oil, and a natural extract.

In the present description, a "fragrance compound" in each fragrance means a single compound or a mixture of two or more compounds.

The terpene-based fragrance compound that can be used include monoterpenes having 10 carbon atoms and derivatives thereof (including compounds having 10 or more carbon atoms). The terpene-based fragrance compound is preferably at least one of a terpene-based hydrocarbon, terpene-based alcohol, or a terpene-based ester. Note that a monoterpene is a fragrance compound made of two isoprene units and having 10 carbon atoms, and includes chain-like (acyclic) monoterpenes and cyclic monoterpenes having ring(s). Furthermore, terpene-based fragrances in the present disclosure also include a terpene-based hydrocarbon, a terpene-based alcohol, a terpene-based ester, and a terpene-based aldehyde.

The terpene-based fragrance to be used is not particularly limited as long as the terpene-based fragrance is a fragrance compound having isoprenes as structural units and derivatives thereof and can be appropriately selected based on the purpose. Examples of the terpene-based fragrance include chain-like monoterpenes such as ocimene, myrcene, cosmene, citral, citronellal, citronellol, and linalool, monocyclic monoterpenes such as limonene, menthane, terpinene, phellandrene, terpinolene, and cymene, and bicyclic monoterpenes such as α-pinene, β-pinene, camphene, carene, sabinene, and thujene.

Among these terpene-based fragrances, terpene-based hydrocarbons such as limonene, pinene, myrcene, carene, terpinene, and camphene, terpene-based alcohols such as geraniol, citronellol, linalool, nerol, and terpineol, terpene-based esters such as linalyl acetate, terpinyl acetate, geranyl acetate, neryl acetate. citronellyl acetate, and isobornyl acetate, and terpene-based aldehydes such as citronellal, citral, neral, and perillaldehyde are preferred.

Examples of the alcohol-based fragrance compound include aliphatic alcohols, terpene-based alcohols, aromatic alcohols, and other alcohols. Among these, aliphatic alcohols are preferred.

Examples of the aromatic alcohol-based fragrance compound include phenylethyl alcohol, benzyl alcohol, dimethyl benzyl carbinol, phenylethyl dimethyl carbinol, and phenyl hexanol.

Examples of the aliphatic alcohol-based fragrance compound include cis-3-hexenol, 1-(2,2,6-trimethylcyclohexyl)-3-hexanol, 2-methyl-4-(2,2,3-trimethyl-3-cyclopenten-1-yl)-2-buten-1-ol, ethyl norbornyl cyclohexanol, 4-methyl-3-decen-5-ol, and isobornyl cyclohexanol.

Examples of other alcohol-based fragrance compounds include glycols such as dipropylene glycol, and 4-methyl-2-(2-methylpropyl)tetrahydro-2H-4-pyranol.

Examples of the phenol-based fragrance compound include guaiacol, eugenol, dihydroeugenol, isoeugenol, thymol, p-cresol, vanillin, and ethylvanillin.

Examples of the ester-based fragrance compound include aliphatic carboxylate, aromatic carboxylate, and other carboxylates. Examples of aliphatic carboxylic acid forming the aliphatic carboxylate include linear and branched carboxylic acids having 1 to 18 carbon atoms. Among these, carboxylic acids having 1 to 6 carbon atoms, such as formic acid, acetic acid, and propionic acid, are preferred, and acetic acid is particularly preferred. Examples of aromatic carboxylic acid forming the aromatic carboxylate include benzoic acid, anisic acid, phenylacetic acid, cinnamic acid, salicylic acid, and anthranilic acid. Examples of alcohol forming the aliphatic and aromatic esters include linear and branched aliphatic alcohols having 1 to 5 carbon atoms and fragrance component alcohols described above.

Examples of the formate include linalyl formate, citronellyl formate, and geranyl formate.

Examples of the acetate include ethyl acetate, isoamyl acetate (isopentyl acetate), benzyl acetate, hexyl acetate, cis-3-hexenyl acetate, linalyl acetate, citronellyl acetate, geranyl acetate, neryl acetate, terpinyl acetate, nopyl acetate, bornyl acetate, isobornyl acetate, acetyl eugenol, acetyl isoeugenol, o-tert-butylcyclohexyl acetate, p-tert-butylcyclohexyl acetate, tricyclodecenyl acetate, benzyl acetate, phenylethyl acetate, styralyl acetate, cinnamyl acetate, dimethylbenzylcarbinyl acetate, phenylethylphenyl acetate, 3-pentyltetrahydropyran-4-yl acetate, and p-cresylphenyl acetate.

Examples of the propionate include citronellyl propionate, tricyclodecenyl propionate, allylcyclohexyl propionate, ethyl 2-cyclohexyl propionate, benzyl propionate, and styralyl propionate. Examples of the butyrate include citronellyl butyrate, dimethylbenzylcarbinyl n-butyrate, and tricyclodecenyl isobutyrate. Examples of the valerate include methyl valerate, ethyl valerate, butyl valerate, amyl valerate, benzyl valerate, and phenylethyl valerate. Examples of the hexanoate include methyl hexanoate, ethyl hexanoate, allyl hexanoate, linalyl hexanoate, and citronellyl hexanoate. Examples of the heptanoate include methyl heptanoate and allyl heptanoate. Examples of the nonenoate include methyl 2-nonenoate, ethyl 2-nonenoate, and ethyl 3-nonenoate.

Examples of the benzoate include methyl benzoate, benzyl benzoate, and 3,6-dimethyl benzoate. Examples of the cinnamate include methyl cinnamate and benzyl cinnamate. Examples of the salicylate include methyl salicylate, n-hexyl salicylate, cis-3-hexenyl salicylate, cyclohexyl salicylate, and benzyl salicylate. Examples of the brassylate include ethylene brassylate. Examples of the tiglate include geranyl tiglate, 1-hexyl tiglate, and cis-3-hexenyl tiglate. Examples of the jasmonate include methyl jasmonate and methyl dihydrojasmonate. Examples of the glycidate include methyl 2,4-dihydroxy-ethylmethylphenyl glycidate and 4-methylphenylethyl glycidate. Examples of the anthranilate include methyl anthranilate, ethyl anthranilate, and dimethyl anthranilate. Examples of the glycolate include allyl cyclohexyl glycolate. Examples of other esters include ethyl dihydrocyclogeranate, ethyl-2-cyclohexylpropionate, ethyl tricyclo[5.2.1.02.6]decane-2-carboxylate, methyl jasmonate, methyl dihydrojasmonate, and methyl (2-pentyl-3-oxocyclopentyl)acetate.

Examples of the carbonate-based fragrance compound include cis-3-hexenyl methyl carbonate, methyl-cyclooctyl carbonate, and ethyl-2-t-butylcyclohexyl carbonate.

Examples of the aldehyde-based fragrance compound include n-octanal, n-nonanal, n-decanal, n-dodecanal, 2-methylundecanal, 10-undecenal, citronellal, citral, hydroxycitronellal, 2,4-dimethyl-3-cyclohexene-1-carboxyaldehyde, dimethyl-3-cyclohexenyl-1-carboxyaldehyde, benzaldehyde, phenylacetaldehyde, phenylpropylaldehyde, cinnamaldehyde, dimethyltetrahydrobenzaldehyde, 3-(4-tert-butylphenyl)propanal, hydroxymyrac aldehyde, 2-cyclohexylpropanal, p-tert-butyl-α-methylhydrocinnamic aldehyde, p-isopropyl-α-methylhydrocinnamic aldehyde, 3-(o-(and p-)ethyl phenyl)-2,2-dimethylpropion aldehyde, α-amylcinnamic aldehyde, α-hexylcinnamic aldehyde, heliotropine, alpha-methyl-1,3-benzodioxole-5-propanal, and 2-methyl-3-(p-methoxyphenyl)propanal.

Examples of the ketone-based fragrance compound include methylheptenone, dimethyloctenone, 3-octanone, hexylcyclopentanone, dihydrojasmone, 2,2,5-trimethyl-5-pentylcyclopentanone, 2-(2-(4-methyl-3-cyclohexen-1-yl)propyl)cyclopentanone, ionone, β-ionone, methylionone, methylionone-G, γ-methylionone, damascone, α-damascone, β-damascone, δ-damascone, 1-(2,4,4-trimethyl-2-cyclohexyl)-trans-2-butanone, damascenone, 1-(5,5-dimethyl-1-cyclohexen-1-yl)-4-penten-1-one, irone, 1,2,3,5,6,7-hexahydro-1,1,2,3,3-pentamethyl-4H-inden-4-one, 1-(1,2,3,4,5,6,7,8-octahydro-2,3,8,8-tetramethyl-2-naphthalenyl)-ethan-1-one, 7-methyl-3,4-dihydro-2H-benzodioxepin-3-one, carvone, menthone, acetyl cedrene, isolongifolanone, nootkatone, benzylacetone, raspberry ketone, benzophenone, 6-acetyl-1,1,2,4,4,7-hexamethyltetrahydronaphthalene, methyl β-naphthyl ketone, ethyl maltol, camphor, muscone, 3-methyl-5-cyclopentadecen-1-one, civetone, 8-cyclohexadecenone, methyl nonyl ketone, cis-jasmone, and p-amylcyclohexanone (4-pentylcyclohexanone).

Examples of the acetals include acetaldehyde ethylphenyl propyl acetal, citral diethyl acetal, phenylacetaldehyde glyceryl acetal, and ethyl acetoacetate ethyleneglycol acetal.

Examples of the ethers include ethyl linalool, cedryl methyl ether, estragole, anethole, β-naphthyl methyl ether, β-naphthyl ethyl ether, limonene oxide, rose oxide, nerol oxide, 1,8-cineol, rosefuran, [3aR-(3aα,5aβ,9aα,9bβ)]dodecahydro-3a,6,6,9a-tetramethylnaphto[2,1-b]furan, 3,3,5-trimethylcyclohexyl ethyl ether, hexamethylhexahydrocyclopentabenzopyran, and phenylacetaldehyde dimethyl acetal.

Examples of the carboxylic acid-based fragrance compound include benzoic acid, phenylacetic acid, cinnamic acid, hydrocinnamic acid, butyric acid, and 2-hexenoic acid.

Examples of the lactone-based fragrance compound include ambrettolide, γ-decalactone, δ-decalactone, γ-valerolactone, γ-nonalactone, γ-undecalactone, δ-hexalactone, γ-jasmolactone, whisky lactone, coumarin, cyclopentadecanolide, cyclohexadecanolide, 11-oxahexadecanolide, and butylidenephthalide.

Examples of the nitrile-based fragrance compound include tridecene-2-nitrile, geranyl nitrile, citronellyl nitrile, and dodecanenitrile.

Examples of the Schiff base-based fragrance compound include aurantiol and ligantraal.

Examples of the natural essential oil and natural extract include orange, lemon, lime, bergamot, vanilla, mandarin orange, peppermint, spearmint, lavender, chamomile, rosemary, eucalyptus, sage, basil, rose, rock rose, geranium, jasmine, ylang-ylang, anise, clove, ginger, nutmeg, cardamom, cedar, cypress, vetiver, patchouli, lemongrass, labdanum, grapefruit, and elemi oil.

Among these fragrance components, even more preferably a component that has a high lingering aroma intensity without adversely affecting the encapsulated antimicrobial component, and at least one selected from the following group Y is desired from the viewpoint of ease in production.

Group Y: borneol, eugenol, 4-terpineol, 1,8-cineol, d-limonene, citral R, geraniol, vanillin, 2-phenylethanol, γ-decalactone, linalool, L-perillaldehyde, raspberry ketone, DL-camphor, L-carvyl acetate, aryophyllene, L-citronellol, D-dihydrocarveol, L-dihydrocarveol, dihydroterpineol, dihydroterpineol acetate, linalool oxide, myrtenyl acetate, allyl isothiocyanate, L-carveol, caryophyllene oxide, 1,4-cineole, p-cymene, D-dihydrocarvone, L-dihydrocarvone, D-dihydrocarvyl acetate, L-dihydrocarvyl acetate, elemene, isobornyl acetate, L-limonene, D-limonene oxide, L-limonene oxide, linalool oxide, p-menthane, L-menthol, menthone-90, myrtenal, myrtenol, 3-octylacetate, L-perillyl alcohol, L-perillyl acetate, α-pinene, β-pinene, D-pulegone, α-terpinene, terpineol C, α-terpineol, L-α-terpineol, terpineol-4, terpinolene, α-terpinyl acetate, verbenol, verbenone, 3-carene, carene oxide, hexyl acetate, nerol, ethyl safranate, eugenol acetate, chavicol, estragole, anethole, manzanate, γ-terpinene, cymene, myrcene phellandrene, farnesene, and thujene.

The fragrance components of the group Y described above may contain a solvent that is acceptable as fragrance. In the fragrance industry, a solvent has been used customarily as a diluent to dilute a component that is olfactorily intense, to dissolve a solid component and to facilitate handling of the solid component by handling it as a liquid, or simply to reduce cost of the entire fragrance per unit mass. Typically, suitable solvents include water-soluble solvents including glycols such as propylene glycol dipropylene glycol and butylene glycol, and a solvent that does not mix with water, such as a solvent having water solubility of less than 10 g/L. Examples of the solvent that is acceptable as fragrance include water-insoluble carbohydrate solvents (e.g., Isopar (trade name) family available from Exxon Mobil), benzyl benzoate, isopropyl myristate, benzyl salicylate, dialkyl adipate, citrate (e.g., acetyltriethyl citrate and acetyltributyl citrate), diethyl phthalate, and methyl hydrogenated rosinate. If present, a water-miscible solvent (e.g., a solvent having water solubility of greater than 10 g/100 mL), such as propylene glycol dipropylene glycol and butylene glycol, should be added at a concentration as low as possible.

In the group Y described above, from the viewpoints of stability over time, operability during production, and safety, particularly preferred fragrance components are borneol, eugenol, 4-terpineol, 1,8-cineol, D-limonene, citral, geraniol, vanillin, 2-phenylethanol, γ-decalactone, linalool, L-perillaldehyde, DL-camphor, L-carvyl acetate, aryophyllene, L-citronellol, linalool oxide, myrtenyl acetate, allyl isothiocyanate, 1,4-cineole, isobornyl acetate, D-limonene, linalool oxide, L-menthol, 3-octyl acetate, L-perillyl alcohol, L-perillyl acetate, α-pinene, β-pinene, α-terpinene, terpineol-4, nerol, ethyl safranate, eugenol acetate, chavicol, estragole, anethole, manzanate, γ-terpinene, cymene, myrcene, phellandrene, farnesene, and thujene, and from the viewpoint of ease in being encapsulated into fragrant particles, the solubility in water is 10 mass% or less, preferably 5 mass% or less, and more preferably 3 mass% or less.

The fragrance component of the group Y described above may be a natural fragrance or a synthetic fragrance or may be a mixture of these. In addition to the group Y described above, for example, fragrance components described in "Synthetic Perfume: Chemistry and Product Knowledge" (by Motoichi Indo, published by The Chemical Daily Co., Ltd.) can be also contained in a range that does not impair the effects of the present disclosure.

The functional particle dispersion of the second embodiment comprises at least functional particles dispersed in water and has a measured zeta potential value of the functional particles of being, similarly to the functional particles of the first embodiment, in a range of -15 to +15 mV, or preferably in a range of -10 to +10 mV; the functional particles comprising at least the (meth)acrylate monomer represented by General Formula (I) provided above described in the first embodiment, at least one of the antimicrobial components selected from Group X described above in the first embodiment, and at least one type of the fragrance component described above.

By setting the measured zeta potential value of the functional particles described above to a range of -15 to +15 mV, in a compounding system that is easily affected by ions, even in a compounding system of an aqueous ink that is sensitive to electric charges and that uses polysaccharide fibers, such as cellulose nanofibers (e.g., gel having a hydrogen bond network of CNF) as a thickener, or in a case that an alkali thickener such as an alkali swelling emulsion is used, an initial state in which dispersion of the functional particles is stable can be maintained, the functional particle dispersion has excellent dispersion stability and stability of surface tension, which is low-irritative, has a high degree of freedom in design of compounding, and can be used in various applications.

When the measured zeta potential value described above is outside the range of -15 to +15 mV, the effects of the second embodiment cannot be exhibited. When the measured zeta potential value is less than - 15 mV, change of the compounding system over time becomes larger, and dispersion tends to fail. On the other hand, a measured zeta potential value of greater than +15 mV is not preferred because, similarly, change of the compounding system over time becomes larger, and dispersion tends to fail.

To adjust the measured zeta potential value described above to a range of -15 to + 15 mV, for example, as the functional particles described above are obtained by emulsion polymerization or the like similarly to the first embodiment described above, a functional particle dispersion having a predetermined zeta potential can be obtained by using a nonionic polymerizable surfactant (e.g., type and amount) as a polymerizable surfactant used during the emulsion polymerization.

For the production of the functional particle dispersion of the second embodiment, for example, a dispersion of functional particles having the target measured zeta potential value described above in a range of -15 to +15 mV can be obtained by dissolving at least one of the antimicrobial components selected from Group X described above and at least one of the fragrance components described above in the (meth)acrylate monomer described above (each alone, or two or more; the same applies hereinafter) or a monomer mixture containing the (meth)acrylate monomer described above and a hydrophobic vinyl monomer besides this, using ammonium persulfate, potassium persulfate, hydrogen peroxide, or the like as a polymerization initiator or using a polymerization initiator further using a reducing agent in combination with the aforementioned polymerization initiator, and using a nonionic polymerizable surfactant (type, suitable amount) as a polymerizable surfactant.

Similarly to the first embodiment described above, the nonionic polymerizable surfactant that can be used and the used amount thereof are only required to be an amount that makes the measured zeta potential value in a range of -15 to +15 mV. Although it varies depending on at least one of the antimicrobial components selected from Group X described above to be used and the like, the used amount of the nonionic polymerizable surfactant is 0.1 to 50 mass%, and preferably 5 to 50 mass%, relative to the total amount of the monomers described above.

Furthermore, similarly to the first embodiment described above, also in the second embodiment, in a range that does not impair the effects of the second embodiment, the production may be performed by emulsion polymerization and the like by using a crosslinking agent, such as triallyl isocyanurate, triallyl isocyanurate, polyethylene glycol dimethacrylate, polypropylene glycol dimethacrylate, pentaerythritol acrylate, ditrimethylolpropane acrylate, dipentaerythritol acrylate, tripentaerythritol acrylate, methoxylated bisphenol A methacrylate, ethoxylated bisphenol A dimethacrylate, pentaerythritol methacrylate, ditrimethylolpropane methacrylate, dipentaerythritol methacrylate, tripentaerythritol methacrylate, ethoxylated polyglycerol methacrylate, allyl methacrylate, or 1,4-butanediol diacrylate, and as necessary, a polymerizable surfactant (emulsifier), such as ammonium polyoxyethylene-1-(allyloxymethyl)-alkyl ether sulfate, ether sulfate, ammonium polyoxyethylene nonyl propenyl phenyl ether sulfate, polyoxyethylene nonyl propenyl phenyl ether, ammonium polyacrylate, styrene-maleic acid copolymer ammonium, polyoxyethylene alkyl ether, polyoxyethylene styrenated phenyl ether, polyoxyethylene polyoxypropylene glycol, polyoxyalkylene decyl ether, polyoxyethylene tridecyl ether, an alkyl benzene sulfonate salt, a dioctyl sulfosuccinate salt, sodium lauryl sulfate, polyoxyethylene alkyl ether phosphate ester, polyoxyethylene styrenated phenyl ether phosphate ester, polyoxyethylene styrenated phenyl ether sulfate salt, polyoxyethylene alkyl ether sulfate salt, polyoxyethylene sorbitan monolaurate (polysorbate 20), polyoxyethylene sorbitan palmitate (polysorbate 40), polyoxyethylene sorbitan monostearate (polysorbate 60), or polyoxyethylene sorbitan oleate (polysorbate 80), and after being produced as a liquid dispersion of the functional particle dispersion, the functional particle dispersion can be formed by drying or the like.

The content of the aforementioned crosslinking agent, such as triallyl isocyanurate, is 0 to 50 mass%, and preferably 0.1 to 25 mass%, relative to the total amount of the monomers described above.

Use of the aforementioned crosslinking agent, such as triallyl isocyanurate, is preferred because heat resistance, mechanical properties, hydrolysis resistance, and weather resistance of the functional particle dispersion can be improved.

Similarly to the first embodiment described above, also in the second embodiment, in the emulsion polymerization described above, a dicyclopenta(te)nyl (meth)acrylate monomer or the like may be further mixed with the aforementioned (meth)acrylate monomer and the like in an appropriate amount to carry out the emulsion polymerization. With the polymer obtained by further mixing the dicyclopenta(te)nyl (meth)acrylate monomer followed by the emulsion polymerization, even when water in the liquid dispersion is volatilized, the stability is less likely to be impaired, and a functional particle dispersion having superior stability can be obtained.

Examples of the dicyclopenta(te)nyl (meth)acrylate monomer that can be used include a dicyclopentanyl acrylate monomer, dicyclopentenyl acrylate, a dicyclopentanyl methacrylate monomer, and dicyclopentenyl methacrylate.

Furthermore, in the second embodiment, in the emulsion polymerization described above, in addition to the (meth)acrylate monomer described above, the hydrophobic vinyl monomers described above besides this, and the dicyclopenta(te)nyl (meth)acrylate monomer described above, monomers having a reactive crosslinking group such as an epoxy group, a hydroxymethylamide group, and an isocyanate group, or a polyfunctional monomer having two or more vinyl groups may be blended in an appropriate amount for crosslinking.

In the second embodiment, in the polymer components constituting the functional particle dispersion, the content of the (meth)acrylate monomer described above needs to be 30 mass% or greater, and is preferably 30 to 95 mass%, and more preferably 30 to 70 mass%, relative to the total of the polymer components constituting the functional particle dispersion.

Note that, in the second embodiment, the term "total of the polymer components" refers to polymerizable components constituting the functional particle dispersion, and specifically, it refers to the total amount of the (meth)acrylate monomer used, the other monomer components used, and the crosslinking agent described below.

When the content of the (meth)acrylate monomer described above is 30 mass% or greater relative to the total of the polymer components, the effects of the present disclosure can be exhibited. On the other hand, when this content is less than 30 mass%, stability over time is poor, which is not preferred.

In the polymer components constituting the functional particle dispersion, the content of the other monomer components other than the (meth)acrylate monomer described above is the remainder of the total amount of the (meth)acrylate monomer used and the crosslinking agent described below.

Preferably, the content of the other monomer components is desired to be 0.5 to 70 mass% relative to the total of the polymer components from the viewpoints of further exhibiting the effects of the present disclosure and of dispersibility and reactivity.

In the second embodiment, from the viewpoints of achieving sufficient antimicrobial performance, durable antimicrobial effects, stability, and the like, the content (solids content) of the antimicrobial component described above is desirably 1 mass% or greater, preferably 5 mass% or greater, more preferably 10 to 50 mass%, and particularly preferably 15 to 40 mass%, relative to the total of the polymer components.

When the content of the antimicrobial component is 1 mass% or greater, sufficient antimicrobial performance and durable antimicrobial effects can be exhibited. On the other hand, when the content of the antimicrobial component is less than 1 mass%, the antimicrobial performance is unsatisfactory, and as a result, the effects of the present disclosure cannot be exhibited. Furthermore, in the second embodiment, from the viewpoints of achieving sufficient fragrance performance, a durable lingering aroma effect, stability, and the like, the content (solids content) of the fragrance component described above is desirably 1 mass% or greater, preferably 5 mass% or greater, more preferably 10 to 50 mass%, and particularly preferably 15 to 40 mass%, relative to the total of the polymer components.

When the content of the aroma component is 1 mass% or greater, sufficient fragrance performance and a durable lingering aroma effect can be exhibited. On the other hand, when the content of the fragrance component is less than 1 mass%, the fragrance performance is unsatisfactory, and as a result, the effects of the second embodiment cannot be exhibited.

In the second embodiment, the functional particle dispersion (liquid dispersion) in which functional particles having antimicrobial properties and fragrance performance and having the predetermined zeta potential (the measured zeta potential value is in a range of -15 to +15 mV) are dispersed in water can be obtained by the preferred aspect described above. The preferred aspect described above specifically includes at least dissolving the antimicrobial component selected from Group X described above and at least one of fragrance components in the (meth)acrylate monomer described above and performing emulsion polymerization using the nonionic polymerizable surfactant (e.g., type and amount) or at least dissolving the antimicrobial component and the fragrance component described above after polymerization of a monomer mixture containing the (meth)acrylate monomer described above and another monomer component and performing emulsion polymerization using the nonionic polymerizable surfactant (e.g., type and amount). The production amount of the functional particles having the antimicrobial properties and the fragrance performance in the functional particle dispersion obtained under these production conditions varies depending on blending amounts of the aforementioned (meth)acrylate monomer, the antimicrobial component, the fragrance component, the nonionic polymerizable surfactant, and the like to be used, the polymerization conditions, and the like. From the viewpoints of productivity, workability, efficiency, and the like, the functional particles are preferably produced to have a solids content of 1 to 50 mass%. More preferably, the functional particles are produced to have a solids content of 10 to 40 mass%.

This functional particle dispersion (liquid dispersion) having the antimicrobial properties and the fragrance performance becomes a functional particle dispersion which has strength of antimicrobial performance and long-lasting effects of fragrance performance without adversely affecting other blended components and the like, and is excellent in stability by being made into the functional particle dispersion of the second embodiment, compared with a case where the antimicrobial component or fragrance component described above is used alone. In particular, a functional particle dispersion, which can maintain or improve the antimicrobial effects of the antimicrobial component and the fragrance performance even after a long period of time, can be obtained. Even in a compounding system that is easily affected by ions, a compounding system such as an aqueous ink, or the like, a functional particle dispersion (liquid dispersion) can maintain an initial state in which dispersion is stable, has excellent dispersion stability and stability of surface tension, is low-irritative, and has a high degree of freedom in design of compounding, and thus the functional particle dispersion that can be used in various applications can be obtained.

In addition, in the second embodiment, an oil-soluble (lipophilic) preservative component or the like is preferably used as the antimicrobial component. Thus, in particular, for the problem that the preservative component or the like is likely to adhere to a plastic container, thereby impairing the antimicrobial effects including an antiseptic power over time, the antimicrobial component and the fragrance component are encapsulated in the particles made of the (meth)acrylic acid component and the like of the second embodiment to suppress adhesion to the container wall surface, thereby achieving a specific effect that the antimicrobial effects and the fragrance performance can be maintained even after a long period of time.

In the second embodiment, an average particle size of the functional particles in the resulting functional particle dispersion varies depending on the (meth)acrylate monomer, the type of the other monomers to be used, the contents, the polymerization conditions in the polymerization, and the like. Similarly to the first embodiment described above, the average particle size of the functional particles is preferably 10 to 800 nm, more preferably 20 to 300 nm, and even more preferably 30 to 200 nm.

When the average particle size is within the preferred range described above, excellent storage stability and the like is achieved, and the functional particles having an average particle suitable for various applications described below can be used. In a case where the functional particle dispersion is used for an aqueous ink for writing instruments, a pen core of writing instruments such as a felt-tip pen, a marking pen, or a ballpoint pen is not clogged, and further, excellent storage stability is exhibited.

In the functional particle dispersion of the second embodiment, the content of the functional particles contained in the dispersion is preferably 0.1 to 50 mass%, more preferably 1 to 30 mass%, in terms of solids content, depending on applications and the like described below.

When the content of the functional particles is less than 0.1 mass% in terms of solids content, the effects of the present disclosure cannot be exhibited. On the other hand, when the content of the functional particles exceeds 50 mass%, long-term storage stability tends to decrease.

The functional particle dispersion having the antimicrobial properties and the fragrance performance of the second embodiment configured as described above has high stability and safety, can maintain the initial state in which dispersion is stable, has excellent dispersion stability and stability of surface tension, is low-irritative, has a high degree of freedom in design of compounding, and can be used for various purposes, such as to impart antimicrobial properties to various products such as medical apparatus, baby products, nursing products, bath products, kitchen utensils, tableware, drinking water piping parts, daily hygiene products, household electrical appliances, clothing, building materials, agricultural materials, automobile interior parts, stationery, and ink compositions for writing instruments and inkjet printers.

The functional particle dispersion having the antimicrobial properties and the fragrance performance of the second embodiment configured as described above can be specifically used to impart antimicrobial properties and fragrance performance to various products such as medical apparatus, baby products, nursing products, bath products, kitchen utensils, tableware, drinking water piping parts, daily hygiene products, household electrical appliances, clothing, building materials, agricultural materials, automobile interior parts, stationery, and ink compositions for writing instruments and inkjet printers.

In addition to the aforementioned applications, as specific applications, the functional particle dispersion can be suitably used for detergents such as a laundry detergent, a softener, a household detergent, a dish detergent, and a detergent for a hard surface; personal care applications such as a shampoo, a conditioner, a skin lotion, a milky lotion, a cream, a sunscreen, a foundation, an eye makeup product, a hair coloring product, a nail care product, an antiperspirant, and a toothpaste; a paint, an adhesive agent, a building material, a resin emulsion, a wood preservative, a cement admixture, a boiler, cooling equipment, waste water treatment equipment, industrial water treatment applications such as for industrial water (papermaking process water in a papermaking process, cooling water and washing water for various industries); a medical instrument, a food additive, and electronic devices such as a solar cell module, an organic device, and a heat-ray shielding film; and a water tank and a chemical bath for suppressing water mildew on aquatic organisms (such as fish).

The functional particle dispersion of the second embodiment has high stability and safety, is excellent in the antimicrobial effects of the antimicrobial component and the fragrance performance after a long period of time, and does not affect other blended components, and in addition, can maintain the initial state, in which dispersion is stable, even in a compounding system of a liquid dispersion that is sensitive to electric charges or a compound system such as an aqueous ink, and has excellent dispersion stability and stability of surface tension. Thus, as described above, the functional particle dispersion can be used to impart antimicrobial properties to various products, and in particular, can be suitably used in detergent applications, personal care applications, industrial water treatment applications, food additives, electronic device applications, and water tank and chemical bath applications as water mold suppression for aquatic organisms (such as fish). For example, hereinafter, a case where the functional particle dispersion is used for an aqueous ink composition for writing instruments is described.

The aqueous ink composition for writing instruments according to the second embodiment contains at least the functional particle dispersion having antimicrobial properties and fragrance performance and having the aforementioned configuration, and can contain a colorant and a water-soluble organic solvent in addition to this functional particle dispersion.

The content of the functional particles in the ink composition is preferably 0.1 to 30.0 mass%, and more preferably 1.0 to 15.0 mass%, in terms of solids content relative to the total amount of the ink composition from the viewpoint of exhibiting the effects of the second embodiment without impairing writing performance and from the viewpoint of storage stability.

As the colorant that can be used, similarly to the first embodiment described above, for example, a water-soluble dye, a pigment such as an inorganic pigment, an organic pigment, a plastic pigment, hollow resin particles having voids within the particles that are used as a white pigment, resin particles (pseudo-pigment) dyed with a dye having excellent color development and dispersibility, a thermochromic pigment, a photochromic pigment, or the like can be used. The content of the colorant varies depending on the type of the writing instrument and the like, and is 1 to 30 mass% relative to the total amount of the ink composition.

The water-soluble organic solvent that can be used and the content thereof are the same as those described for the aforementioned first embodiment, and the explanation thereof is omitted.

Besides the functional particle dispersion described above, the colorant, and the water-soluble solvent, the aqueous ink composition for writing instruments of the second embodiment can appropriately contain, as the balance, water as a solvent (e.g., tap water, purified water, distilled water, ion-exchanged water, or pure water) as well as a dispersant, a lubricant, a pH adjuster, a corrosion inhibitor, a thickener, an evaporation inhibitor, a surfactant, or the like, within a range in which the effects of the second embodiment is not impaired, similarly to the first embodiment described above. The explanation of these components is the same as those for the first embodiment described above, and the explanation of the components is omitted.

The aqueous ink composition for writing instruments of the second embodiment can be prepared by appropriately combining the functional particle dispersion described above, the water-soluble solvent, and other components, depending on the application of the ink for writing instruments (e.g., for a ballpoint pen, a marking pen), and then mixing those by stirring using a mixer such as a homomixer, a homogenizer, or a disperser, and, as necessary, further filtering or centrifuging the mixture to remove coarse particles in the ink composition.

In addition, similarly to the first embodiment described above, the pH (25°C) of the aqueous ink composition for writing instruments of the second embodiment is desired to be adjusted to preferably 5 to 10, and more preferably 6 to 9.5, using a pH adjuster from the viewpoints of usability, safety, stability of the ink itself, and matching properties with the ink container.

Similarly to the first embodiment described above, the aqueous ink composition for writing instruments of the second embodiment is loaded in a ballpoint pen, a marking pen, or the like provided with a pen tip such as a ballpoint pen tip, a fiber tip, a felt tip, or a plastic tip. The structures of the ballpoint pen and marking pen are the same as those described for the aforementioned first embodiment, and the explanation thereof is omitted.

In the aqueous ink composition for writing instruments of the second embodiment configured as described above, the functional particle dispersion having the antimicrobial properties and the fragrance performance described above is blended in the aqueous ink composition for writing instruments. Thus, the functional particles have the strength of the antimicrobial properties and the fragrance performance and the long-lasting effects of these in the ink composition without adversely affecting other blended components or the like, and the persistent effect is maintained for a long period of time. In addition, these particles do not impair the storage stability and the writing performance, which can further enhance a degree of freedom in ink design. Accordingly, it is possible to obtain an aqueous ink composition for writing instruments suitable for writing instruments such as a ballpoint pen and a marking pen.

Furthermore, because the measured zeta potential value of the functional particles having the antimicrobial properties and the fragrance performance described above in the dispersion used in the aqueous ink composition for writing instruments of the second embodiment is in the predetermined range, the initial state in which dispersion of the functional particles is stable can be maintained even in a compounding system of an aqueous ink that is sensitive to electric charges and that uses polysaccharide fibers, such as cellulose nanofibers (e.g., gel having a hydrogen bond network of CNF), as a thickener of the ink for the writing instrument. In addition, irritation is less even in a compounding system of an unstable aqueous ink, such as an ionic compounding system, an aqueous ink composition for writing instruments having excellent dispersion stability can be obtained without impairing satisfactory drawn line density.

The case where the functional particle dispersion of the second embodiment is used for the aqueous ink composition for writing instruments has been described above. The functional particle dispersion of the second embodiment is excellent in antimicrobial effects of the antimicrobial component and excellent in the fragrance performance even after a long period of time, has high safety, has a wide antibacterial spectrum by selecting the antimicrobial component to be used without adversely affecting other blended components, blend physical properties, and the like, has excellent stability, and can be used at a blending proportion suitable for the detergent application, personal care application, industrial water treatment application, food additive application, electronic device application, and water tank and chemical bath application as water mold suppression for aquatic organisms (such as fish) described above.

Functional Particle Dispersion of Third Embodiment A functional particle dispersion of the third embodiment is characterized by at least containing functional particles dispersed in water and a measured zeta potential value of the functional particles being in a range of -15 to +15 mV, the functional particles containing a (meth)acrylate monomer represented by General Formula (I) provided above and at least one reducing component selected from Group Z set forth below.

Group Z: polyphenols, copper chlorophyll, flavonoids, anthocyanidins, dibutylhydroxytoluene, and butylhydroxyanisole. The functional particle dispersion of the third embodiment is different from the functional particle dispersion of the first embodiment described above in that the functional particles are obtained by using at least one of the reducing components selected from Group Z described above in place of the antimicrobial component, with the (meth)acrylate monomer represented by General Formula (I) provided above. Because the type and the content of the (meth)acrylate monomer represented by General Formula (I) provided above and the like are the same as those described in detail for the functional particle dispersion of the first embodiment described above, and the explanation and the like thereof are omitted.

The reducing component used in the third embodiment is a component having reduction performance against oxygen (oxygen absorbing ability) and is at least one selected from Group Z: polyphenols, copper chlorophyll, flavonoids, anthocyanidins, dibutylhydroxytoluene (BHT), and butylhydroxyanisole (BHA).

Polyphenols that can be used are those having a phenolic molecule with multiple hydroxy groups. Examples of polyphenols, flavonoids, and anthocyanidins that can be used include catechins (e.g., epicatechin, epigallocatechin, epicatechin gallate, epigallocatechin gallate), tannic acid, tannin, chlorogenic acid, caffeic acid, neochlorogenic acid, cyanidin, proanthocyanidin, thearubidin, rutin, flavonoids (e.g., quercitrin, anthocyanin, flavanone, flavanol, flavonol, isoflavone), ferulic acid, gingerol, anthocyanidins (pelargodinin, cyanidin, delphinidin, peonidin, malvidin, petunidin), flavone, chalcones (e.g., naringenin chalcone), xanthophyll, carnosic acid, eriocitrin, nobiletin, tangeretin, magnolol, honokiol, ellagic acid, lignan, curcumin, coumarin, catechol, procyanidin, theaflavin, rosmarinic acid, xanthone, quercetin, resveratrol, gallic acid, propyl gallate, phlorotannin, piceatannol [5-(dihydroxyphenylethenyl)resorcin] (product name "Pasenol PA"), and resveratrol (3,5,4'-trihydroxy-trans-stilbene).

Preferable reducing components include, from the viewpoints of reducing strength and safety, catechin, tannin, chlorogenic acid, piceatannol, copper chlorophyll, ferulic acid, curcumin, gingerol, rutin, anthocyanin, isoflavone, anthocyanidins (pelargodinin, cyanidin, delphinidin, peonidin, malvidin, petunidin), dibutylhydroxytoluene (BHT), and butylhydroxyanisole (BHA), among which more preferable are chlorogenic acid, tannin, catechin, ferulic acid, piceatannol, dibutylhydroxytoluene (BHT), and butylhydroxyanisole (BHA).

The functional particle dispersion of the third embodiment is characterized by that functional particles being formed from (meth)acrylate monomer represented by General Formula (I) provided above and at least one of the reducing components selected from Group Z described above are dispersed in water and have a measured zeta potential value of the functional particles in a range of -15 to +15 mV, or preferably in a range of -10 to +10 mV.

By setting the measured zeta potential value of the functional particles described above to a range of -15 to +15 mV, even in a compounding system that is easily affected by ions and/or even when polysaccharide fibers, such as cellulose nanofibers that are sensitive to electric charges (e.g., gel having a hydrogen bond network of CNF) are used as a thickener, or even when an alkali thickener such as an alkali swelling emulsion is used, a functional particle dispersion, for which an initial state in which dispersion of the functional particles having reduction properties is stable can be maintained, which is low-irritative, which has a high degree of freedom in design of compounding, and which can be used in various applications, can be obtained.

When the measured zeta potential value described above is outside the range of -15 to +15 mV, the effects of the present disclosure cannot be exhibited. When the measured zeta potential value is less than - 15 mV, change of the compounding system over time becomes larger, and dispersion failure occurs. On the other hand, a measured zeta potential value of greater than +15 mV is not preferred because, similarly, change of the compounding system over time becomes larger, and dispersion failure occurs.

To adjust the measured zeta potential value described above to a range of -15 to + 15 mV, for example, although the functional particles having reduction properties described above are obtained by emulsion polymerization or the like, a functional particle dispersion having reduction properties and having a predetermined zeta potential can be obtained by using a nonionic polymerizable surfactant (e.g., type and amount) as a polymerizable surfactant used during the emulsion polymerization.

For the production of the functional particle dispersion of the third embodiment, for example, a dispersion of functional particles having reduction properties and having the target measured zeta potential value described above in a range of -15 to +15 mV can be obtained by dissolving at least one of the reducing components selected from Group Z described above in the (meth)acrylate monomer described above (each alone, or two or more; the same applies hereinafter) or a monomer mixture containing the (meth)acrylate monomer described above and a hydrophobic vinyl monomer besides this, using ammonium persulfate, potassium persulfate, hydrogen peroxide, or the like as a polymerization initiator or using a polymerization initiator further using a reducing agent in combination with the aforementioned polymerization initiator, and using a nonionic polymerizable surfactant (type, suitable amount) as a polymerizable surfactant.

Similarly to the first embodiment and the second embodiment described above, the nonionic polymerizable surfactant that can be used and the used amount thereof are only required to be an amount that makes the measured zeta potential value in a range of -15 to +15 mV. Although the used amount varies depending on at least one of the antimicrobial components selected from Group X described above to be used and the like, the used amount of these nonionic polymerizable surfactants is 0.1 to 50 mass%, and preferably 5 to 50 mass%, relative to the total amount of the monomers described above.

Furthermore, similarly to the first embodiment and the like described above, also in the third embodiment, in a range that does not impair the effects of the third embodiment, the production may be performed by emulsion polymerization and the like by using a crosslinking agent, such as triallyl isocyanurate, triallyl isocyanurate, polyethylene glycol dimethacrylate, polypropylene glycol dimethacrylate, pentaerythritol acrylate, ditrimethylolpropane acrylate, dipentaerythritol acrylate, tripentaerythritol acrylate, methoxylated bisphenol A methacrylate, ethoxylated bisphenol A dimethacrylate, pentaerythritol methacrylate, ditrimethylolpropane methacrylate, dipentaerythritol methacrylate, tripentaerythritol methacrylate, ethoxylated polyglycerol methacrylate, allyl methacrylate, or 1,4-butanediol diacrylate, and as necessary, a polymerizable surfactant (emulsifier), such as ammonium polyoxyethylene-1-(allyloxymethyl)-alkyl ether sulfate, ether sulfate, ammonium polyoxyethylene nonyl propenyl phenyl ether sulfate, polyoxyethylene nonyl propenyl phenyl ether, ammonium polyacrylate, styrene-maleic acid copolymer ammonium, polyoxyethylene alkyl ether, polyoxyethylene styrenated phenyl ether, polyoxyethylene polyoxypropylene glycol, polyoxyalkylene decyl ether, polyoxyethylene tridecyl ether, an alkyl benzene sulfonate salt, a dioctyl sulfosuccinate salt, sodium lauryl sulfate, polyoxyethylene alkyl ether phosphate ester, polyoxyethylene styrenated phenyl ether phosphate ester, polyoxyethylene styrenated phenyl ether sulfate salt, polyoxyethylene alkyl ether sulfate salt, polyoxyethylene sorbitan monolaurate (polysorbate 20), polyoxyethylene sorbitan palmitate (polysorbate 40), polyoxyethylene sorbitan monostearate (polysorbate 60), or polyoxyethylene sorbitan oleate (polysorbate 80), and after being produced as a liquid dispersion of the functional particle dispersion, the functional particle dispersion can be formed by drying or the like.

The content of the aforementioned crosslinking agent, such as triallyl isocyanurate, is 0 to 50 mass%, and preferably 0.1 to 25 mass%, relative to the total amount of the monomers described above.

Use of the aforementioned crosslinking agent, such as triallyl isocyanurate, is preferred because heat resistance, mechanical properties, hydrolysis resistance, and weather resistance of the functional particle dispersion can be improved.

Similarly to the first embodiment described above, also in the third embodiment, in the emulsion polymerization described above, a dicyclopenta(te)nyl (meth)acrylate monomer may be further mixed with the aforementioned (meth)acrylate monomer and the like in an appropriate amount to carry out the emulsion polymerization. With the polymer obtained by further mixing the dicyclopenta(te)nyl (meth)acrylate monomer followed by the emulsion polymerization, even when water in the liquid dispersion is volatilized, the stability is less likely to be impaired, and a functional particle dispersion having superior stability can be obtained.

Examples of the dicyclopenta(te)nyl (meth)acrylate monomer that can be used include a dicyclopentanyl acrylate monomer, dicyclopentenyl acrylate, a dicyclopentanyl methacrylate monomer, and dicyclopentenyl methacrylate.

Furthermore, in the third embodiment, in the emulsion polymerization described above, in addition to the (meth)acrylate monomer described above, the hydrophobic vinyl monomers described above besides this, and the dicyclopenta(te)nyl (meth)acrylate monomer described above, monomers having a reactive crosslinking group such as an epoxy group, a hydroxymethylamide group, and an isocyanate group, or a polyfunctional monomer having two or more vinyl groups may be blended in an appropriate amount for crosslinking.

In the third embodiment, in the polymer components constituting the functional particle dispersion, the content of the (meth)acrylate monomer described above needs to be 30 mass% or greater, and is preferably 30 to 95 mass%, and more preferably 30 to 70 mass%, relative to the total of the polymer components constituting the functional particle dispersion.

Note that, in the third embodiment, the term "total of the polymer components" refers to polymerizable components constituting the functional particle dispersion, and specifically, it refers to the total amount of the (meth)acrylate monomer used, the other monomer components used, and the crosslinking agent described below.

When the content of the (meth)acrylate monomer described above is 30 mass% or greater relative to the total of the polymer components, the effects of the present disclosure can be exhibited. On the other hand, when this content is less than 30 mass%, stability over time is poor, which is not preferred.

In the polymer components constituting the functional particle dispersion, the content of the other monomer components other than the (meth)acrylate monomer described above is the remainder of the total amount of the (meth)acrylate monomer used and the crosslinking agent described below.

Preferably, the content of the other monomer components is desired to be 0.5 to 70 mass% relative to the total of the polymer components from the viewpoints of further exhibiting the effects of the present disclosure and of dispersibility and reactivity.

In the third embodiment, from the viewpoints of achieving sufficient reduction performance, a durable reduction effect, stability, and the like, the content (solids content) of the reducing component described above is desirably 1 mass% or greater, preferably 5 mass% or greater, more preferably 10 to 50 mass%, and particularly preferably 15 to 40 mass%, relative to the total of the polymer components.

When the content of the reducing component is 1 mass% or greater, sufficient reduction performance and a durable reduction effect can be exhibited. On the other hand, when the content of the reducing component is less than 1 mass%, the reduction performance is unsatisfactory, and as a result, the effects of the third embodiment cannot be exhibited.

In the third embodiment, from the viewpoint of further exhibiting a wide antibacterial spectrum and antimicrobial effects, functional particles containing an antimicrobial component together with the reducing component described above may be formed.

As the antimicrobial component that can be used, a known antimicrobial component can be used. Preferably, the antimicrobial component is highly safe without adversely affecting the encapsulated reducing component, and is a compound having antibacterial properties over a long period of time. The preservative component is at least one selected from Group X described above for the first embodiment, for example.

Among Group X described above for the aforementioned first embodiment, the following components are desirable as more preferable antimicrobial components, from the viewpoints of stability over time, relatively easy availability and low cost, and safety: an iodopropargyl compound, 1,2-benzisothiazolin-3-one, 2,3,5,6-tetrachloro-4(methylsulfonyl)pyridine, sodium benzoate, sodium dehydroacetate, potassium sorbate, cresol, methylisothiazolinone, chloromethylisothiazolinone, octylisothiazolinone, dichlorooctylisothiazolinone, hexahydro-1,3,5-tris(2-hydroxyethyl)-1,3,5-triazine, 2-bromo-2-nitropropane-1,3-diol, sodium 2-pyridinethiol-1-oxide, sodium pyrithione, 2-(4-thiozolyl)benzimidazole, 4-terpineol, 1,8-cineol, diisothiocyanate, isopropylmethylphenol, 2-methyl-4-isothiazolin-3-one, citral, eugenol, allyl isothiocyanate, D-limonene, tannic acid, ethylparaben, benzalkonium chloride, glycerin fatty acid ester, salicylic acid, ethyl parahydroxybenzoate, butyl parahydroxybenzoate, propyl parahydroxybenzoate, methyl parahydroxybenzoate, hinokitiol, phenylethyl alcohol, phenethyl alcohol, phenoxyethanol, butylparaben, propylparaben, , methylparaben, and 2-(4-thiazolyl)benzimidazole.

In the third embodiment, from the viewpoints of achieving durable antimicrobial effects, stability, and the like, the content (solids content) of the antimicrobial component is desirably 1 mass% or greater, preferably 5 mass% or greater, more preferably 10 to 50 mass%, and particularly preferably 15 to 40 mass%, relative to the total of the polymer components.

When the content of the antimicrobial component is 1 mass% or greater, more sufficient antimicrobial effects (oxygen absorbing ability) and a durable reduction effect can be exhibited. On the other hand, when the content of the preservative component is less than 1 mass%, the additional effect of the third embodiment containing the antimicrobial component cannot be exhibited.

In the third embodiment, the functional particle dispersion (liquid dispersion) in which functional particles having the reduction properties as well as the reduction properties and the antimicrobial effects and having the predetermined zeta potential (the measured zeta potential value is in a range of -15 to +15 mV) are dispersed in water can be obtained by the preferred aspect described above. The preferred aspect described above specifically includes at least dissolving the reducing component selected from Group Z described above in the (meth)acrylate monomer described above, dissolving also the antimicrobial component, and performing emulsion polymerization using the nonionic polymerizable surfactant (e.g., type and amount) or at least dissolving the reducing component (and the antimicrobial component) described above after polymerization of a monomer mixture containing the (meth)acrylate monomer described above and another monomer component and performing emulsion polymerization using the nonionic polymerizable surfactant (e.g., type and amount). The production amount of the functional particles having the reduction performance and the like in the functional particle dispersion obtained under these production conditions varies depending on blending amounts of the aforementioned (meth)acrylate monomer, the reducing component or the antimicrobial component, the nonionic polymerizable surfactant, and the like to be used, the polymerization conditions, and the like. From the viewpoints of productivity, workability, efficiency, and the like, the functional particles are preferably produced to have a solids content of 1 to 50 mass%. More preferably, the functional particles are produced to have a solids content of 10 to 40 mass%.

This functional particle dispersion (liquid dispersion) having the reduction properties becomes a functional particle dispersion which has strength and long-lasting effects of reduction performance without adversely affecting other blended components and the like, and is excellent in stability by being made into the functional particle dispersion of the third embodiment, compared with a case where the functional particles described above are used alone. In particular, a functional particle dispersion, which can maintain or improve the reduction effect of the reducing component even after a long period of time, can be obtained. This functional particle dispersion (liquid dispersion) can maintain the initial state, in which dispersion is stable, even in a compounding system that is easily affected by ions or a compound system such as an aqueous ink, is low-irritative, has unprecedentedly a high degree of freedom in design, and can be suitably used in various applications.

In addition, in the third embodiment, an oil-soluble (lipophilic) reducing component or the like is preferably used as the reducing component. Thus, in particular, for the problem that the reducing component or the like easily adhere to a plastic container, thereby impairing the reduction performance over time, the antimicrobial component and the fragrance component are encapsulated in the particles made of the (meth)acrylic acid component and the like of the third embodiment to suppress adhesion to the container wall surface, thereby achieving a specific effect that the reduction effect can be maintained even after a long period of time.

In the third embodiment, an average particle size of the functional particles in the resulting functional particle dispersion varies depending on the (meth)acrylate monomer, the type of the other monomers to be used, the contents, the polymerization conditions in the polymerization, and the like. Similarly to the first embodiment described above, the average particle size of the functional particles is preferably 10 to 800 nm, more preferably 20 to 300 nm, and even more preferably 30 to 200 nm.

When the average particle size is within the preferred range described above, excellent storage stability and the like is achieved, and the functional particles having an average particle suitable for various applications described below can be used. In a case where the functional particle dispersion is used for an aqueous ink for writing instruments, a pen core of writing instruments such as a felt-tip pen, a marking pen, or a ballpoint pen is not clogged, and further, excellent storage stability is exhibited.

In the functional particle dispersion of the third embodiment, the content of the functional particles contained in the dispersion is preferably 0.1 to 50 mass%, more preferably 1 to 30 mass%, in terms of solids content, depending on applications and the like described below.

When the content of the functional particles is less than 0.1 mass% in terms of solids content, the effects of the present disclosure cannot be exhibited. On the other hand, when the content of the functional particles exceeds 50 mass%, long-term storage stability tends to decrease.

The functional particle dispersion of the third embodiment configured as described above has reduction performance or has reduction performance and antimicrobial effects, has high stability and safety, can maintain the initial state in which dispersion is stable, is low-irritative, has unprecedentedly a high degree of freedom in design of compounding, and can be used for various purposes, such as to impart reduction performance or reduction performance and antimicrobial performance to various products such as medical apparatus, baby products, nursing products, bath products, kitchen utensils, tableware, drinking water piping parts, daily hygiene products, household electrical appliances, clothing, building materials, agricultural materials, automobile interior parts, stationery, and ink compositions for writing instruments and inkjet printers.

In addition to the aforementioned applications, as specific applications, the functional particle dispersion can be suitably used for detergents such as a laundry detergent, a softener, a household detergent, a dish detergent, and a detergent for a hard surface; personal care applications such as a shampoo, a conditioner, a skin lotion, a milky lotion, a cream, a sunscreen, a foundation, an eye makeup product, a hair coloring product, a nail care product, an antiperspirant, and a toothpaste; a paint, an adhesive agent, a building material, a resin emulsion, a wood preservative, a cement admixture, a boiler, cooling equipment, waste water treatment equipment, industrial water treatment applications such as for industrial water (papermaking process water in a papermaking process, cooling water and washing water for various industries); a medical instrument, a food additive, and electronic devices such as a solar cell module, an organic device, and a heat-ray shielding film; and a water tank and a chemical bath for suppressing water mildew on aquatic organisms (such as fish).

The functional particle dispersion of the third embodiment has high stability and safety, has reduction performance or reduction performance and antimicrobial effects, can maintain the initial state in which dispersion is stable, is low-irritative, has unprecedentedly a high degree of freedom in design, and does not affect other blended components, and in addition, causes less change over time of the dispersion stability even in a compounding system of a liquid dispersion that is sensitive to electric charges or a compound system such as an aqueous ink and can maintain the initial state. Thus, as described above, the functional particle dispersion can be used to impart reduction performance or reduction performance and antimicrobial effects to various products, and in particular, can be suitably used in detergent applications, personal care applications, industrial water treatment applications, food additives, electronic device applications, and water tank and chemical bath applications as water mold suppression for aquatic organisms (such as fish). For example, hereinafter, a case where the functional particle dispersion is used for an aqueous ink composition for writing instruments is described.

The aqueous ink composition for writing instruments according to the third embodiment contains at least the functional particle dispersion having reduction properties and having the aforementioned configuration, and can contain a colorant and a water-soluble organic solvent in addition to this functional particle dispersion.

The content of the functional particles in the ink composition is preferably 0.1 to 30.0 mass%, and more preferably 1.0 to 15.0 mass%, in terms of solids content relative to the total amount of the ink composition, from the viewpoint of exhibiting the effects of the third embodiment without impairing writing performance and from the viewpoint of storage stability.

As the colorant that can be used, similarly to the first embodiment described above, for example, a water-soluble dye, a pigment such as an inorganic pigment, an organic pigment, a plastic pigment, hollow resin particles having voids within the particles that are used as a white pigment, resin particles (pseudo-pigment) dyed with a dye having excellent color development and dispersibility, a thermochromic pigment, a photochromic pigment, or the like can be used. The content of the colorant varies depending on the type of the writing instrument and the like, and is 1 to 30 mass% relative to the total amount of the ink composition.

The water-soluble organic solvent that can be used and the content thereof are the same as those described for the aforementioned first embodiment, and the explanation thereof is omitted.

Besides the particles having the aforementioned properties, the colorant, and the water-soluble solvent, the aqueous ink composition for writing instruments of the third embodiment can appropriately contain, as the balance, water as a solvent (e.g., tap water, purified water, distilled water, ion-exchanged water, or pure water) as well as a dispersant, a lubricant, a pH adjuster, a corrosion inhibitor, a thickener, an evaporation inhibitor, a surfactant, or the like, within a range in which the effects of the second embodiment is not impaired, similarly to the first embodiment described above. The explanation of these components is the same as those for the first embodiment described above, and the explanation thereof is omitted.

The aqueous ink composition for writing instruments of the third embodiment can be prepared by appropriately combining the functional particle dispersion described above, the water-soluble solvent, and other components, depending on the application of the ink for writing instruments (e.g., for a ballpoint pen, a marking pen), and then mixing those by stirring using a mixer such as a homomixer, a homogenizer, or a disperser, and, as necessary, further filtering or centrifuging the mixture to remove coarse particles in the ink composition.

In addition, similarly to the first embodiment described above, the pH (25°C) of the aqueous ink composition for writing instruments of the third embodiment is desired to be adjusted to preferably 5 to 10, and more preferably 6 to 9.5, using a pH adjuster from the viewpoints of usability, safety, stability of the ink itself, and matching properties with the ink container.

Similarly to the first embodiment described above, the aqueous ink composition for writing instruments of the third embodiment is loaded in a ballpoint pen, a marking pen, or the like provided with a pen tip such as a ballpoint pen tip, a fiber tip, a felt tip, or a plastic tip. The structures of the ballpoint pen and marking pen are the same as those described for the aforementioned first embodiment, and the explanation thereof is omitted.

In the aqueous ink composition for writing instruments of the third embodiment configured as described above, the functional particle dispersion having the reduction properties described above is blended in the aqueous ink composition for writing instruments. Thus, the functional particles have the strength and long-lasting effects of the reduction performance in the ink composition without adversely affecting other blended components or the like, and the persistent effect is maintained for a long period of time. In addition, these particles do not impair the storage stability and the writing performance, which can further enhance a degree of freedom in ink design. Accordingly, it is possible to obtain an aqueous ink composition for writing instruments suitable for writing instruments such as a ballpoint pen and a marking pen.

Furthermore, because the measured zeta potential value of the functional particles having the reduction properties described above in the dispersion used in the aqueous ink composition for writing instruments of the third embodiment is in the predetermined range, the initial state in which dispersion of the functional particles is stable can be maintained even in a compounding system of an aqueous ink that is sensitive to electric charges and that uses polysaccharide fibers, such as cellulose nanofibers (e.g., gel having a hydrogen bond network of CNF), as a thickener of the ink for the writing instrument. In addition, irritation is less even in a compounding system of an unstable aqueous ink, such as an ionic compounding system, an aqueous ink composition for writing instruments having excellent dispersion stability can be obtained without impairing satisfactory drawn line density.

The case where the functional particle dispersion of the third embodiment is used for the aqueous ink composition for writing instruments has been described above. The functional particle dispersion of the third embodiment is excellent in the reduction properties or excellent in the reduction properties and the antimicrobial effects even after a long period of time, has high safety, has a wide antibacterial spectrum by selecting the antimicrobial component to be used without adversely affecting other blended components, blend physical properties, and the like, has excellent stability, and can be used at a blending proportion suitable for the detergent application, personal care application, industrial water treatment application, food additive application, electronic device application, and water tank and chemical bath application as water mold suppression for aquatic organisms (such as fish) described above.

### Cosmetic: Functional Particle Dispersion-Containing Cosmetic

The cosmetic of the present disclosure is characterized by containing each of the functional particle dispersions of the first embodiment to the third embodiment described above and specifically characterized by 1) at least containing the functional particle dispersion having the antimicrobial properties and having the aforementioned composition, 2) at least containing the functional particle dispersion having the antimicrobial properties and aroma function and having the aforementioned composition, and 3) containing the functional particle dispersion having excellent reduction properties or reduction properties and antimicrobial effects and having the aforementioned composition. Examples of the cosmetic include skin care cosmetics such as a skin lotion, a milky lotion, a beauty essence, a cream, a body lotion, a body powder, and a body gel, makeup cosmetics such as a foundation, a sunscreen, a makeup base, an eye shadow, an eyeliner, a blusher, a lipstick, a mascara, and a face powder, hair cosmetics such as a hair mist, a hair oil, a hair gel, a nail care product, a shampoo, a rinse, a conditioner, a treatment, a spray, and a mousse, and deodorant cosmetics. For each of these cosmetic applications (e.g., skin care cosmetics, makeup cosmetics, hair cosmetics, deodorant cosmetics), a base component, a colorant (coloring material), a water-soluble resin, a lower alcohol, a humectant (softener, emollient), an astringent (antiperspirant), a refrigerant, a whitening agent, an ultraviolet absorber, and the like can be used. The components of the cosmetic are not limited to these examples, and all materials that are obvious to a person skilled in the art can be used.

The content of the functional particles in these cosmetics is preferably 0.1 to 30.0 mass%, and more preferably 1.0 to 20.0 mass%, in terms of solids content relative to the total amount of the cosmetic from the viewpoints of exhibiting the effects of each of the embodiments, storage stability, each cosmetic application, and the like.

The cosmetic of the present disclosure is used by being loaded in a cosmetic applicator having a structure and a form that are ordinarily used in the skin care cosmetics, makeup cosmetics, hair cosmetics, and deodorant cosmetics described above and the like, and the form of the cosmetic to be used may be a liquid, a solid, a semisolid, or a gel.

For example, use of a cosmetic applicator having excellent usability, simplicity, and applicability is desired. The cosmetic applicator includes, as the application means, a brush (brush pen), a pen core, or an application body that is an application means formed of rubber, an elastomer, or a closed-cell foam having restorability, and including a container for charging the liquid cosmetic or the like. Specifically, the applicator has a mechanism that is a rotary extension type, a cosmetic-containing applicator type, or a collector-type applicator type.

As illustrated in FIG. 1, a cosmetic applicator of a rotary extension type includes an application portion 30 made of a brush (brush pen) provided in front of a cosmetic container serving as a reservoir 11 configured to discharge, by a liquid pressing mechanism 10, the cosmetic (liquid type) contained in front of the liquid pressing mechanism 10.

The liquid pressing mechanism 10 has such a configuration that a feeding member 13 disposed at a rear end portion of a barrel body 12 is relatively rotated with respect to the barrel body 12 in a circumferential direction to feed the liquid cosmetic in the container (reservoir) 11, and then the liquid cosmetic is supplied to the application portion 30.

The liquid pressing mechanism 10 of the applicator has the feeding member 13 rotatably fitted to the rear end of the barrel body 12; a driving cylinder 15 configured to transmit the rotational force of the feeding member 13 by a user to a screw rod 14; a screw body 16 fixed to the barrel body 12 and screwed with the screw rod 14; the screw rod 14 rotatably engaged with a piston body 17 at its tip; and the piston body 17 configured to slide in the reservoir 11 of the barrel body 12. The rotation of the feeding member 13 is transmitted to the screw rod 14 via the driving cylinder 15, and the screw rod 14 and the piston body 17 are advanced through a female screw of the nut-like screw body 16 by the rotation of the screw rod 14, to thereby feed the liquid cosmetic from the reservoir 11 to the application portion 30.

As illustrated in FIG. 1, an operating portion of the feeding member 13 is rotatably fitted into the rear end portion of the barrel body 12 and is exposed therefrom, the operating portion having a cylindrical shape closed by fitting a crown 13a into the rear end thereof. The driving cylinder 15 is fitted into the feeding member 13 and fixed in the rotating direction. The screw body 16 is mounted in the driving cylinder 15 so as to be fixed in the rotating direction and to be relatively movable in the axial direction. Reference numeral 13b denotes a spring member that rearwardly biases the feeding member 13 serving as a rotating body.

In this applicator, a sealing portion 18, a joint member 19, a front barrel 20, and the application portion 30 are fitted into the front end portion 12a of the barrel body 12. The liquid cosmetic is stored in the reservoir 11 of the barrel body 12, and the liquid cosmetic fed out from the reservoir 11 is discharged to the application portion 30 through a flow path in the joint member 19 to be applicable. The applicator is configured such that, after use, a cap 40 can be attached to the front barrel 20 so as to cover the application portion 30 and the front barrel 20.

In FIG. 1, reference numeral 21 denotes a stirring ball configured to stir the liquid cosmetic in the reservoir 11 by reciprocating motion, and reference numeral 22 denotes a sealing ball. Reference numeral 41 denotes an inner cap in the cap 40, and reference numeral 42 denotes a spring for biasing the inner cap rearward. The stirring ball 21 may be omitted.

Reference numeral 23 denotes a stopper having a ring-shaped portion mounted between the rear end of the front barrel 20 and the front surface of a stepped portion of the front end portion 12a of the barrel body 12, in order to locate the sealing portion 18, the joint member 19, the front barrel 20, and the application portion 30 at a position for closing the flow path of the liquid cosmetic toward the application portion 30 when not in use. In the stopper 23, a part of a ring-shaped portion is cut off, and a knob piece is integrally formed on the opposite side of the cut-off portion. When the knob piece is pulled, the diameter of the ring-shaped portion is enlarged from the cut-off portion, to be removed from between the rear end of the front barrel 20 and the front end portion 12a of the barrel body 12.

As illustrated in FIG. 1, when not in use, the sealing ball 22 fits into and seals the inner diameter portion of the sealing portion 18 serving as a sealing ball receiver, and thus the liquid cosmetic does not flow toward the application portion 30. At the time of use, when a user pulls out the stopper 23 from the barrel body 12 and pushes the front barrel 20 to the rear end side, a small-diameter portion of the rear end of the joint member 19 abuts against the sealing ball 22, and the sealing ball 22 is removed from the inner diameter portion of the sealing portion 18 and enters into the reservoir 11. Then, the liquid cosmetic in the reservoir 11 flows into a liquid flow channel of the application portion 30 from an inner diameter portion of the joint member 19 and is supplied into the application portion 30 from its inside. Then, the liquid cosmetic is applicable to a target portion.

The cosmetic applicator of a rotary extension type of FIG. 1 is an example and, without limitation to this, cosmetic applicators having various other structures and forms can be also used.

In the cosmetic of the present disclosure, in a case where the functional particle dispersion of each of the embodiments described above is used, for example, when the functional particles having the specific physical properties of the first embodiment, the functional particles containing at least the (meth)acrylate monomer represented by General Formula (I) provided above and at least one of the antimicrobial components selected from Group X described above, are used in a cosmetic, long-lasting effects of the antimicrobial effects can be achieved without deteriorating the dispersion stability, fixation, water resistance, and sebum resistance. In addition, adjustment for the appropriate components can be performed based on the use such as application to skin or application to hair by changing the type of the antimicrobial component to be added to the cosmetic, and thus the commercial value can be improved.

Furthermore, in a case of the functional particles having the specific physical properties of the second embodiment, the functional particles at least containing the (meth)acrylate monomer represented by General Formula (I) provided above, at least one of the antimicrobial components selected from Group X described above, and the fragrance component, the commercial value can be improved by changing the type of the fragrance perfuming the cosmetic and the type of the fragrance blended into the cosmetic together with the antimicrobial effects by the antimicrobial component, and long-lasting effects of the antimicrobial and fragrance effects can be achieved without deteriorating the dispersion stability, fixation, water resistance, and sebum resistance. When the fragrance added to the cosmetic and the functional particle-containing fragrance having similar fragrance note are used, by touching a part where it is applied after application of the cosmetic, it feels that the aroma comes back and/or gets intensified. When the fragrance added to the cosmetic and the fragrance encapsulated in the functional particles have different fragrance note, by touching a part where it is applied after application of the cosmetic, different aroma can be felt. Furthermore, when no fragrance is added to the cosmetic but blending of the functional particles into the cosmetic is performed, no aroma is felt after application of the cosmetic but the aroma is felt when the applied part is touched. Furthermore, when the functional particles having the specific physical properties of the third embodiment are used in cosmetics, the functional particles containing the (meth)acrylate monomer represented by General Formula (I) provided above and at least one of the reducing components selected from Group Z described above, long-lasting effects of the oxidation degradation suppression and bubble suppression in the ink component can be achieved without deteriorating the dispersion stability, fixation, water resistance, and sebum resistance.

### Examples

Next, the present disclosure (the first embodiment to the third embodiment) will be described in more detail using Production Examples, Examples, and Comparative Examples, but the present disclosure is not limited to the following Examples.

First Embodiment: Production Examples 1 to 9: Functional Particle Dispersions Having Antimicrobial Properties (Particles 1 to 9) Functional particle dispersions having antimicrobial properties were produced in the following Production Examples 1 to 9. Note that the "parts" described below refers to parts by mass. The antimicrobial component is a solids content.

### Production Example 1

A 2-liter flask was equipped with a stirrer, a reflux condenser, a thermometer, a nitrogen gas inlet tube, and a 1000-mL separatory funnel for addition of a monomer and set in a hot water bath, and 344.5 parts of distilled water, 5 parts of glycerol monomethacrylate [BLEMMER GLM, available from NOF Corporation], 5 parts of sodium 2-sulfoethyl methacrylate [acrylic ester SEM-Na, available from Mitsubishi Chemical Corporation], 40 parts of a nonionic polymerizable surfactant [available from Kao Corporation, LATEMUL PD-430], and 0.5 parts of ammonium persulfate were placed in the flask, and the internal temperature was raised to 50°C while nitrogen gas was introduced.

On the other hand, 20 parts of an antimicrobial component [iodopropargyl compound: 3-iodo-2-propynylcarbamate, (Omacide IPBC 100) available from LONZA K.K.], 14 parts of tannin [(Tannic acid S) available from Fuji Chemical Industries, Co., Ltd.], and 10 parts of a crosslinking agent [triallyl isocyanurate, (TAIC) available from Nippon Kasei Chemical Company Limited] were mixed with a monomer mixture containing 55 parts of cyclohexyl methacrylate monomer and 20 parts of n-butyl methacrylate to prepare a solution.

The prepared solution was added to the aforementioned flask maintained at a temperature of approximately 50°C from the aforementioned separatory funnel over 3 hours with stirring to carry out emulsion polymerization. Furthermore, the solution was aged for 5 hours to terminate the polymerization, and thus a functional particle dispersion (liquid dispersion) having antimicrobial properties (particles 1) was obtained.

The content of the methacrylate monomer was 54.8 mass% relative to the total of the polymer components constituting the functional particles, and the content of the antimicrobial component was 12.9 mass% relative to the total of the polymer components. The functional particles had an average particle size of 70 nm. The zeta potential of the functional particles was -5.8 mV.

### Production Example 2

A functional particle dispersion (liquid dispersion) having antimicrobial properties (particles 2) was obtained in the same manner as in the aforementioned Production Example 1, except that 334.5 parts of distilled water was used, the amount of cyclohexyl methacrylate monomer was 30 parts, the amount of n-butyl methacrylate was 45 parts, and 30 parts of an antimicrobial component [iodopropargyl compound: 3-iodo-2-propynylcarbamate, (Omacide IPBC 100) available from LONZA K.K.] was used as the antimicrobial component.

The content of the methacrylate monomer was 51.5 mass% relative to the total of the polymer components constituting the functional particles, and the content of the antimicrobial component was 18.2 mass% relative to the total of the polymer components. The functional particles had an average particle size of 72 nm. The zeta potential of the functional particles was -8.8 mV.

### Production Example 3

A functional particle dispersion (liquid dispersion) having antimicrobial properties (particles 3) was obtained in the same manner as in the aforementioned Production Example 1, except that 314.5 parts of distilled water was used, the amount of cyclohexyl methacrylate monomer was 60 parts, the amount of n-butyl methacrylate was 35 parts, and 30 parts of an antimicrobial component [phenoxyethanol, available from Yokkaichi Chemical, Co., Ltd.] was used as the antimicrobial component.

The content of the methacrylate monomer was 56.8 mass% relative to the total of the polymer components constituting the functional particles, and the content of the antimicrobial component was 16.2 mass% relative to the total of the polymer components. The functional particles had an average particle size of 108 nm. The zeta potential of the functional particles was +10.1 mV.

### Production Example 4

A functional particle dispersion (liquid dispersion) having antimicrobial properties (particles 4) was obtained in the same manner as in the aforementioned Production Example 1, except that 324.5 parts of distilled water was used, the amount of cyclohexyl methacrylate monomer was 30 parts, the amount of n-butyl methacrylate was 45 parts, and 40 parts of an antimicrobial component [paraben, available from Ueno Fine Chemicals Industry, Ltd.] was used as the antimicrobial component.

The content of the methacrylate monomer was 48.6 mass% relative to the total of the polymer components constituting the functional particles, and the content of the antimicrobial component was 22.9 mass% relative to the total of the polymer components. The functional particles had an average particle size of 124 nm. The zeta potential of the functional particles was +3.8 mV.

### Production Example 5

A functional particle dispersion (liquid dispersion) having antimicrobial properties (particles 5) was obtained in the same manner as in the aforementioned Production Example 1, except that 349.5 parts of distilled water was used, the amount of cyclohexyl methacrylate monomer was 30 parts, the amount of n-butyl methacrylate was 45 parts, and 15 parts of an antimicrobial component [chloromethylisothiazolinone/methylisothiazoline (CMIT/MIT), available from Daiwa Chemical Industries, Co., Ltd.] was used as the antimicrobial component.

The content of the methacrylate monomer was 56.7 mass% relative to the total of the polymer components constituting the functional particles, and the content of the antimicrobial component was 10.0 mass% relative to the total of the polymer components. The functional particles had an average particle size of 95 nm. The zeta potential of the functional particles was -7.6 mV.

### Production Example 6

A functional particle dispersion (liquid dispersion) having antimicrobial properties (particles 6) was obtained in the same manner as in the aforementioned Production Example 1, except that the aforementioned Production Example 1, in which 334.5 parts of distilled water was used, the amount of cyclohexyl methacrylate monomer was 30 parts, the amount of n-butyl methacrylate was 45 parts, and 30 parts of an antimicrobial component [benzisothiazolinone (BIT), available from Daiwa Chemical Industries, Co., Ltd.] was used as the antimicrobial component. The content of the methacrylate monomer was 51.5 mass% relative to the total of the polymer components constituting the functional particles, and the content of the antimicrobial component was 18.2 mass% relative to the total of the polymer components. The functional particles had an average particle size of 60 nm. The zeta potential of the functional particles was -2.1 mV.

### Production Example 7

A functional particle dispersion (liquid dispersion) having antimicrobial properties (particles 7) was obtained in the same manner as in the aforementioned Production Example 1, except that 339.0 parts of distilled water was used, the amount of cyclohexyl methacrylate monomer was 30 parts, the amount of n-butyl methacrylate was 45 parts, and 25 parts of an antimicrobial component [methylisothiazoline (MIT), available from Daiwa Chemical Industries, Co., Ltd.] was used as the antimicrobial component.

The content of the methacrylate monomer was 53.3 mass% relative to the total of the polymer components constituting the functional particles, and the content of the antimicrobial component was 15.6 mass% relative to the total of the polymer components. The functional particles had an average particle size of 49 nm. The zeta potential of the functional particles was -9.9 mV.

### Production Example 8

A functional particle dispersion (liquid dispersion) having antimicrobial properties (particles 8) was obtained in the same manner as in the aforementioned Production Example 1, except that 339.5 parts of distilled water was used, the amount of cyclohexyl methacrylate monomer was 30 parts, the amount of n-butyl methacrylate was 45 parts, and 25 parts of an antimicrobial component [thiabendazole, MOLDBAN-TZ, available from Mitsui Bussan Chemicals, Co., Ltd.] was used as the antimicrobial component.

The content of the methacrylate monomer was 53.1 mass% relative to the total of the polymer components constituting the functional particles, and the content of the antimicrobial component was 15.6 mass% relative to the total of the polymer components. The functional particles had an average particle size of 65 nm. The zeta potential of the functional particles was -3.8 mV.

### Production Example 9

A functional particle dispersion (liquid dispersion) having antimicrobial properties (particles 9) was obtained in the same manner as in the aforementioned Production Example 1, except that 334.5 parts of distilled water was used, the amount of cyclohexyl methacrylate monomer was 45 parts, the amount of n-butyl methacrylate was 30 parts, and 30 parts of an antimicrobial component [2-bromo-2-nitropropane-1,3-diol, MOLDBAN-BNP, available from Mitsui Bussan Chemicals, Co., Ltd.] was used as the antimicrobial component.

The content of the methacrylate monomer was 51.5 mass% relative to the total of the polymer components constituting the functional particles, and the content of the antimicrobial component was 18.2 mass% relative to the total of the polymer components. The functional particles had an average particle size of 62 nm. The zeta potential of the functional particles was +5.1 mV.

### Comparative Production Example 1

Particles A of Example 1 described in JP 2023-001833 were used. The content of the methacrylate monomer was 50 mass% relative to the total of the polymer components constituting the functional particles, and the content of the antimicrobial component was 36.2 mass% relative to the total of the polymer components. The functional particles had an average particle size of 56 nm. The zeta potential of the functional particles was -30.3 mV.

The obtained functional particle dispersion of each of Production Examples 1 to 9 and Comparative Production Example 1 described above was evaluated for antimicrobial effects (bacterial flora, yeast, and filamentous fungus) by the following evaluation method. The functional particle dispersion (liquid dispersion) obtained in each of Production Examples 1 to 9 described above was used. The solids content of the functional particles in the functional particle dispersion having the antimicrobial properties obtained in each of Production Examples 1 to 9 and Comparative Production Example 1 was 35 to 40 mass%.

For the functional particle dispersion having the antimicrobial properties obtained in each of Production Examples 1 to 9 and Comparative Production Example 1, dispersion stability, initial antimicrobial effects (bacterial flora, yeast, and filamentous fungus) and antimicrobial effects (bacterial flora, yeast, and filamentous fungus) after storage at 40°C for 3 months, and presence of an aggregate were evaluated by the following evaluation methods.

These results are shown in Table 1 below.

### Method for Evaluating Dispersion Stability

Using the functional particle dispersion (liquid dispersion) having the antimicrobial properties obtained in each of Production Examples 1 to 9 described above and the dispersion of Comparative Production Example 1, 10 mL of the obtained functional particle dispersion was filled in a 15 mL glass bottle with a lid together with a stirring ball (φ 6.4 mm, made of stainless steel), sealed, then stored for one month under the condition of 40°C with the lid facing upward, and then shaken. The dispersion stability was evaluated by the number of shakings performed until the stirring ball started to move in the glass bottle with the lid according to the following evaluation criteria.

### Evaluation criteria:

A: 0 to 3 times.
B: 4 to 10 times.
C: 11 times or more.
* 0 times: Movement of the stirring ball was observed when the glass bottle with the lid was tilted.

### Method for Testing Antimicrobial Effect

Using the functional particle dispersion (liquid dispersion) having the antimicrobial properties obtained in each of Production Examples 1 to 9 described above and the dispersion of Comparative Production Example 1, evaluation was performed by the following microbial testing method in accordance with ISO 11930:2012 (procedure for interpretation of data generated by the preservation efficacy test or by the microbiological risk assessment, or both).

The challenge test was performed by using the three groups including the bacterial flora, the yeast, and the filamentous fungus described below for initial dispersions and the dispersions after storage at 40°C for 3 months.
Bacterial flora: Staphylococcus aureus NBRC13276, Escherichia coli NBRC3972
Yeast: Candida albicans NBRC1594
Filamentous fungus: Aspergillus brasiliensis

### Preparation of Inocula

Preparation of inoculum: Bacterial cultures were prepared in accordance with ISO 11930:2012.
Bacterial flora: Each bacterial culture was prepared in accordance with ISO 11930:2012 for each bacterial strain. Three types of the bacterial cultures, each of which has its bacterial strain adjusted to 1 × 10⁷ to 1 × 10⁸ cfu/mL, were mixed using equal amounts, and thus an inoculum was prepared.
Yeast: In accordance with ISO 11930:2012, a bacterial culture was prepared to be 1 × 10⁶ to 1 × 10⁷ cfu/mL.
Filamentous fungus: In accordance with ISO 11930:2012, a bacterial culture was prepared to be 1 × 10⁶ to 1 × 10⁷ cfu/mL.

### Inoculation

For each of the functional particle dispersions having the antimicrobial properties, 1 mass% of the bacterial culture was inoculated.

### Storage

The inoculated antimicrobial particle dispersion was stored at temperature of 22.5 ± 2.5°C, and was observed for a designated period of time by detection culture.

### Detection Culture

A total of 1 g was applied on 10 sheets of SCD agar media for the bacterial flora, SD agar media for the yeast, and PD agar media for the filamentous fungus, and the bacterial flora and the yeast were cultured at 32.5°C for 2 days, and the filamentous fungus was cultured for 22.5°C for 5 days.

### Initial Antimicrobial Effect

For the functional particle dispersion having the antimicrobial properties obtained in each of Production Examples 1 to 9 and Comparative Production Example 1, the antimicrobial effect test described above was performed, and evaluation was performed based on the following evaluation criteria.

### Evaluation Criteria

A+: Colonies did not appear at day 3.
A: Colonies did not appear at day 7.
B: Colonies did not appear at day 21.
C: Several to tens of colonies emerged at day 28.
D: Obvious increase was observed at day 28.

### Antimicrobial Effect After Period of 3 Months at Temperature of 40°C

Each of the obtained functional particle dispersions having the antimicrobial properties of Production Examples 1 to 9 and Comparative Production Example 1 was filled in a bottle with a lid made of polyethylene in an amount of 60 mL, sealed, and stored for a period of 3 months under the condition at a temperature of 40°C. Then, each of the dispersions was subjected to the aforementioned antimicrobial effect test, and evaluated based on the following evaluation criteria.

### Evaluation Criteria

A+: Colonies did not appear at day 3.
A: Colonies did not appear at day 7.
B: Colonies did not appear at day 21.
C: Several to tens of colonies emerged at day 28.
D: Obvious increase was observed at day 28.

### Presence or Absence of Aggregate

Using the functional particle dispersion (liquid dispersion) having the antimicrobial properties obtained in each of Production Examples 1 to 9 described above and the dispersion of Comparative Production Example 1, 10 mL of the obtained functional particle dispersion was filled in a glass bottle with a lid, sealed, then stored for 3 months under the condition of 26°C with the lid facing upward, and then subjected to sensory evaluation for presence or absence of aggregates of the functional particle dispersion.

**[Table 1]**

| | | Production Example | | | | | | | | | Comparative Production Example |
|---|---|---|---|---|---|---|---|---|---|---|---|
| | | 1 | 2 | 3 | 4 | 5 | 6 | 7 | 8 | 9 | 1 |
| Dispersion stability 40°C, Duration: 1 month | | A | A | A | B | A | A | A | A | A | C |
| Antimicrobial effects (challenge test) initial | Bacterial flora | A+ | A+ | A+ | A+ | A+ | A+ | A+ | A+ | A | A |
| | Yeast | A+ | A+ | A+ | A+ | A+ | A+ | A+ | A+ | A+ | A+ |
| | Filamentous fungus | A+ | A+ | A+ | A+ | A+ | A+ | A+ | A+ | A+ | A+ |
| Antimicrobial effects (challenge test) 40°C, Duration: 3 months | Bacterial flora | A | A+ | A+ | A | A+ | A | A | A | A | C |
| | Yeast | A+ | A+ | A | A | A+ | A+ | A+ | A+ | A+ | B |
| | Filamentous fungus | A+ | A+ | A+ | A+ | A+ | A+ | A | A+ | B | D |
| Presence of aggregate 26°C, Duration: 3 months | | No | No | No | No | No | No | No | No | No | Yes |

From Table 1 above, it was found that Production Examples 1 to 9 within the scope of the present disclosure had excellent antimicrobial effects against bacterial flora, yeasts, filamentous fungi, and bacteria (Escherichia coli, Staphylococcus aureus) which are contamination sources and the like even after a long period of time (at 40°C, after 3 months), had high safety, did not cause aggregates after time had passed, and had excellent dispersion stability.

On the other hand, in Comparative Production Example 1, dispersion stability and antimicrobial effects could not be provided in a compatible manner at high levels, and the effects of the present disclosure could not be achieved.

### Examples 1 to 9 and Comparative Examples 1 to 4: Preparation of Aqueous Ink Composition for Writing Instrument

Aqueous ink compositions for writing instruments were prepared by a common method using the functional particle dispersions having the antimicrobial properties obtained in Production Examples 1 to 9 described above for Examples 1 to 9, Comparative Production Example 1 for Comparative Example 1, and antimicrobial component-containing dispersions each having a content of an antimicrobial component before being formed into particles of 15 mass% (IPBC, phenoxyethanol, paraben as antimicrobial components as Comparative Examples 2 to 4) for Comparative Examples 2 to 4, with the following formulation (total amount 100 mass%).

| | |
|---|---|
| Ink composition: (Total amount 100 mass%) | |
| Colorant: Colored resin fine particles (*1) | 50 mass% |
| Thickener: Polysaccharide fibers (*2) | 0.3 mass% |
| Lubricant: Phosphate (*3) | 0.5 mass% |
| Each functional particle dispersion (Production Examples 1 to 9, Comparative Production Example 1, Comparative Examples 2 to 4) | 20 mass% |
| pH Adjuster (triethanolamine) | 1 mass% |
| Water-soluble organic solvent (ethylene glycol) | 5 mass% |
| Water (distilled water) | Balance |

| | |
|---|---|
| *1: Lumikol NKW-2100E, available from D&C Commercial Co., Ltd. *2: RHEOCRYSTA I-2SX, available from DKS Co., Ltd. *3: Phosphanol RS-610, available from Toho Chemical Industry Co., Ltd. | |

The obtained aqueous ink compositions for writing instruments (total amount 100 mass%) were evaluated for writing performance (vertical line density difference), stability, initial antimicrobial effects, and antimicrobial effects after storage at 40°C for a period of 3 months by using writing instruments having the following configurations and according to the following evaluation method.

The evaluation results of Examples 1 to 9 and Comparative Examples 1 to 4 are shown in the following Table 2.

### Preparation of Writing Instrument: Ballpoint Pen

Using a shaft of a ballpoint pen [trade name: Signo UM-100, available from Mitsubishi Pencil Co., Ltd.], a refill including an ink storage tube made of polypropylene having an inner diameter of 4.0 mm and a length of 113 mm, a stainless steel tip (cemented carbide ball, ball diameter: 0.5 mm) and a joint connecting the storage tube and the tip was filled with each of the aqueous ink compositions described above, and an ink follower containing a mineral oil as a main component was inserted at the rear end of the ink. Thus, an aqueous ballpoint pen was prepared.

### Method for Evaluating Writing Performance (Vertical Line Density Difference)

Each aqueous ballpoint pen having the aforementioned configuration was left to stand at room temperature (25°C, the same applies hereinafter) for one month with the pen tip facing downward, and then writing was performed up to the final writing point, and the difference in density of the drawn line at the start of writing and at the end of writing was compared and evaluated according to the following evaluation criteria.

### Evaluation criteria:

A: No difference in density is observed.
B: A slight difference in density is observed.
C: A difference in density is clearly observed.
D: A difference in density is remarkable, and a portion where the drawn line is difficult to be visually recognized is observed.

### Method for Evaluating Stability

After each of the ballpoint pens having the aforementioned configurations was stored for 3 months under the condition of 50°C with the pen tip facing downward, the state of each ink in the refill was visually observed and evaluated according to the following criteria.

### Evaluation criteria:

A: Neither separation nor aggregation occurs.
B: Slight separation or aggregation.
C: Separation or aggregation is observed.
D: Significant separation or aggregation is observed.

### Method for Testing Antimicrobial Effect

Also for the aqueous ink compositions for writing instruments of Examples 1 to 9 that fall within the scope of the present disclosure and Comparative Examples 1 to 4, the test for the initial antimicrobial effects and the antimicrobial effects after storage at 40°C for a period of 3 months were performed in accordance with the method for testing antimicrobial effects performed with the antimicrobial particle dispersions described above (by inoculating a bacterial liquid in an amount of 1 mass% relative to the aqueous ink composition for writing instruments). For the antimicrobial effects after storage at 40°C for a period of 3 months, to check the antimicrobial effects more strictly, each of the aqueous ink compositions for writing instruments was charged in Posca PC-5M (available from Mitsubishi Pencil Co., Ltd.; shaft material: PP resin; stirring ball: stainless steel (φ: 6.4 mm)) and stored at a temperature of 40°C for a period of 3 months. The ink taken out from the pen body was then subjected to the evaluation method described above (challenge test by using the three groups including the bacterial flora, the yeast, and the filamentous fungus, evaluation criteria).

**[Table 2]**

| | | Example | | | | | | | | | Comparative Example | | | |
|---|---|---|---|---|---|---|---|---|---|---|---|---|---|---|
| | | 1 | 2 | 3 | 4 | 5 | 6 | 7 | 8 | 9 | 1 | 2 | 3 | 4 |
| Production Example No. or Comparative Production Example No. | | 1 | 2 | 3 | 4 | 5 | 6 | 7 | 8 | 9 | 1 | 2 | 3 | 4 |
| Writing performance 25°C, Duration: 1 month | | A | A | A | A | A | A | A | B | A | C | C | C | C |
| Stability 50°C, Duration: 3 months | | A | A | A | B | A | A | A | A | A | C | C | C | C |
| Antimicrobial effects (challenge test) initial | Bacterial flora | A+ | A+ | A | A+ | A | A+ | A | A+ | A+ | A | A | A | A |
| | Yeast | A+ | A+ | A+ | A+ | A+ | A+ | A+ | A+ | A+ | A+ | A | A+ | A+ |
| | Filamentous fungus | A+ | A+ | A+ | A+ | A+ | A+ | A+ | A+ | A+ | A | B | A | A+ |
| Antimicrobial effects (challenge test) 40°C, Duration: 3 months | Bacterial flora | A | A | A+ | A+ | A+ | A+ | A | A+ | A | C | B | B | B |
| | Yeast | A+ | A | A | A+ | A+ | A+ | A+ | A+ | A+ | B | B | A | B |
| | Filamentous fungus | A+ | A+ | A | A+ | A+ | A+ | A+ | A+ | A+ | A | D | B | C |

From Table 2 above, it was observed that Examples 1 to 9, which fall within the scope of the present disclosure, were superior to Comparative Examples 1 to 4, which fall outside the scope of the present disclosure, in writing performance (vertical line density difference) and stability, and did not adversely affect other blended components of the ink while maintaining strength and long-lasting effects of antimicrobial performance.

It has been also observed that the ballpoint pen prepared above does not cause blur or feathering, and has a sufficient drawn line density to give a sharp drawn line.

### Second Embodiment: Production Examples 10 to 22: Production of Functional Particle Dispersions (Particles 10 to 22)

Functional aromatic particle dispersions having antimicrobial properties and fragrance performance were produced in accordance with the following Production Examples 10 to 22. Note that the "parts" described below refers to parts by mass. The antimicrobial component and the aroma component are solids content.

### Production Example 10

A 2-liter flask was equipped with a stirrer, a reflux condenser, a thermometer, a nitrogen gas inlet tube, and a 1000-mL separatory funnel for addition of a monomer and set in a hot water bath, and 349.5 parts of distilled water, 5 parts of glycerol monomethacrylate [BLEMMER GLM, available from NOF Corporation], 5 parts of sodium 2-sulfoethyl methacrylate [acrylic ester SEM-Na, available from Mitsubishi Chemical Corporation], 40 parts of a nonionic polymerizable surfactant [available from Kao Corporation, LATEMUL PD-430], and 0.5 parts of ammonium persulfate were placed in the flask, and the internal temperature was raised to 50°C while nitrogen gas was introduced.

On the other hand, 17 parts of an aroma component [d-limonene, available from Nippon Terpene Chemicals, Inc.], 3 parts of an antimicrobial component [phenoxyethanol, available from Yokkaichi Chemical Company Limited] and 10 parts of a crosslinking agent [triallyl isocyanurate, (TAIC) available from Nippon Kasei Chemical Company Limited] were mixed with a monomer mixture including 50 parts of cyclohexyl methacrylate monomer and 20 parts of n-butyl methacrylate to prepare a solution.

The prepared solution was added to the aforementioned flask maintained at a temperature of approximately 50°C from the aforementioned separatory funnel over 3 hours with stirring to carry out emulsion polymerization. Furthermore, the solution was aged for 5 hours to terminate the polymerization, and thus a functional particle dispersion (liquid dispersion) having antimicrobial properties and aroma properties (particles 10) was obtained.

The content of the methacrylate monomer was 54.4 mass% relative to the total of the polymer components constituting the functional particles, the content of the antimicrobial component was 2.0 mass% relative to the total of the polymer components, and the content of the aroma component was 11.3 mass% relative to the total of the polymer components. The functional particles had an average particle size of 99 nm. The zeta potential of the functional particles was -1.0 mV.

### Production Example 11

A functional particle dispersion (liquid dispersion) having antimicrobial properties and aroma properties (particles 11) was obtained in the same manner as in the aforementioned Production Example 10, except that 347.5 parts of distilled water was used, the amount of cyclohexyl methacrylate monomer was 53 parts, the amount of n-butyl methacrylate was 17 parts, 20 parts of 2-phenylethanol (2-phenylethyl alcohol) [available from Inoue Perfumery Mfg. Co., Ltd.] was used as the aroma component, 2 parts of benzisothiazolinone (BIT) [available from Daiwa Chemical Industries, Co., Ltd.] was used as the antimicrobial component. The content of the methacrylate monomer was 52.6 mass% relative to the total of the polymer components constituting the functional particles, the content of the antimicrobial component was 1.3 mass% relative to the total of the polymer components, and the content of the aroma component was 13.2 mass% relative to the total of the polymer components. The functional particles had an average particle size of 88 nm. The zeta potential of the functional particles was -0.8 mV.

### Production Example 12

A functional particle dispersion (liquid dispersion) having antimicrobial properties and aroma properties (particles 12) was obtained in the same manner as in the aforementioned Production Example 10, except that 347.5 parts of distilled water was used, the amount of cyclohexyl methacrylate monomer was 55 parts, the amount of n-butyl methacrylate was 15 parts, 20 parts of CITRAL [available from Kuraray Co., Ltd.] was used as the aroma component, 2 parts of methylisothiazolinone (MIT) [available from Daiwa Chemical Industries, Co., Ltd.] was used as the antimicrobial component.

The content of the methacrylate monomer was 52.6 mass% relative to the total of the polymer components constituting the functional particles, the content of the antimicrobial component was 1.3 mass% relative to the total of the polymer components, and the content of the aroma component was 13.2 mass% relative to the total of the polymer components. The functional particles had an average particle size of 105 nm. The zeta potential of the functional particles was -2.7 mV.

### Production Example 13

A functional particle dispersion (liquid dispersion) having antimicrobial properties and aroma properties (particles 13) was obtained in the same manner as in the aforementioned Production Example 10, except that 327.5 parts of distilled water was used, the amount of cyclohexyl methacrylate monomer was 50 parts, the amount of n-butyl methacrylate was 20 parts, 30 parts of linalool (linalool oxide) [available from Inoue Perfumery Mfg. Co., Ltd.] as the aroma component, and 12 parts of chloromethylisothiazolinone/methylisothiazoline (CMIT/MIT) [available from Daiwa Chemical Industries, Co., Ltd.] was used as the antimicrobial component.

The content of the methacrylate monomer was 46.5 mass% relative to the total of the polymer components constituting the functional particles, the content of the antimicrobial component was 7.0 mass% relative to the total of the polymer components, and the content of the aroma component was 17.4 mass% relative to the total of the polymer components. The functional particles had an average particle size of 118 nm. The zeta potential of the functional particles was -5.8 mV.

### Production Example 14

A functional particle dispersion (liquid dispersion) having antimicrobial properties and aroma properties (particles 14) was obtained in the same manner as in the aforementioned Production Example 10, except that 329.5 parts of distilled water was used, the amount of cyclohexyl methacrylate monomer was 45 parts, the amount of n-butyl methacrylate was 25 parts, 26 parts of eugenol [available from Nippon Terpene Chemicals, Inc.] was used as the aroma component, and 14 parts of paraben [available from Ueno Fine Chemicals Industry, Ltd.] was used as the antimicrobial component. The content of the methacrylate monomer was 47.1 mass% relative to the total of the polymer components constituting the functional particles, the content of the antimicrobial component was 8.2 mass% relative to the total of the polymer components, and the content of the aroma component was 15.3 mass% relative to the total of the polymer components. The functional particles had an average particle size of 127 nm. The zeta potential of the functional particles was +3.8 mV.

### Production Example 15

A functional particle dispersion (liquid dispersion) having antimicrobial properties and aroma properties (particles 15) was obtained in the same manner as in the aforementioned Production Example 10, except that 314.5 parts of distilled water was used, the amount of cyclohexyl methacrylate monomer was 50 parts, the amount of n-butyl methacrylate was 30 parts, 35 parts of γ-decalactone [available from Inoue Perfumery Mfg. Co., Ltd.] was used as the aroma component, and 10 parts of iodopropargyl compound 3-iodo-2-propynylcarbamate [(Omacide IPBC 100) available from LONZA K.K.] was used as the antimicrobial component.

The content of the methacrylate monomer was 48.6 mass% relative to the total of the polymer components constituting the functional particles, the content of the antimicrobial component was 5.4 mass% relative to the total of the polymer components, and the content of the aroma component was 18.9 mass% relative to the total of the polymer components. The functional particles had an average particle size of 109 nm. The zeta potential of the functional particles was -8.5 mV.

### Production Example 16

A functional particle dispersion (liquid dispersion) having antimicrobial properties and aroma properties (particles 16) was obtained in the same manner as in the aforementioned Production Example 10, except that 324.5 parts of distilled water was used, the amount of cyclohexyl methacrylate monomer was 30 parts, the amount of n-butyl methacrylate was 45 parts, 25 parts of 1,8-cineol [available from Nippon Terpene Chemicals, Inc.] was used as the aroma component, 15 parts of benzisothiazolinone (BIT) [available from Daiwa Chemical Industries, Co., Ltd.] was used as the preservative component.

The content of the methacrylate monomer was 48.6 mass% relative to the total of the polymer components constituting the functional particles, the content of the antimicrobial component was 8.6 mass% relative to the total of the polymer components, and the content of the aroma component was 14.3 mass% relative to the total of the polymer components. The functional particles had an average particle size of 80 nm. The zeta potential of the functional particles was -10.5 mV.

### Production Example 17

A functional dispersion (liquid dispersion) having antimicrobial properties and aroma properties (particles 17) was obtained in the same manner as in the aforementioned Production Example 1, except that 334.5 parts of distilled water was used, the amount of cyclohexyl methacrylate monomer was 30 parts, the amount of n-butyl methacrylate was 45 parts, 25 parts of γ-terpinene was used as the aroma component, 5 parts of methylisothiazoline (MIT) [available from Daiwa Chemical Industries, Co., Ltd.] was used as the antimicrobial component.

The content of the methacrylate monomer was 51.5 mass% relative to the total of the polymer components constituting the functional particles, the content of the antimicrobial component was 3.0 mass% relative to the total of the polymer components, and the content of the aroma component was 15.2 mass% relative to the total of the polymer components. The functional particles had an average particle size of 67 nm. The zeta potential of the functional particles was +9.0 mV.

### Production Example 18

A fragrant particle dispersion (liquid dispersion) (particles 18) was obtained in the same manner as in the aforementioned Production Example 10, except that 334.5 parts of distilled water was used, the amount of cyclohexyl methacrylate monomer was 30 parts, the amount of n-butyl methacrylate was 45 parts, 25 parts of cymene was used as the aroma component, and 5 parts of methylisothiazoline (MIT) [available from Daiwa Chemical Industries, Co., Ltd.] was used as the antimicrobial component. The content of the methacrylate monomer was 51.5 mass% relative to the total of the polymer components constituting the functional particles, the content of the antimicrobial component was 3.0 mass% relative to the total of the polymer components, and the content of the aroma component was 15.2 mass% relative to the total of the polymer components. The functional particles had an average particle size of 102 nm. The zeta potential of the functional particles was -2.9 mV.

### Production Example 19

A functional particle dispersion (liquid dispersion) having antimicrobial properties and aroma properties (particles 19) was obtained in the same manner as in the aforementioned Production Example 10, except that 329.5 parts of distilled water was used, the amount of cyclohexyl methacrylate monomer was 35 parts, the amount of n-butyl methacrylate was 40 parts, 25 parts of myrcene was used as the aroma component, and 10 parts of iodopropargyl compound 3-iodo-2-propynylcarbamate [(Omacide IPBC 100) available from LONZA K.K.] was used as the antimicrobial component.

The content of the methacrylate monomer was 50.0 mass% relative to the total of the polymer components constituting the functional particles, the content of the antimicrobial component was 5.9 mass% relative to the total of the polymer components, and the content of the aroma component was 14.7 mass% relative to the total of the polymer components. The functional particles had an average particle size of 91 nm. The zeta potential of the functional particles was -0.2 mV.

### Production Example 20

A functional particle dispersion (liquid dispersion) having antimicrobial properties and aroma properties (particles 20) was obtained in the same manner as in the aforementioned Production Example 10, except that 329.5 parts of distilled water was used, the amount of cyclohexyl methacrylate monomer was 35 parts, the amount of n-butyl methacrylate was 40 parts, 25 parts of phellandrene was used as the aroma component, and 10 parts of iodopropargyl compound 3-iodo-2-propynylcarbamate [(Omacide IPBC 100) available from LONZA K.K.] was used as the antimicrobial component.

The content of the methacrylate monomer was 50.0 mass% relative to the total of the polymer components constituting the functional particles, the content of the antimicrobial component was 5.9 mass% relative to the total of the polymer components, and the content of the aroma component was 14.7 mass% relative to the total of the polymer components. The functional particles had an average particle size of 111 nm. The zeta potential of the functional particles was -3.5 mV.

### Production Example 21

A functional particle dispersion (liquid dispersion) having antimicrobial properties and aroma properties (particles 21) was obtained in the same manner as in the aforementioned Production Example 10, except that 329.5 parts of distilled water was used, the amount of cyclohexyl methacrylate monomer was 45 parts, the amount of n-butyl methacrylate was 30 parts, 25 parts of farnesene was used as the aroma component, and 10 parts of paraben [available from Ueno Fine Chemicals Industry, Ltd.] was used as the antimicrobial component.

The content of the methacrylate monomer was 50.0 mass% relative to the total of the polymer components constituting the functional particles, the content of the antimicrobial component was 5.9 mass% relative to the total of the polymer components, and the content of the aroma component was 14.7 mass% relative to the total of the polymer components. The functional particles had an average particle size of 84 nm. The zeta potential of the functional particles was +0.8 mV.

### Production Example 22

A functional particle dispersion (liquid dispersion) having antimicrobial properties and aroma properties (particles 22) was obtained in the same manner as in the aforementioned Production Example 10, except that 329.5 parts of distilled water was used, the amount of cyclohexyl methacrylate monomer was 45 parts, the amount of n-butyl methacrylate was 30 parts, 25 parts of thujene was used as the aroma component, and 10 parts of paraben [available from Ueno Fine Chemicals Industry, Ltd.] was used as the antimicrobial component.

The content of the methacrylate monomer was 50.0 mass% relative to the total of the polymer components constituting the functional particles, the content of the antimicrobial component was 5.9 mass% relative to the total of the polymer components, and the content of the aroma component was 14.7 mass% relative to the total of the polymer components. The functional particles had an average particle size of 97 nm. The zeta potential of the functional particles was -6.3 mV.

The functional particle dispersions (liquid dispersions) having the antimicrobial properties and the aroma properties obtained in Production Examples 10 to 22 described above were obtained. The solids content of the functional particles having the antimicrobial properties and the aroma properties in the functional particle dispersion having the antimicrobial properties and the aroma properties obtained in each of Production Examples 10 to 22 was 30 to 40 mass%.

Using the functional particle dispersion (liquid dispersion) having the antimicrobial properties and the aroma properties of each of Production Examples 10 to 22 obtained as described above, lingering aroma intensity, dispersion stability, and antimicrobial effects were evaluated based on the following evaluation methods. As Comparative Production Example 2, a functional particle dispersion (liquid dispersion) (particles) having the antimicrobial properties and the aroma properties was obtained in the same manner as in Production Example 10, except that 40 parts of anionic surfactant [ADEKA REASOAP SE-10N, available from ADEKA Corporation] was used as the polymerizable surfactant. In the particles, the content of the methacrylate monomer was 53.3 mass% relative to the total of the polymer components constituting the functional particles, the content of the antimicrobial component was 2.0 mass% relative to the total of the polymer components, and the content of the aroma component was 11.3 mass% relative to the total of the polymer components. The functional particles had an average particle size of 61 nm. The zeta potential of the functional particles was -31.5 mV.

These results are shown in Table 3 below.

### Method for Evaluating Lingering Aroma Intensity

The functional particle dispersion (liquid dispersion) having the antimicrobial properties and the aroma properties of each of Production Examples 10 to 22 and Comparative Production Example 2 obtained as described above was diluted in a beaker to adjust the solids content to 40 mass%. A cotton cloth (cotton 100%, size: 50 × 100 mm) was immersed therein, stirred for 30 times by a hand, and then left to stand for 15 minutes.

Then, the cotton cloth was squeezed by hands to remove water and dried for 3 weeks under the constant temperature and constant humidity condition at 25°C and 35%RH, and thus used as a treated cloth for evaluation.

The sensory evaluation of the lingering aroma intensity was performed for the aroma felt from the cotton cloth obtained as described above based on the following evaluation criteria.

### Lingering Aroma Intensity

For the treated cloth for evaluation that was treated by the method described above, the lingering aroma intensity was evaluated by five evaluators based on the following criteria. These evaluation results by the five people are shown in Table 1 below.

### Evaluation Criteria of Lingering Aroma Intensity

A: Lingering aroma was intensely felt.
B: Lingering aroma was weakly felt.
C: No lingering aroma was felt.

### Method for Evaluating Dispersion Stability

Using the functional particle dispersion (liquid dispersion) having the antimicrobial properties and the aroma properties obtained in each of Production Examples 10 to 22 and Comparative Production Example 2 described above, 50 mL of the obtained functional particle dispersion was filled in a 100 mL plastic bottle with a lid that was made of PP having high transparency, sealed, then stored for one month under the condition of 40°C with the cap facing upward, and then evaluated for dispersion stability based on the following evaluation criteria.

### Evaluation criteria:

A: No separation of the liquid dispersion was observed and no precipitates were observed at the bottom, which were good.
B: Slight separation was observed in the liquid dispersion, and slight precipitates were observed at the bottom.
C: Remarkable separation was observed in the liquid dispersion, and many precipitates were observed at the bottom.

### Method for Testing Antimicrobial Effect

Using the functional particle dispersion (liquid dispersion) having the antimicrobial properties and the aroma properties of each of Production Examples 10 to 22 and Comparative Production Example 2 obtained as described above, the microbial testing method described in detail for the first embodiment described above (challenge test by using the three groups including the bacterial flora, the yeast, and the filamentous fungus, including evaluation criteria) in accordance with ISO 11930:2012 (procedure for interpretation of data generated by the preservation efficacy test or by the microbiological risk assessment, or both) was performed for initial and after storage at 40°C for 3 months.

**[Table 3]**

| | | Production Example | | | | | | | | | | | | | Comparative Production Example |
|---|---|---|---|---|---|---|---|---|---|---|---|---|---|---|---|
| | | 10 | 11 | 12 | 13 | 14 | 15 | 16 | 17 | 18 | 19 | 20 | 21 | 22 | 2 |
| Lingering aroma intensity 25°C, 35%RH, Duration: 3 weeks | | A | A | A | A | A | A | A | A | A | A | A | A | A | B |
| Dispersion stability 40°C, Duration: 1 month | | A | A | B | A | A | A | A | A | A | A | A | A | A | C |
| Antimicrobial effects (challenge test) initial | Bacterial flora | A+ | A+ | A+ | A+ | A | A | A+ | A | A+ | A+ | A+ | A+ | A+ | A |
| | Yeast | A+ | A+ | A+ | A | A+ | A+ | A+ | A+ | A+ | A | A+ | A+ | A+ | A+ |
| | Filamentous fungus | A+ | A+ | A+ | A+ | A+ | A+ | A+ | A+ | A+ | A+ | A+ | A+ | A+ | A |
| Antimicrobial effects (challenge test) 40°C, Duration: 3 months | Bacterial flora | A+ | A+ | A | A+ | A+ | A+ | A+ | A+ | A+ | A | A+ | A+ | A+ | D |
| | Yeast | A+ | A+ | A+ | A+ | A+ | A+ | A+ | A+ | A+ | A+ | A+ | A+ | A+ | C |
| | Filamentous fungus | A+ | A | A+ | A | A+ | A+ | A | A+ | A+ | A | B | A+ | A | D |

As is clear from the results in Table 3 described above, it was found that the functional particle dispersion (liquid dispersion) having the antimicrobial properties and the aroma properties of each of Production Examples 10 to 22 obtained as described above had superior lingering aroma intensity, dispersion stability, and antimicrobial effects to those of Comparative Production Example 2.

### Second Embodiment: Examples 10 to 22 and Comparative Example 5: Preparation of Aqueous Ink Composition for Writing Instrument

The functional particle dispersions (liquid dispersions) having the antimicrobial properties and the aroma properties obtained in Production Examples 10 to 22 described above for Examples 10 to 22, and Comparative Production Example 2 were obtained. The solids content of the fragrant particles in the fragrant particle dispersion obtained in each of Production Examples 10 to 22 was 35 to 40 mass%.

Meanwhile, for Comparative Example 5, the functional particle dispersion (liquid dispersion) having the antimicrobial properties and the aroma properties obtained in Comparative Production Example 2 was used. In addition, for Comparative Examples 6 to 8, the following three types of known fragrances and preservatives were used.

Comparative Example 6 used an aroma component (d-limonene, available from Nippon Terpene Chemicals, Inc.) and an antimicrobial component (phenoxyethanol, available from Yokkaichi Chemical Company Limited). Comparative Example 7 used an aroma component 2-phenylethanol (2-phenylethyl alcohol) [available from Inoue Perfumery Mfg. Co., Ltd.] and an antimicrobial component benzisothiazolinone (BIT) [available from Daiwa Chemical Industries, Co., Ltd.]. Comparative Example 8 used an aroma component CITRAL [available from Kuraray Co., Ltd.] and an antimicrobial component methylisothiazoline (MIT) [Daiwa Chemical Industries, Co., Ltd.]. An ink composition for writing instruments was adjusted so that the solids content of the aroma component and the antimicrobial component is equivalent as those of Examples 10 to 22.

Each of the functional particle dispersions having the antimicrobial properties and the aroma properties produced in Production Examples 10 to 22 and Comparative Production Example 2 described above (particles 10 to 22 and particles of Comparative Production Example 2) and Comparative Examples 6 to 8 described above was used to prepare an aqueous ink composition for writing instruments by an ordinary method according to the blend composition (total amount 100 mass%) described below.

| | |
|---|---|
| Ink composition: (Total amount 100 mass%) | |
| Colorant: Colored resin fine particles (*4) | 15 mass% |
| Thickener: Polysaccharide fibers (*5) | 0.3 mass% |
| Lubricant: Phosphate (*6) | 0.5 mass% |
| Each functional particle dispersion (Production Examples 10 to 22, Comparative Production Example 2, Comparative Examples 6 to 8) | 20 mass% |
| pH Adjuster (triethanolamine) | 1 mass% |
| Water-soluble organic solvent (ethylene glycol) | 5 mass% |
| Water (distilled water) | Balance |

| | |
|---|---|
| *4: Thermochromic microcapsule pigment black: Average particle size 0.6 µm *5: Xanthan gum KELSAN S (available from Sansho Co., Ltd.) *6: Phosphanol RS-610 (available from Toho Chemical Industry Co., Ltd.) | |

The obtained aqueous ink compositions for writing instruments (total amount 100 mass%) were evaluated for lingering aroma intensity, dispersion stability, and antimicrobial performance according to the following evaluation methods. These evaluation results are shown in Table 4 below.

### Method for Evaluating Lingering Aroma Intensity of Ink for Writing Instrument

The aqueous ink composition for writing instruments obtained as described above was charged in an aqueous felt-tip pen [PM-120T: available from Mitsubishi Pencil Co., Ltd., including an ink absorbent, pen core: (fine) PET fibers, (extra fine) POM resin, the same applies hereinafter], and thus a pen body was produced. Furthermore, a pen body shaft thereof was placed laterally and stored at a temperature of 40°C for a period of one month. Then, the pen core (fine) was used for writing on a paper (high-quality paper), and lingering aroma intensity after 24 hours was evaluated by five evaluators based on the following criteria.

### Evaluation criteria:

A: Aroma was confirmed during writing, and lingering aroma of handwriting was intensely felt after 24 hours.
B: Aroma was confirmed during writing, and lingering aroma of handwriting after 24 hours was slightly felt.
C: Aroma was slightly confirmed during writing, and lingering aroma of handwriting after 24 hours was not felt.

### Method for Evaluating Dispersion Stability

Using PM-120T (available from Mitsubishi Pencil Co., Ltd.), the aqueous ink composition for writing instruments obtained as described above was stored at 40°C for a period of one month with the fine pen tip side facing downward. Thereafter, circles were written with the fine pen tip side on writing paper and evaluated based on the following criteria.

### Evaluation criteria:

A: Same level as initial writing
B: Blur occurred when fast writing was performed
C: Slight blurring occurred
D: Writing failure (unable to write)

For each of the obtained aqueous ink compositions for writing instruments (total amount 100 mass%), the following microbial testing method in accordance with ISO 11930:2012 (procedure for interpretation of data generated by the preservation efficacy test or by the microbiological risk assessment, or both) was performed. For the microbial testing method, to check the antimicrobial effects more strictly, each of the aqueous ink compositions for writing instruments was charged in Posca PC-5M (available from Mitsubishi Pencil Co., Ltd.; shaft material: PP resin; stirring ball: stainless steel (φ: 6.4 mm)) and stored at a temperature of 40°C for a period of 3 months. The initial ink, and the ink taken out from the pen body was then subjected to the evaluation method of the first embodiment described above (challenge test by using the three groups including the bacterial flora, the yeast, and the filamentous fungus, evaluation criteria).

**[Table 4]**

| | | Example | | | | | | | | | | | | | Comparative Example | | | |
|---|---|---|---|---|---|---|---|---|---|---|---|---|---|---|---|---|---|---|
| | | 10 | 11 | 12 | 13 | 14 | 15 | 16 | 17 | 18 | 19 | 20 | 21 | 22 | 5 | 6 | 7 | 8 |
| Production Example No. or Comparative Production Example No. | | 10 | 11 | 12 | 13 | 14 | 15 | 16 | 17 | 18 | 19 | 20 | 21 | 22 | 5 | 6 | 7 | 8 |
| Lingering aroma intensity after 24 hours | | A | A | A | A | A | A | A | A | A | A | A | A | A | B | B | B | C |
| Dispersion stability 40°C, Duration: 1 month | | A | A | A | A | A | A | A | A | A | A | A | A | A | C | C | C | C |
| Antimicrobial effects (challenge test) initial | Bacterial flora | A+ | A+ | A+ | A+ | A+ | A+ | A+ | A+ | A | A+ | A+ | A+ | A+ | A | A | A | A |
| | Yeast | A+ | A+ | A+ | A+ | A+ | A+ | A+ | A+ | A+ | A+ | A+ | A+ | A+ | A | A+ | A+ | A |
| | Filamentous fungus | A+ | A+ | A+ | A+ | A+ | A+ | A+ | A+ | A+ | A+ | A+ | A+ | A+ | A | A | A+ | A |
| Antimicrobial effects (challenge test) 40°C, Duration: 3 months | Bacterial flora | A | A+ | A | A+ | A | A+ | A+ | A+ | A | A+ | A+ | A | A+ | B | B | C | D |
| | Yeast | A+ | A+ | A+ | A+ | A | A+ | A+ | A+ | A+ | A+ | A+ | A | A | C | B | B | C |
| | Filamentous fungus | A | A+ | A | A+ | A+ | A+ | A+ | A+ | A+ | A+ | A | A+ | A+ | D | B | C | C |

Taking Table 4 above into consideration, it was confirmed that Examples 10 to 22, which fall within the scope of the present disclosure, did not affect other blended components of the ink and had superior dispersion stability, lingering aroma intensity, and antimicrobial effects even in the compounding system containing the polysaccharide fibers as the thickener, compared to Comparative Examples 5 and 6 to 8, which fall outside the scope of the present disclosure. Furthermore, when evaluation was performed for the writing performance of Examples 10 to 22 by writing on a paper surface, it was confirmed that good hand-drawn lines were formed without scratchiness.

### Third Embodiment: Production Examples 23 to 33: Production of Functional Particle Dispersions (Particles 23 to 33)

Functional particle dispersions having reduction properties were produced in the following Production Examples 23 to 33. Note that the "parts" described below refers to parts by mass. The reducing component is a solids content.

### Production Example 23

A 2-liter flask was equipped with a stirrer, a reflux condenser, a thermometer, a nitrogen gas inlet tube, and a 1000-mL separatory funnel for addition of a monomer and set in a hot water bath, and 324.5 parts of distilled water, 5 parts of glycerol monomethacrylate [BLEMMER GLM, available from NOF Corporation], 5 parts of sodium 2-sulfoethyl methacrylate [acrylic ester SEM-Na, available from Mitsubishi Chemical Corporation], 40 parts of a nonionic polymerizable surfactant [available from Kao Corporation, LATEMUL PD-430], and 0.5 parts of ammonium persulfate were placed in the flask, and the internal temperature was raised to 50°C while nitrogen gas was introduced.

Meanwhile, 40 parts of a reducing component [tannin, (Tannic acid S) available from Fuji Chemical Industries, Co., Ltd.] and 10 parts of a crosslinking agent [triallyl isocyanurate, (TAIC) available from Nippon Kasei Chemical Company Limited] were mixed with a monomer mixture including 40 parts of cyclohexyl methacrylate monomer and 35 parts of n-butyl methacrylate to prepare a solution.

The prepared solution was added to the aforementioned flask maintained at a temperature of approximately 50°C from the aforementioned separatory funnel over 3 hours with stirring to carry out emulsion polymerization. Furthermore, the solution was aged for 5 hours to terminate the polymerization, and thus a functional particle dispersion (liquid dispersion) having reduction properties (particles 23) was obtained.

The content of the methacrylate monomer was 48.6 mass% relative to the total of the polymer components constituting the functional particles, and the content of the reducing component was 22.9 mass% relative to the total of the polymer components. The functional particles had an average particle size of 61 nm. The zeta potential of the functional particles having the reduction properties was -7.5 mV.

### Production Example 24

A functional particle dispersion (liquid dispersion) having reduction properties (particles 24) was obtained in the same manner as in the aforementioned Production Example 23, except that 320.5 parts of distilled water was used, the amount of cyclohexyl methacrylate monomer was 30 parts, the amount of n-butyl methacrylate was 45 parts, and 44 parts of an reducing component [chlorogenic acid, (Caffenol P-100) available from Fuji Chemical Industries, Co., Ltd.] was used as the reducing component.

The content of the methacrylate monomer was 47.5 mass% relative to the total of the polymer components constituting the functional particles, and the content of the reducing component was 24.6 mass% relative to the total of the polymer components. The functional particles had an average particle size of 82 nm. The zeta potential of the functional particles having the reduction properties was -9.8 mV.

### Production Example 25

A liquid dispersion of functional particle dispersion having reduction properties (particles 25) was obtained in the same manner as in the aforementioned Production Example 23, except that 313.5 parts of distilled water was used, the amount of cyclohexyl methacrylate monomer was 60 parts, the amount of n-butyl methacrylate was 30 parts, and 36 parts of a reducing component [catechin, ((-)-catechin, derived from green tea) available from FUJIFILM Wako Pure Chemical Corporation] was used as the reducing component.

The content of the methacrylate monomer was 53.8 mass% relative to the total of the polymer components constituting the functional particles, and the content of the reducing component was 19.4 mass% relative to the total of the polymer components. The functional particles had an average particle size of 107 nm. The zeta potential of the functional particles having the reduction properties was -2.8 mV.

### Production Example 26

A functional particle dispersion (liquid dispersion) having reduction properties (particles 26) was obtained in the same manner as in the aforementioned Production Example 23, except that 299.5 parts of distilled water was used, the amount of cyclohexyl methacrylate monomer was 60 parts, the amount of n-butyl methacrylate was 35 parts, and 45 parts of a reducing component [copper chlorophyll, (trisodium copper chlorophyllin) available from FUJIFILM Wako Pure Chemical Corporation ] was used as the reducing component.

The content of the methacrylate monomer was 52.5 mass% relative to the total of the polymer components constituting the functional particles, and the content of the reducing component was 22.5 mass% relative to the total of the polymer components. The functional particles had an average particle size of 119 nm. The zeta potential of the functional particles having the reduction properties was +5.8 mV.

### Production Example 27

A functional particle dispersion (liquid dispersion) having reduction properties (particles 27) was obtained in the same manner as in the aforementioned Production Example 23, except that 320.5 parts of distilled water was used, the amount of cyclohexyl methacrylate monomer was 30 parts, the amount of n-butyl methacrylate was 45 parts, and 44 parts of a reducing component [piceatannol, (Passienol LA) available from Morinaga&Co., Ltd.] was used as the reducing component.

The content of the methacrylate monomer was 47.5 mass% relative to the total of the polymer components constituting the functional particles, and the content of the reducing component was 24.6 mass% relative to the total of the polymer components. The functional particles had an average particle size of 128 nm. The zeta potential of the functional particles having the reduction properties was +9.1 mV.

### Production Example 28

A functional particle dispersion (liquid dispersion) having reduction properties (particles 28) was obtained in the same manner as in the aforementioned Production Example 23, except that 320.5 parts of distilled water was used, the amount of cyclohexyl methacrylate monomer was 30 parts, the amount of n-butyl methacrylate was 45 parts, and 44 parts of a reducing component [propyl gallate, available from FUJIFILM Wako Pure Chemical Corporation] was used as the reducing component.

The content of the methacrylate monomer was 47.5 mass% relative to the total of the polymer components constituting the functional particles, and the content of the reducing component was 24.6 mass% relative to the total of the polymer components. The functional particles had an average particle size of 78 nm. The zeta potential of the functional particles having the reduction properties was -8.8 mV.

### Production Example 29

A functional particle dispersion (liquid dispersion) having reduction properties (particles 29) was obtained in the same manner as in the aforementioned Production Example 23, except that 320.0 parts of distilled water was used, the amount of cyclohexyl methacrylate monomer was 30.5 parts, the amount of n-butyl methacrylate was 45 parts, and 44 parts of an reducing component [dibutylhydroxytoluene (BHT), "BHT", available from Nikki-Universal Co., Ltd.] was used as the reducing component.

The content of the methacrylate monomer was 47.6 mass% relative to the total of the polymer components constituting the functional particles, and the content of the reducing component was 24.5 mass% relative to the total of the polymer components. The functional particles had an average particle size of 54 nm. The zeta potential of the functional particles having the reduction properties was -11.7 mV.

### Production Example 30

A functional particle dispersion (liquid dispersion) having reduction properties (particles 30) was obtained in the same manner as in the aforementioned Production Example 23, except that 319.5 parts of distilled water was used, the amount of cyclohexyl methacrylate monomer was 25 parts, the amount of n-butyl methacrylate was 50 parts, and 45 parts of a reducing component [butylhydroxyanisole (BHA), "Sasuten 1-F (BHA)", available from Nikki-Universal Co., Ltd.] was used as the reducing component.

The content of the methacrylate monomer was 47.2 mass% relative to the total of the polymer components constituting the functional particles, and the content of the reducing component was 25.0 mass% relative to the total of the polymer components. The functional particles had an average particle size of 79 nm. The zeta potential of the functional particles having the reduction properties was -6.4 mV.

### Production Example 31

A 2-liter flask was equipped with a stirrer, a reflux condenser, a thermometer, a nitrogen gas inlet tube, and a 1000-mL separatory funnel for addition of a monomer and set in a hot water bath, and 296.2 parts of distilled water, 5 parts of glycerol monomethacrylate [BLEMMER GLM, available from NOF Corporation], 5 parts of sodium 2-sulfoethyl methacrylate [acrylic ester SEM-Na, available from Mitsubishi Chemical Corporation], 53.3 parts of a nonionic polymerizable surfactant [ADEKA REASOAP ER-20, available from ADEKA Corporation], and 0.5 parts of ammonium persulfate were placed in the flask, and the internal temperature was raised to 50°C while nitrogen gas was introduced.

Meanwhile, 30 parts of a reducing component [tannin, (Tannic acid S) available from Fuji Chemical Industries, Co., Ltd.], 10 parts of a preservative component [phenoxyethanol, available from Yokkaichi Chemical Company Limited], and 10 parts of a crosslinking agent [triallyl isocyanurate, (TAIC) available from Nippon Kasei Chemical Company Limited] were mixed with a monomer mixture including 50 parts of cyclohexyl methacrylate monomer and 40 parts of n-butyl methacrylate to prepare a solution.

The prepared solution was added to the aforementioned flask maintained at a temperature of approximately 50°C from the aforementioned separatory funnel over 3 hours with stirring to carry out emulsion polymerization. Furthermore, the solution was aged for 5 hours to terminate the polymerization, and thus a functional particle dispersion (liquid dispersion) having reduction properties (particles 31) was obtained.

The content of the methacrylate monomer was 54.2 mass% relative to the total of the polymer components constituting the functional particles, and the content of the reducing component was 16.7 mass% relative to the total of the polymer components. The functional particles had an average particle size of 59 nm. The zeta potential of the functional particles having the reduction properties was -8.5 mV.

### Production Example 32

A functional particle dispersion (liquid dispersion) having reduction properties (particles 32) was obtained in the same manner as in the aforementioned Production Example 31, except that 296.2 parts of distilled water was used, the amount of cyclohexyl methacrylate monomer was 45 parts, the amount of n-butyl methacrylate was 45 parts, 28 parts of a reducing component [tannin, (Tannic acid S) available from Fuji Chemical Industries, Co., Ltd.] was used as the reducing component, and 12 parts of benzisothiazolinone ((BIT), available from Daiwa Chemical Industries, Co., Ltd.) as the preservative component.

The content of the methacrylate monomer was 54.2 mass% relative to the total of the polymer components constituting the functional particles, and the content of the reducing component was 15.2 mass% relative to the total of the polymer components. The functional particles had an average particle size of 85 nm. The zeta potential of the functional particles having the reduction properties was -6.8 mV.

### Production Example 33

A liquid dispersion of a functional particle dispersion having reduction properties (particles 33) was obtained in the same manner as in the aforementioned Production Example 31, except that 306.2 parts of distilled water was used, the amount of cyclohexyl methacrylate monomer was 30 parts, the amount of n-butyl methacrylate was 45 parts, 30.5 parts of chlorogenic acid, available from Fuji Chemical Industries, Co., Ltd. (Caffenol P-100) was used as the reducing component, and 14.5 parts of methylisothiazoline (MIT) available from Daiwa Chemical Industries, Co., Ltd. was used as the preservative component.

The content of the methacrylate monomer was 48.7 mass% relative to the total of the polymer components constituting the functional particles, and the content of the reducing component was 17.5 mass% relative to the total of the polymer components. The functional particles had an average particle size of 106 nm. The zeta potential of the functional particles having the reduction properties was 2.8 mV.

The functional particle dispersions (liquid dispersions) having the reduction properties obtained in Production Examples 23 to 33 described above were obtained. The solids content of the functional particles in the functional particle dispersion having the reduction properties obtained in each of Production Examples 22 to 33 was 35 to 40 mass%.

Using the functional particle dispersions (liquid dispersions) having the reduction properties of Production Examples 23 to 33 obtained as described above, the long-lasting effects of the reduction performance (ability to remove dissolved oxygen), the dispersion stability, and the antimicrobial effects were evaluated by the following evaluation methods.

Furthermore, as Comparative Production Example 3, a functional particle dispersion (liquid dispersion) (particles) having the reduction properties was obtained in the same manner as in Production Example 23, except that 40 parts of an anionic surfactant [ADEKA REASOAP SE-10N, available from ADEKA Corporation] was used as the polymerizable surfactant. In the particles, the content of the methacrylate monomer was 48.6 mass% relative to the total of the polymer components constituting the functional particles, and the content of the reducing component was 22.9 mass% relative to the total of the polymer components. The functional particles had an average particle size of 58 nm. The zeta potential of the functional particles having the reduction properties was -51.4 mV.

These results are shown in Table 5 below.

### Method for Evaluating Reduction Performance

The reduction properties were evaluated by measuring the amount of dissolved oxygen using the functional particle dispersions (liquid dispersions) having the reduction properties of Production Examples 23 to 33 obtained as described above. For the evaluation, a dissolved oxygen meter: WQ-320 (available from Horiba, Ltd.) was used, and after the preparation of the functional particle dispersion, the functional particle dispersion was allowed to stand at a temperature of 25°C, for storage periods of 48 hours and 3 months, and the long-lasting effects of the reduction performance measured at a measurement temperature of 25°C was evaluated according to the following evaluation criteria. Evaluation criteria:
A: The amount of dissolved oxygen was 0.1 mg/L or greater and less than 10 mg/L.
B: The amount of dissolved oxygen was 10 mg/L or greater and less than 20 mg/L.
C: The amount of dissolved oxygen was 20 mg/L or greater.

### Method for Evaluating Dispersion Stability

Using the functional particle dispersion (liquid dispersion) obtained in each of Production Examples 23 to 33 described above, 10 mL of the obtained functional particle dispersion was filled in a 15 mL glass bottle with a lid together with a stirring ball (φ 6.4 mm, made of stainless steel), sealed, then stored for one month under the condition of 40°C with the lid facing upward, and then shaken. The dispersion stability was evaluated by the number of shakings performed until the stirring ball started to move in the glass bottle with the lid according to the following evaluation criteria.

### Evaluation criteria:

A: 0 to 3 times.
B: 4 to 10 times.
C: 11 times or more.
* 0 times: Movement of the stirring ball was confirmed when the glass bottle with the lid was tilted.

### Method for Testing Antimicrobial Effect

Using the functional particle dispersion (liquid dispersion) obtained in each of Production Examples 23 to 33 described above, evaluation was performed by the following microbial testing method in accordance with ISO 11930:2012 (procedure for interpretation of data generated by the preservation efficacy test or by the microbiological risk assessment, or both).

The challenge test was performed by using the three groups including the bacterial flora, the yeast, and the filamentous fungus described below for initial and after storage at 40°C for 3 months.
Bacterial group: Staphylococcus aureus NBRC13276, Escherichia coli NBRC3972
Yeast: Candida albicans NBRC1594
Filamentous fungus: Aspergillus brasiliensis

### Preparation of Inocula

Preparation of inoculum: Bacterial cultures were prepared in accordance with ISO 11930:2012.
Bacterial flora: Each bacterial culture was prepared in accordance with ISO 11930:2012 for each bacterial strain. Three types of the bacterial cultures, each of which has its bacterial strain adjusted to 1 × 10⁷ to 1 × 10⁸ cfu/mL, were mixed using equal amounts, and thus an inoculum was prepared.
Yeast: In accordance with ISO 11930:2012, a bacterial culture was prepared to be 1 × 10⁶ to 1 × 10⁷ cfu/mL.
Filamentous fungus: In accordance with ISO 11930:2012, a bacterial culture was prepared to be 1 × 10⁶ to 1 × 10⁷ cfu/mL.

### Inoculation

For the ink composition for writing instruments, 1 mass% of a bacterial culture was inoculated. <Storage> The inoculated ink composition for writing instruments was stored at temperature of 22.5 ± 2.5°C, and was observed for a designated period of time by detection culture.

### Detection Culture

A total of 1 g was applied on 10 sheets of SCD agar media for the bacterial flora, SD agar media for the yeast, and PD agar media for the filamentous fungus, and the bacterial flora and the yeast were cultured at 32.5°C for 2 days, and the filamentous fungus was cultured for 22.5°C for 5 days.

### Evaluation Criteria

A+: Colonies did not appear at day 3.
A: Colonies did not appear at day 7.
B: Colonies did not appear at day 21.
C: Several to tens of colonies emerged at day 28.
D: Obvious increase was observed at day 28.

**[Table 5]**

| | | Production Example | | | | | | | | | | | Comparative Production Example |
|---|---|---|---|---|---|---|---|---|---|---|---|---|---|
| | | 23 | 24 | 25 | 26 | 27 | 28 | 29 | 30 | 31 | 32 | 33 | 3 |
| Reduction performance (amount of dissolved oxygen) after 48 hours | | A | A | A | A | A | A | A | A | A | A | A | A |
| Persistence of reduction performance after 3 months | | B | A | A | A | A | A | A | A | A | A | A | B |
| Dispersion stability 40°C, Duration: 1 month | | A | A | A | A | A | A | A | A | A | A | A | C |
| Antimicrobial effects (challenge test) initial | Bacterial flora | A+ | A+ | A+ | A+ | A+ | A+ | A+ | B | A+ | A+ | A+ | A+ |
| | Yeast | A+ | A+ | A+ | A+ | A+ | A+ | A+ | A+ | A+ | A+ | A+ | A+ |
| | Filamentous fungus | A | A+ | A+ | A+ | A+ | A+ | A+ | A+ | A+ | A+ | A+ | A+ |
| Antimicrobial effects (challenge test) 40°C, Duration: 3 months | Bacterial flora | A+ | A+ | A+ | A+ | A+ | A+ | A+ | B | A+ | A+ | A+ | C |
| | Yeast | A+ | A+ | A+ | A+ | A+ | A+ | A+ | A+ | A+ | A+ | A+ | D |
| | Filamentous fungus | A | A+ | A+ | A+ | A+ | A+ | A+ | A+ | A+ | A+ | A+ | D |

As is clear from the results in Table 5 described above, it was found that the functional particle dispersion (liquid dispersion) having the reduction properties of each of Production Examples 23 to 33 obtained as described above had long-lasting effects of reduction performance (ability to remove dissolved oxygen), dispersion stability, and antimicrobial effects all superior to those of Comparative Production Example 3.

In addition, it was confirmed that functional particle dispersions (liquid dispersions) of Production Examples 31 to 33, which further contained a preservative component together with the reducing component to enhance the antimicrobial effects, had further enhanced antimicrobial performance as compared with the functional particle dispersions (liquid dispersions) having the reduction properties and containing the reducing component of Production Examples 23 to 30, without adversely affecting the long-lasting effect, other blended components, and the like, and had excellent dispersion stability.

Third Embodiment: Examples 23 to 33 and Comparative Examples 9 to 12: Preparation of Aqueous Ink Composition for Writing Instrument For Examples 23 to 33, the functional particle dispersions (liquid dispersions) having the reduction properties obtained in Production Examples 23 to 33 described above were obtained. The solids content of the functional particles in the functional particle dispersion having the reduction properties obtained in each of Production Examples 23 to 33 was 35 to 40 mass%.

On the other hand, as Comparative Example 9, the dispersion of Comparative Production Example 3 was used. The following three types of known oxygen absorbents were used for Comparative Examples 10 to 12.

In Comparative Example 10, sodium L-ascorbate was used. In Comparative Example 11, N-acetyl-cysteine was used. In Comparative Example 12, an oligomer of N-vinyl-2-pyrrolidone (degree of polymerization: 2 to 6) was used.

Each of the functional particle dispersions having the reduction properties (particles 23 to 33) produced in Production Examples 23 to 33 described above and Comparative Examples 9 to 12 described above was used to prepare each of the aqueous ink compositions for writing instruments by an ordinary method according to the blend composition (total amount 100 mass%) described below.

| | |
|---|---|
| Ink composition: (Total amount 100 mass%) | |
| Colored resin fine particles (*7) | 50 mass% |
| Thickener: Alkali swelling emulsion (*8) | 0.3 mass% |
| Lubricant: Phosphate (*9) | 0.5 mass% |
| Each functional particle dispersion (Production Examples 23 to 33, Comparative Production Example 3, Comparative Examples 10 to 12) | 20 mass% |
| pH Adjuster (triethanolamine) | 1 mass% |
| Water-soluble organic solvent (ethylene glycol) | 5 mass% |
| Water (distilled water) | Balance |

| | |
|---|---|
| *7: RUBCOULEUR 224 (SMD) black, available from Dainichiseika Color & Chemicals Mfg. Co., Ltd. *8: RHEOTECH 3800, available from Arkema *9: Phosphanol RS-610, available from Toho Chemical Industry Co., Ltd. | |

The obtained aqueous ink compositions for writing instruments (total amount 100 mass%) were evaluated for writing performance (vertical line density difference), the bubble generation situation after the lapse of time, and the bubble generation situation after giving an impact, by writing instruments A and B having the following configurations and the following evaluation method.

The evaluation results of Examples 23 to 33 and Comparative Examples 9 to 12 are shown in the following Table 6.

### Preparation of Writing Instrument: Ballpoint Pen

Using a shaft of a ballpoint pen A [trade name: Signo UM-100, available from Mitsubishi Pencil Co., Ltd.], a refill including an ink storage tube made of polypropylene having a shaft diameter of 6.2 mm and a total length of 129 mm, a stainless steel tip (cemented carbide ball, ball diameter: 0.5 mm) and a joint connecting the storage tube and the tip was filled with each of the aqueous ink compositions described above, and an ink follower containing a mineral oil as a main component was inserted at the rear end of the ink. Thus, an aqueous ballpoint pen was prepared. Using a shaft of a ballpoint pen B [trade name: Signo UMN-155, available from Mitsubishi Pencil Co., Ltd.], a refill including an ink storage tube made of polypropylene having a shaft diameter of 6.2 mm and a total length of 112 mm, a stainless steel tip (cemented carbide ball, ball diameter: 0.5 mm) and a joint connecting the storage tube and the tip was filled with the aqueous ink compositions described above, and an ink follower containing a mineral oil as a main component was inserted at the rear end of the ink, thus preparing a retractable aqueous ballpoint pen.

### Method for Evaluating Writing Performance (Vertical Line Density Difference)

Each aqueous ballpoint pen A having the aforementioned configuration was left to stand at room temperature (25°C, the same applies hereinafter) for one month, and then writing was performed up to the final writing point, and the difference in density of the drawn line at the start of writing and at the end of writing was compared and evaluated according to the following evaluation criteria.

### Evaluation criteria:

A: No difference in density is observed.
B: A slight difference in density is observed.
C: A difference in density is clearly observed.
D: A difference in density is remarkable, and a portion where the drawn line is difficult to be visually recognized is observed.

### Method for Evaluating Bubble Generation Situation after Lapse of Time

Each ballpoint pen A having the aforementioned configuration was stored with the pen tip facing downward in an atmosphere of 50°C. and 30% RH for one month, and after the aforementioned period of time, the ballpoint pen A was left to stand with the pen tip facing downward at room temperature for 6 hours. Then, air bubbles appearing at an interface between the ink and the ink follower were visually confirmed, and evaluation was performed according to the evaluation criteria indicated below.

### Evaluation criteria:

A: No bubbles are present at the interface between the ink and the ink follower.
B: One bubble having a diameter smaller than 1 mm is present at the interface between the ink and the ink follower.
C: One or more bubbles having diameters equal to or larger than 1 mm or two or more bubbles having diameters smaller than 1 mm are present at the interface between the ink and the ink follower.
D: The ink follower is pushed up by bubbles, and a gap is present between the ink follower and the ink interface.

### Method for Evaluating Bubble Generation after Giving Impact

Each retractable ballpoint pen B having the aforementioned configuration was knocked 5 times with the pen point facing downward, then stored with the pen tip facing downward in an atmosphere of 50°C and 30% RH described above for one week, and after the aforementioned period of time, the retractable ballpoint pen B was left to stand with the pen tip facing downward at room temperature for 6 hours. Then, air bubbles appearing at an interface between the ink and the ink follower were visually confirmed, and evaluation was performed according to the following evaluation criteria.

### Evaluation criteria:

A: No bubbles are present at the interface between the ink and the ink follower.
B: One bubble having a diameter smaller than 1 mm is present at the interface between the ink and the ink follower.
C: One or more bubbles having diameters equal to or larger than 1 mm or two or more bubbles having diameters smaller than 1 mm are present at the interface between the ink and the ink follower.
D: The ink follower is pushed up by bubbles, and a gap is present between the ink follower and the ink interface.

For the obtained aqueous ink composition for writing instruments (total amount 100 mass%), the following microbial testing method in accordance with ISO 11930:2012 (procedure for interpretation of data generated by the preservation efficacy test or by the microbiological risk assessment, or both) was performed.

The challenge test was performed by using the three groups including the bacterial flora, the yeast, and the filamentous fungus described below for initial and after storage at 40°C for 3 months. To check the antimicrobial effects more strictly, each of the aqueous ink compositions for writing instruments was charged in Posca PC-5M (available from Mitsubishi Pencil Co., Ltd.; shaft material: PP resin; stirring ball: stainless steel (φ: 6.4 mm)) and stored at a temperature of 40°C for a period of 3 months. The ink taken out from the pen body was then subjected to the evaluation method described above (challenge test by using the three groups including the bacterial flora, the yeast, and the filamentous fungus, evaluation criteria).
Bacterial group: Staphylococcus aureus NBRC13276, Escherichia coli NBRC3972
Yeast: Candida albicans NBRC1594
Filamentous fungus: Aspergillus brasiliensis

### Preparation of Inocula

Preparation of inoculum: Bacterial cultures were prepared in accordance with ISO 11930:2012.
Bacterial flora: Each bacterial culture was prepared in accordance with ISO 11930:2012 for each bacterial strain. Three types of the bacterial cultures, each of which has its bacterial strain adjusted to 1 × 10⁷ to 1 × 10⁸ cfu/mL, were mixed using equal amounts, and thus an inoculum was prepared.
Yeast: In accordance with ISO 11930:2012, a bacterial culture was prepared to be 1 × 10⁶ to 1 × 10⁷ cfu/mL.
Filamentous fungus: In accordance with ISO 11930:2012, a bacterial culture was prepared to be 1 × 10⁶ to 1 × 10⁷ cfu/mL.

### Inoculation

For the ink composition for writing instruments, 1 mass% of a bacterial culture was inoculated.

### Storage

The inoculated ink composition for writing instruments was stored at a temperature of 22.5 ± 2.5°C, and was observed for a designated period of time by detection culture. <Detection Culture> A total of 1 g was applied on 10 sheets of SCD agar media for the bacterial flora, SD agar media for the yeast, and PD agar media for the filamentous fungus, and the bacterial flora and the yeast were cultured at 32.5°C for 2 days, and the filamentous fungus was cultured for 22.5°C for 5 days.

### Evaluation Criteria

A+: Colonies did not appear at day 3.
A: Colonies did not appear at day 7.
B: Colonies did not appear at day 21.
C: Several to tens of colonies emerged at day 28.
D: Obvious increase was observed at day 28.

**[Table 6]**

| | | Example | | | | | | | | | | | Comparative Example | | | |
|---|---|---|---|---|---|---|---|---|---|---|---|---|---|---|---|---|
| | | 23 | 24 | 25 | 26 | 27 | 28 | 29 | 30 | 31 | 32 | 33 | 9 | 10 | 11 | 12 |
| Production Example No. or Comparative Production Example No. | | 1 | 2 | 3 | 4 | 5 | 6 | 7 | 8 | 9 | 10 | 11 | 9 | 10 | 11 | 12 |
| Occurrence of aggregation (writing performance) | | A | A | A | A | A | A | A | A | A | A | A | D | C | C | C |
| Bubble generation after lapse of time (ballpoint pen A) | | A | A | A | B | A | A | A | A | A | A | A | C | D | D | D |
| Bubble generation after impact was given (ballpoint pen B) | | A | A | A | A | A | A | A | A | A | A | A | D | C | D | D |
| Antimicrobial effects (challenge test) initial | Bacterial flora | A+ | A+ | A+ | A+ | A | A+ | A+ | A | A+ | B | A+ | A+ | A | A+ | A+ |
| | Yeast | A+ | A+ | A | A+ | A | A+ | A+ | A+ | A+ | A+ | A+ | A | A+ | B | A+ |
| | Filamentous fungus | A+ | A+ | A+ | A+ | A+ | A+ | A+ | B | A+ | A+ | A+ | A | A+ | A+ | A+ |
| Antimicrobial effects (challenge test) 40°C, Duration: 3 months | Bacterial flora | A+ | A+ | A+ | A+ | A | A | A | A+ | A+ | A | A+ | C | C | B | D |
| | Yeast | A+ | A | A+ | A | A+ | A | A+ | A+ | A+ | A | B | C | C | C | D |
| | Filamentous fungus | A+ | A+ | A | A | A+ | A+ | A+ | A | A+ | A | A+ | C | D | D | D |

In considering Table 6 above, as compared with Comparative Examples 9 to 12, which fall outside the scope of the present disclosure, it was confirmed that Examples 23 to 33, which fall within the scope of the present disclosure, were writing performance (vertical line density difference), generated no bubble even after the lapse of time, and generated no bubble even after an impact was given, and other blended components of the ink were not adversely affected while strength and long-lasting effects of reduction performance against oxygen (oxygen absorbing ability) were maintained.

In addition, it was confirmed that the aqueous ink compositions for writing instruments using the functional particle dispersions (liquid dispersions) having the reduction properties of Examples 31 to 33, which further contained a preservative component together with the reducing component to enhance the antimicrobial effects, had further enhanced antimicrobial performance as compared with the functional particle dispersions (liquid dispersions) having the reduction properties of Examples 23 to 30, without adversely affecting the long-lasting effect, other blended components, and the like, and had excellent dispersion stability. It was also confirmed that the ballpoint pens A and B prepared above did not cause blur and feathering, and had a sufficient drawn line concentration to give a sharp drawn line.

### Examples 34 to 37: Preparation of Cosmetic

As Examples 34 to 37, using each of the functional particle dispersions (liquid dispersions) obtained in Production Examples 1, 10, 11, and 23 described above, each cosmetic was prepared by an ordinary method according to the blend composition described below (total amount 100 mass%).

### Example 34

| Composition of cosmetic for eyeliner: | (Total amount 100mass%) |
|---|---|
| Functional particle dispersion (Production Example 1): | 20 mass% |
| Coloring material (pigment): Iron oxide *1: | 14 mass% |
| Film forming agent: Emulsion resin particles *2: | 14 mass% |
| Nonionic surfactant: POE (20) cetyl ether: | 0.05 mass% |
| Additive component: EDTA-Na: | 0.07 mass% |
| Dispersant: Sodium polyaspartate *3: | 1 mass% |
| Water-based solvent: 1,3-Butylene glycol: | 10 mass% |
| Water-based solvent: Water (purified water): | Balance |

| | |
|---|---|
| *1 TALOX ABL-412HP/R-516P, available from Titan Kogyo, Ltd. *2 YODOSOL GH800F, available from Nouryon Japan K.K. *3 AQUADEW SPA-30B, available from Ajinomoto Co., Inc. | |

### Example 35

| Composition of cosmetic for eyebrows: | (Total amount 100mass%) |
|---|---|
| Functional particle dispersion (Production Example 10): | 10 mass% |
| Coloring material (pigment): Iron oxide *1: | 10 mass% |
| Coloring material (pigment): Mica *2: | 5 mass% |
| Coloring material (pigment): Carbon black *3: | 5 mass% |
| Coloring material (pigment): Titanium oxide *4: | 5 mass% |
| Film forming agent: Trimethylsiloxysilicate *5: | 10 mass% |
| Surfactant: Sodium stearate: | 2 mass% |
| Oil: Squalane: | 10 mass% |
| Water-based solvent: Glycerin: | 20 mass% |
| Water-based solvent: Water (purified water): | Balance |

| | |
|---|---|
| *1 TALOX ABL-412HP/R-516P (available from Titan Kogyo, Ltd.) *2 MICA POWDER Y-2300 (available from Yamaguchi Mica Co., Ltd.) *3 DK BLACK No. 2 (available from Daito Kasei Kogyo Co., Ltd.) *4 TIPAQUE CR-50 (available from Ishihara Sangyo Kaisha, Ltd.) *5 KF-7312K (available from Shin-Etsu Chemical Co., Ltd.) | |

### Example 36

| Composition of cosmetic for body art: | (Total amount 100mass%) |
|---|---|
| Functional particle dispersion (Production Example 11): | 10 mass% |
| Film forming agent *1: | 15 mass% |
| Coloring material (dye): Blue 1 (C.I. 42090): | 5 mass% |
| pH Adjuster: 5 mol/L HCl: | 1 mass% |
| Water-based solvent: n-Propyl alcohol: | 30 mass% |
| Water-based solvent: Water (purified water): | Balance |
| *1: EMUPOLY-CE 119N (available from Gifu Shellac Mfg. Co., Ltd.) | |

### Example 37

| Composition of cosmetic for hair dye: | | (Total amount 100mass%) |
|---|---|---|
| Functional particle dispersion (Production Example 23) | | 10 mass% |
| Coloring material (dye) Black 401 | | 1 mass% |
| pH Adjuster Lactic acid | | 2 mass% |
| Water-based solvent | Benzyl alcohol | 10 mass% |
| Water-based solvent | Ethanol | 40 mass% |
| Water-based solvent | Water (purified water) | Balance |

For cosmetics obtained in Examples 34 to 37 described above, the usability, effects, and the like thereof were evaluated.

The cosmetic for eyeliner obtained in Example 34 described above was loaded in a pen-type applicator (FIG. 1) having a brush in an application part and subjected to evaluation of writing performance on skin, stability, and antimicrobial effects (challenge test, regarding the antimicrobial effects after storage at 40°C for a period of 3 months, a sample was obtained by taking out the cosmetic charged in the applicator of FIG. 1). It was confirmed that the cosmetic for eyeliner obtained in Example 34 was superior compared to Comparative Production Example 1.

The cosmetic for eyebrows obtained in Example 35 described above was formed into a solid cosmetic (square having 3 cm length × 3 cm width, 0.5 cm thickness) and subjected to evaluation of lingering aroma intensity, storage stability (no occurrence of separation or the like), and antimicrobial performance. It was confirmed that the cosmetic for eyebrows of Example 35 was superior compared to Comparative Production Example 2. Note that, for the antimicrobial performance of Example 35 described above, the test bacterial liquid was directly sprayed onto a surface of the cosmetic for eyebrows to evaluate the antimicrobial performance on the solid cosmetic surface.

The cosmetic for body art obtained in Example 36 described above was loaded in a pen-type applicator (FIG. 1) having a brush in an application part and subjected to evaluation of lingering aroma intensity, dispersion stability, and antimicrobial effects. It was confirmed that the cosmetic for body art obtained in Example 36 was superior compared to Comparative Production Example 2.

The cosmetic for hair dye obtained in Example 37 described above was loaded in a pen-type applicator (FIG. 1) having a brush in an application part and subjected to evaluation of occurrence of aggregation and antimicrobial performance. It was confirmed that the cosmetic for hair dye obtained in Example 37 was superior compared to Comparative Production Example 3. Note that, for the evaluation of reduction properties, measurement of amount of dissolved oxygen (value measured by using a dissolved oxygen meter: WQ-320 (available from Horiba, Ltd.) at a measurement temperature of 25°C after a sample was allowed to stand at a temperature of 25°C, for storage periods of 48 hours and 3 months) was used and evaluated. The amount of dissolved oxygen was compared to that of Comparative Production Example 3, and all the cosmetics for hair dyes using the reducing particles of the present disclosure had lower measured values of the amounts of dissolved oxygen, and it was confirmed that the cosmetics for hair dyes using the reducing particles of the present disclosure were superior.

### Industrial Applicability

The functional particle dispersion of the present disclosure has excellent dispersion stability and excellent antimicrobial effects and fragrance performance or reduction performance against oxygen (oxygen absorbing ability) without adversely affecting other blended components and the like, is low-irritative even in a compounding system that is easily affected by ions or the like, has a high degree of freedom in design of the functional particles, and can be used in various applications. Examples of such purposes include medical apparatus, baby products, nursing products, bath products, kitchen utensils, tableware, drinking water piping parts, daily hygiene products, household electrical appliances, clothing, building materials, agricultural materials, automobile interior parts, stationery, and ink compositions for writing instruments and inkjet printers, skin care cosmetics such as a skin lotion and a milky lotion, makeup cosmetics (liquid or solid) such as a foundation and an eye shadow, hair cosmetics such as a hair mist and a hair oil, and deodorant cosmetics.

## Claims

1. A functional particle dispersion, comprising functional particles dispersed in water and having a measured zeta potential value of the functional particles being in a range of -15 to +15 mV, the functional particles being formed from at least a (meth)acrylate monomer represented by General Formula (I) and at least one antimicrobial component selected from Group X set forth below: 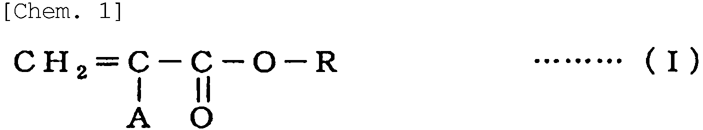 where in Formula (I), A represents a hydrogen atom (H) or a methyl group (CH₃), and R represents a hydrogen atom (H), an alkyl group having 1 to 22 carbon atoms, or a substituent having a polyalkylene glycol chain containing an alkylene chain of 2 to 18 carbon atoms, where the alkyl group or the substituent having a polyalkylene glycol chain may have, as a substituent, a phenyl group, a benzyl group, an epoxy group, a hydroxyl group, a dialkylamino group, an alkoxy group having 1 to 18 carbon atoms, a perfluoroalkyl group having 1 to 18 carbon atoms, or a trialkoxysilyl group;
Group X: an iodopropargyl compound, thiabendazole, sodium pentachlorophenol, 1,2-benzisothiazolin-3-one, 2,3,5,6-tetrachloro-4(methylsulfonyl)pyridine, phenol, sodium benzoate, sodium dehydroacetate, potassium sorbate, morpholine, cresol, methylisothiazolinone, chloromethylisothiazolinone, octylisothiazolinone, dichlorooctylisothiazolinone, hexahydro-1,3,5-tris(2-hydroxyethyl)-1,3,5-triazine, 2-bromo-2-nitropropane-1,3-diol, sodium 2-pyridinethiol-1-oxide, sodium pyrithione, 2-(4-thiozolyl)benzimidazole, 4-terpineol, 1,8-cineol, thymol, diisothiocyanate, eucalyptus oil, longifolene, isopropylmethylphenol, 2-methyl-4-isothiazolin-3-one, citral, eugenol, allyl isothiocyanate, d-limonene, tannic acid, ethylparaben, benzalkonium chloride, glyceryl caprylate, a glycerin fatty acid ester, chlorphenesin, salicylic acid, ethyl parahydroxybenzoate, butyl parahydroxybenzoate, propyl parahydroxybenzoate, methyl parahydroxybenzoate, bisabolol, hinokitiol, phenylethyl alcohol, phenethyl alcohol, phenoxyethanol, butylparaben, propylparaben, and methylparaben.

2. A functional particle dispersion, comprising functional particles dispersed in water and having a measured zeta potential value of the functional particles being in a range of -15 to +15 mV, the functional particles being formed from at least a (meth)acrylate monomer represented by General Formula (I), at least one antimicrobial component selected from Group X set forth below, and at least one type of fragrance component: 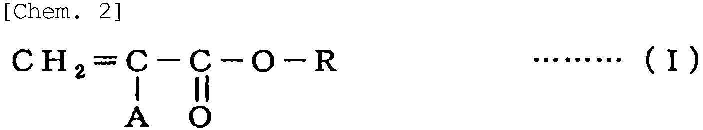 where in Formula (I), A represents a hydrogen atom (H) or a methyl group (CH₃), and R represents a hydrogen atom (H), an alkyl group having 1 to 22 carbon atoms, or a substituent having a polyalkylene glycol chain containing an alkylene chain of 2 to 18 carbon atoms, where the alkyl group or the substituent having a polyalkylene glycol chain may have, as a substituent, a phenyl group, a benzyl group, an epoxy group, a hydroxyl group, a dialkylamino group, an alkoxy group having 1 to 18 carbon atoms, a perfluoroalkyl group having 1 to 18 carbon atoms, or a trialkoxysilyl group;
Group X: an iodopropargyl compound, thiabendazole, sodium pentachlorophenol, 1,2-benzisothiazolin-3-one, 2,3,5,6-tetrachloro-4(methylsulfonyl)pyridine, phenol, sodium benzoate, sodium dehydroacetate, potassium sorbate, morpholine, cresol, methylisothiazolinone, chloromethylisothiazolinone, octylisothiazolinone, dichlorooctylisothiazolinone, hexahydro-1,3,5-tris(2-hydroxyethyl)-1,3,5-triazine, 2-bromo-2-nitropropane-1,3-diol, sodium 2-pyridinethiol-1-oxide, sodium pyrithione, 2-(4-thiozolyl)benzimidazole, 4-terpineol, 1,8-cineol, thymol, diisothiocyanate, eucalyptus oil, longifolene, isopropylmethylphenol, 2-methyl-4-isothiazolin-3-one, citral, eugenol, allyl isothiocyanate, d-limonene, tannic acid, ethylparaben, benzalkonium chloride, glyceryl caprylate, a glycerin fatty acid ester, chlorphenesin, salicylic acid, ethyl parahydroxybenzoate, butyl parahydroxybenzoate, propyl parahydroxybenzoate, methyl parahydroxybenzoate, bisabolol, hinokitiol, phenylethyl alcohol, phenethyl alcohol, phenoxyethanol, butylparaben, propylparaben, and methylparaben.

3. A functional particle dispersion, comprising functional particles dispersed in water and having a measured zeta potential value of the functional particles being in a range of -15 to +15 mV, the functional particles -being formed from at least a (meth)acrylate monomer represented by General Formula (I) and at least one reducing component selected from Group Z set forth below: 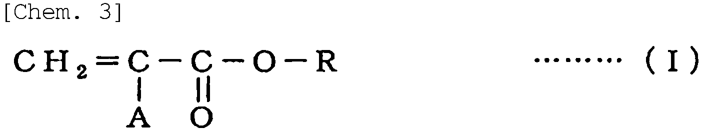 where in Formula (I), A represents a hydrogen atom (H) or a methyl group (CH₃), and R represents a hydrogen atom (H), an alkyl group having 1 to 22 carbon atoms, or a substituent having a polyalkylene glycol chain containing an alkylene chain of 2 to 18 carbon atoms, where the alkyl group or the substituent having a polyalkylene glycol chain may have, as a substituent, a phenyl group, a benzyl group, an epoxy group, a hydroxyl group, a dialkylamino group, an alkoxy group having 1 to 18 carbon atoms, a perfluoroalkyl group having 1 to 18 carbon atoms, or a trialkoxysilyl group;
Group Z: polyphenols, copper chlorophyll, flavonoids, anthocyanidins, dibutylhydroxytoluene, and butylhydroxyanisole.

4. An aqueous ink composition for writing instruments, comprising the functional particle dispersion according to any one of claims 1 to 3.

5. A cosmetic, comprising the functional particle dispersion according to any one of claims 1 to 3.
